(19) Europäisches Patentamt
European Patent Office
Office européen des brevets

(11) EP 0 911 400 A1

(12) # EUROPEAN PATENT APPLICATION
published in accordance with Art. 158(3) EPC

(43) Date of publication:
**28.04.1999 Bulletin 1999/17**

(21) Application number: **98905810.2**

(22) Date of filing: **09.03.1998**

(51) Int. Cl.[6]: **C12N 15/12**, A61K 31/70

(86) International application number:
**PCT/JP98/00953**

(87) International publication number:
**WO 98/39438 (11.09.1998 Gazette 1998/36)**

(84) Designated Contracting States:
**AT BE CH DE DK ES FI FR GB GR IE IT LI LU MC NL PT SE**

(30) Priority: **07.03.1997 JP 53578/97**

(71) Applicant:
**MOCHIDA PHARMACEUTICAL CO., LTD.
Shinjuku-ku Tokyo 160-8515 (JP)**

(72) Inventors:
- **FURUSAKO, Shoji,
Mochida pharmaceutical Co., Ltd.
Shinjuku-ku, Tokyo 160-8515 (JP)**
- **HORISAWA, Yoshifumi,
Mochida pharm. Co., Ltd
Shinjuku-ku, Tokyo 160-8515 (JP)**
- **KUSUYAMA, Takeshi,
Mochida pharm. Co., Ltd.
Shinjuku-ku, Tokyo 160-8515 (JP)**

(74) Representative:
**Grünecker, Kinkeldey,
Stockmair & Schwanhäusser
Anwaltssozietät
Maximilianstrasse 58
80538 München (DE)**

(54) **ANTISENSE COMPOUNDS TO CD14**

(57) The present invention relates to an oligonucleotide and derivatives, hybridizable with or being complementary to at least a part of a gene encoding human CD14; and to pharmaceutical compositions, comprising the oligonucleotide or derivatives thereof as effective ingredient; and is utilisable of cure of systemic inflammatory response sydorome, etc., by the use of the pharmaceutical composition.

EP 0 911 400 A1

## Description

Technical Field:

The present invention relates to an oligonucleotide containing a sequence complementary to a part of a gene encoding human CD14. Further, it relates to a pharmaceutical composition comprising said nucleotide and a pharmacologically acceptable carrier.

Background Technology:

500,000 people in the United States suffer from sepsis caused by bacterial infection and 175,000 people die. The disease is highly lethal and effective therapeutic method is not established (Science, Volume 264, page 365, 1994). The cause has been considered to be a direct effect of lipopoly succharide (hereinafter designated as "LPS", which is almost synonymic for endotoxin). 1985 Beutler et al. reported that anti-TNF antibody-administered mouse exhibits resistance to a lethal amount of endotoxin (Science, Volume 229, page 869, 1995). On the other hand, Tracy et al. discovered that endotoxin-analogous shock and organic impairment occur in recombinant TNFα-administered animal (Science, Volume 234, page 470, 1996), whereby it was found that the septic shock is caused not by direct effect of LPS, but by excess cytokine production from a macrophage activated by stimulation of LPS, namely hyper -cytokinemia. This discovery was an opportunity to try a therapeutic method targeting TNFα produced in an excess amount by stimulation of LPS. However, the clinical test targeting the TNFα conducted in the beginning of 1990 years ended up with disappointing result, wherein good result was not obtained in indexes, e.g. a survival rate of 28 days after (Nature Medicine, Volume 3, page 1193, 1997).

Antibiotics are employed for the purpose preventing bacterial infection at present, whereas it is reported that these antibiotics destroy bactrial bodies and a large amount of LPS is released into blood (Scand. J. Infect. Dis., Volume 101, page 3, 1996). This means that the use of antibiotics may cause septic shock or endotoxic shock. Accordingly, in order to prevent the shock it is important to block the stimulation of LPS together with administration of antibiotics.

CD14 is a glycosyl phosphatidylinositol-linked type glycoprotein with a molecular weight of 55 kd, expressed accompanied by of differentiation maturation of bone marrow cell. Todd et al. reported the CD14 as surface antigen of human peripheral blood monocytes (New York, Springer-Verlag, pages 424 to 433, 1984) . Now it is clarified that CD14 is present on membrane of macrophage, monocyte, Kupffer cells, and neutrophil.

Goyert et al reported DNA sequence of human CD14 in 1988 (Nucleic Acid Research, Volume 16, No. 9, page 4173, 1988), and Yamamoto et al. reported DNA sequence of mouse CD14 in 1988 (Somat. Cell Mol. Genet., Volume 14, page 427, 1988). It has been suggested that the CD14 gene is present on the fifth chromosome within a gene cluster where a hematopoietiesis differentiating proliferating factor group, such as IL-3 or GM-CSF, G-CSF, etc. of fifth chromosome, is present, and concern the differentiation maturation of hematopoietiesis tissue. However, detailed function thereof was unknown.

In 1990, Wright et al. reported that the CD14 is a receptor of LPS of Gram-negative Bacillus (Wright et al., Science, Volume 249, page 1431, 1990). Further, recent study discovered that the CD14 binds not only to LPS but also to proteoglycan (Gupta et al., J. Biol. Chem, Volume 271, No. 38, page 23310, 1996). It is also reported that the ingredients of Gram-negative bacteria and Gram-positive bacteria activate the cells through CD14 (Jerome et al., Immunityl Volume, page 509, 1994). In other words, it is estimated that when organisms are bacterially infected, CD14 binds to bacterial ingredients, whereby macrophage and monocyte expressing the CD14 are activated and various inflammatory factors (inflammatory cytokine, e.g. TNFα, IL-1, IL-6, IL-8, PAI-2, MCP-1, etc., arachidonic metabolites, PAF and nitrogen monoxide, etc.) are released and induced, whereby it contributes to the bacterial infection prevention in the early phase of infection (Matthew et al., J. Biol. Chem., Volume 60, page 728, 1996). On the other hand, it is also estimated that under disease conditions, such as sepsis, activation of macrophage due to a large quantity of LPS from bacteria leads to release of a large amount of TNFα into blood, and causes shock (Fearn. S et al., J. Exp. Med., Volume 181, page 857, 1995).

At present, the cytokine production mechanism by LPS via CD14 is estimated below. In short, aggregated LPS originated from bacterium together with LPS-binding protein (LBP) forms complexes in blood, consequently the LPS monomer becomes capable of efficiently binding to CD14 molecules on the macrophage in a proportion of 1:1. Singal of the LPS bound on the surface of cells is transmitted into cell via a route analogous to ceramide or an unknown route; NFkB as transcription factor is activated in the cell, the production of various cytokines including TNFα is induced (Ulevith et al., Annual Review of Immunology, 13, 437, 1995). These facts indicate that primary response of the host in case of bacterial infection initiates from that the CD14 on monocyte/macrophage response to LPS or Gram-positive bacterium ingredients.

By the way, there are two forms of the CD14 molecule, i.e. membrane-binding form and soluble-form. The production of the soluble CD14 is assumed that the membrane-binding CD14 is cleaved by protease to become soluble CD14 (Philip et al., Eur. J. Immunol., Volume 2, page 604, 1995).

It is reported that the soluble CD14 binds to LPS molecule in the blood and transports it to HDL, so that the soluble CD14 serves for the clearance of the LPS (Wurfel et al., J. Exp. Med., Volume 186, page 1743, 1995). On the other hand, it is assumed that the membrane CD14 binds to LPS, allows to transmit the signal into cells to induce inflammatory cytokine. In short, the CD14 possesses functions contrary to each other, i.e. an effect removing LPS and another effect inducing inflammatory factors.

JP Patent Application Laid-Open No. 5-501399 discloses a curing method of sepsis employing anti-CD14 antibody. The anti-CD14 antibody inhibits the binding between CD14 and LPS, and capable of blocking the signal via CD14, suppresses the expression of inflammatory cytokine, and consequently cures the sepsis. WO93/19772 and WO96/2057 disclose the curing of sepsis employing soluble-type CD14.

Nevertheless, taking high mortality and numbers of patients of septic shock into consideration, provision of more effective medicines is required.

Disclosure of the invention

The present inventors have investigated in order to provide more effective medicines against septic shock. They have foreseen that the inflammatory cytokine produced from liver Kupffer cells in liver by LPS stimulation plays an important role, and have assumed that specific blocking of the binding between LPS and CD14 on Kupffer cells would be clinically effective in a way of not affecting the soluble-type CD14 contributing the removal of LPS, or the CD14 on aveolar macrophage or peritoneal macrophage, or on other macrophages contributing for bacterial infection prevention on each site. They have assumed that the use of antisense oligonucleotide accumulative to liver would work on the CD14 on the liver Kupffer cells in high selectivity.

It is known that: Mouse Kupffer cell in normal state merely expresses CD14 weakly, but when the cell is stimulated by LPS, it comes to express the CD13 strongly. On the other hand, the liver is the most susceptible organ to shock, it is also known that the reduction of liver function considerably affects constitutional symptom. The present inventors provide a medicine effective to sepsis or septic shock based on new view selectively inhibiting CD14 on Kupffer cell, expression of which is induced by LPS stimulation, and mainly inhibiting the production of inflammatory cytokine from Kupffer cells. In other words, the present inventors provide an antisense oligonucleotide to CD14 as medicament effective to sepsis or septic shock.

It has been totally unknown, whether the antisense oligonucleotide of CD14 inhibits the expression of CD14 so as to be utilisable as medicine and is applicable to the treatment of sepsis or not. The inventors have investigated and confirmed that the antisense oligonucleotide of CD14 is utilisable as medicine. Further, the inventors have succeeded in the following manner to determine a particularly effective region as target of antisense nucleotide within the gene of ca. 1.4 kb encoding the CD14.

In other words, they have identified the active regions for 5' non-coding region and translation initiation region, by translation inhibition experiment using a human CD14 luciferase fusion protein expression system, and combination of CD14 protein expression inhibitory activity due to recombinant HeLa cell and TNF$\alpha$ production inhibitory activity due to human macrophage-like cell lines . In respect of the coding region the active region of which cannot easily identified, and 3' non-coding region, they have succeeded to identify the active regions by employing a screening using RNaseH which specifically cleaves the duplex of a target RNA and an antisense oligonucleotide. Consequently, they have confirmed the effect and toxicity of these active regions by culture cell or animal system, and completed the invention.

In short, the present invention provides oligonucleotides hybridizing with at least part of a gene encoding human CD14. Of the oligonucleotides, an oligonucleotide containing a sequence complementary to at least part of a gene encoding human CD14 is preferred.

Moreover, the invention provides oligonucleotides containing a sequence complementary to at least one sequence selected from the group consisting of 5' non-coding region, translation initiation region, coding region and 3' non-coding region of a human CD14 mRNA, and at least part thereof.

Further, the invention provides oligonucleotides, hybridizing with or being complementary to any one of sequences or at least a part of sequence selected from the group consisting of:

(1) a nucleotide sequence of 40 mer of nucleotides positioning from 23th cytosine to 62th adenine,
(2) a nucleotide sequence or 39 mer of positioning from 93th guanine to 131th cytosine,
(3) a nucleotide sequence of 29 mer of positioning from 117th guanine to 145th uridine,
(4) a nucleotide sequence of 40 mer of positioning from 1241th adenine to 1280th guanine,
(5) a nucleotide sequence of 22 mer of positioning from 1264th guanine to 1285th cytosine,
(6) a nucleotide sequence of 54 mer of positioning from 1267th cytosine to 1320th adenine,
(7) a nucleotide sequence of 50 mer of positioning from 1301th guanine to 1350th adenine,
(8) a nucleotide sequence of 20 mer of positioning from 184th cytosine to 203th adenine,

(9) a nucleotide sequence of 20 mer of positioning from 324th adenine to 343th cytosine,
(10) a nucleotide sequence of 20 mer of positioning from 394th uridine to 413th guanine,
(11) a nucleotide sequence of 46 mer of positioning from 444th cytosine to 489th cytosine,
(12) a nucleotide sequence of 20 mer of positioning from 534th guanine to 553th uridine,
(13) a nucleotide sequence of 25 mer s of positioning from 644th uridine to 668th uridine,
(14) a nucleotide sequence of 75 mer of positioning from 684th cytosine to 758th uridine,
(15) a nucleotide sequence of 35 mer of positioning from 794th adenine to 828th guanine,
(16) a nucleotide sequence of 55 mer of positioning from 864th cytosine to 918th guanine,
(17) a nucleotide sequence of 55 mer of positioning from 994th guanine to 1048th cytosine,
(18) a nucleotide sequence of 45 mer of positioning from 1064th guanine to 1108th uridine, and
(19) a nucleotide sequence of 30 mer of positioning from 1194th guanine to 1223th guanine,
in a nucleotide sequence of SEQ.ID. No. 1.

Of these oligonucleotides, oligonucleotides capable of inhibiting the human CD14 expression are preferred. For instance, an oligonucleotide exhibiting a high binding ability with a human CD14 gene in an RNase H cleavage experiment, and an oligonucleotide capable of suppressing the expression of human CD14 by at least 30 % in a translation inhibition experiment are preferred.
The nucleotide number of present oligonucleotides is preferably any one of 10 to 50, in particular preferably any one of 15 to 30.
The present invention also provides oligonucleotides wherein at least one of internucleotides linkages contains a sulphur atom.
Further, the present invention provides oligonucleotides containing at least one of nucleotide sequences selected from the group consisting of SEQ.ID. Nos. 10, 11, 12, 13, 16, 19, 20, 21, 22, 23, 24, 25, 26, 27, 28, 29, 32, 33, 34, 35, 36, 37, 39, 40, 41, 42, 45, 46, 47, 48, 49, 50, 51, 52, 53, 54, 55, 56, 57, 58, 59, 61, 62, 63, 64, 70, 71, 72, 73, 74, 75, 76, 77, 78, 79, 81, 83, 85, 86, 87, 88, 89, 90, 102, 103, 109, 123, 124, 125, 130, 135, 136, 137, 138, 144, 155, 156, 159, 160, 161, 162, 163, 164, 165, 170, 171, 172, 177, 178, 179, 180, 181, 190, 191, 192, 193, 194, 196, 197, 198, 199, 209, 210, 215, 216, 220, 221, 224, 225, 226, 227, 228, 229, 230, 231, 232, 233, 234, 235, 236, 237, 238, 239, 240, 241, 242, 243, 244, 245, 246, 247 and 248; and composed of 30 or less nucleotides.
Further, the present invention provides pharmaceutical compositions comprising an oligonucleotide hybridizing with a gene encoding the CD14 as effective ingredient. In addition to the oligonucleotides hybridizing with a gene encoding the CD14, if necessary, the present pharmaceutical composition comprises a pharmacologicaly acceptable carrier. The pharmaceutical composition is preferably a prophylactic/therapeutic agent against sepsis or septic shock, or disorders caused by an inflammatory factor induced by human CD14.

Brief Explanation of Drawings:

Fig. 1: A graph indicating CD14 translation inhibitory activity of antisense oligonucleotides complementary to a gene encoding human CD14.

Fig. 2: A graph indicating the effects of the nucleotide length of antisense oligonucleotides complementary to a gene encoding human CD14.

Fig. 3: A graph indicating human TNFα production inhibitory activity of antisense oligonucleotides complementary to 5' non-coding region and AUG neighbouring region of mRNA encoding human CD14.

Fig. 4: A graph indicating human TNFα production inhibitory activity of antisense oligonucleotides complementary to 3' non-coding region of mRNA encoding human CD14.

Fig. 5: A graph indicating mouse TNFα production inhibitory activity of antisense oligonucleotides complementary to 5' non-coding region and AUG neighbouring region of mRNA encoding mouse CD14.

Fig. 6: A graph indicating the effect of oiligonucleotide SMO105A in endotoxin shock model.

Fig. 7: A graph indicating the effect of oiligonucleotide SMO105A on liver function in endotoxin shock model.

Fig. 8: A graph indicating the inhivitory activity of antisense oligonucleotides to a gene encoding human CD14 to expression of human CD14/luciferase fusion protein.

Fig. 9: A graph indicating inhibitory activity inhibitory activity of antisense oligonucleotides complementary to the coding region of mRNA encoding human CD14 to human TNFα production.

Fig. 10: A drawing indicating comparison of human antisense oligonucleotide and mouse antisense oligonucleotide around the translation initiation region.

Fig. 11: A graph indicating human CD14/luciferase fusion protein expression inhibition activity of consensus oligonucleotides.

Fig. 12: A graph indicating mouse TNFα production inhibitory activity of consensus oligonucleotides.

Summary of the Invention:

Hereinafter the present invention is illustrated.

The oligonucleotides in the present invention are capable of hybridizing with at least a part of a gene encoding human CD14. Preferably, the oligonucleotides contains a sequence complementary to at least a part of the gene encoding human CD14.

In the description of the present invention, the word "oligonucleotide" includes all the oligonucleotides wherein a plurality of nucleotide composed of base, phosphate and sugar is bound, and derivatives thereof. The representative oligonucleotides are DNA and RNA. The oligonucleotide derivatives include all the ones, steric structure and function of which are analogous to oligonucleotides. For instance, there are a derivative wherein other substance is bound to 3'-end or 5'-end of oligonucleotide, derivatives wherein any one of base, sugar and phosphate of an oligonucleotide is substituted or modified, substances not present in nature, and comprising a base, sugar and phosphate which are not in natre and derivatives having a skeleton other than sugar-phosphate framework (backbone).

The word "gene" in the present specification means chromosome DNA or transcript (mRNA and precursor thereof). The word "gene encoding CD14" means a structural gene defining the CD14 amino acid sequence, intervening sequences (introns) present in the midst of the structural gene, and base sequences concerning the expression of CD14 which are present in the up stream of the structure gene (promoters, operators, etc.) or down stream of the structure gene. The representative sequences of the gene encoding human CD14 are indicated by SEQ.ID. No. 1 and No. 2 in the sequence listing.

The wording "to hybridize" in the present specification means to form a specific binding with bases of DNA or RNA. The strength of hybridizing may be any one with Tm value of at least 45 °C in 0.15 M phosphate buffer, preferably the one with Tm value of at least 55 °C. The specific binding is generally formed by complementary binding, however the binding form is not limited herein. In short, the present oligonucleotides may not necessarily have sequences completely complementary to target sequence, as far as the oligonucleotide is specifically bound to at least a part of the gene encoding human CD14; may contain universal bases represented by inosine and 5-nitroindole; and may partially contain bases or sequences, which are not complementary sequences. The term "to hybridize" includes the case of forming double-stranded or triple-stranded conformation in Watson-Crick base pairing or Hoogsteen base pairing or of the both base pairings. The term "complementary sequence" designates such base pairs as form complementary base pairs being base-sepcific to nucleotide sequences of DNA or RNA. In general, the complementary base pairs are formed between C (cytosine) and G (guanine), between T (thymine) and A (adenine), and between U (uracil) and A (adenine).

The oligonucleotides of the present invention preferably are hybridized with at least a part of mRNA encoding human CD14 or precursor thereof.

The length of the present oligonucleotides is not particularly limited. In general, any nucleotide sequence containing at least 10 nucleotide is considered to have specific sequence. Accordingly, every present oligonucleotides which has a nucleotide sequence of at least 10 is expected to be hybridized specifically with a gene encoding human CD14.

On the other hand, too long oligonucleotide is not suitable for taking-up of oligonucleotides into cells. Any length of the oligonucleotides in the invention is acceptable. Considering that the present oligonucleotides are taken up into cells in order to inhibit the human CD14 expression, it is preferred that the present oligonucleotide is hybridized with a gene encoding human CD14, and the nucleotide length is 10 mer to 50 mer, preferably 15 mer to 30 mer. In other words, the present antisense oligonucleotides are, for instance, oligonucleotides which are hybridized with or complementary to sequences of n to n+10th, n to n+11th, n to n+12th, n to n+13th, n to n+14th, n to n+15th, n to n+16th, n to n+17th, n to n+18th, n to n+19th, n to n+20th, n to n+21th, n to n+22th, n to n+23th, ........ n to n+50th (n = 1 to 1341) within SEQ. ID. No. 1 or No. 2.

The present oligonucleotides may target any sites of the gene encoding human CD14, mRNA encoding human CD14, or precursor thereof. In short, the sites, to which the present oligonucleotides are bound, are not particularly limited. However, the present oligonucleotides are preferably bound to any of translation initiation regions, coding regions, 5, non-coding regions, 3' non-coding regions, ribosome-binding regions, capping regions, splicing regions, and loop portions forming the hairpin structure, of mRNA or mRNA precursors. Above of all, the translation initiation region of human CD14 mRNA is most suitable for the target of the present oligonucleotides in view of the effect. The coding regions are preferred, if accumulation of the present oligonucleotide in nucleus is presumed.

Specifically, the present oligonucleotides are preferably designed to target any region chosen from the group consisting of the following (1) to (19) within mRNA to human CD14 of SEQ. ID. No. 1.

(1) a nucleotide sequence of 40 mer of nucleotides positioning from 23th cytosine to 62th adenine,
(2) a nucleotide sequence of 39 mer of nucleotides positioning from 93th guanine to 131th cytosine,
(3) a nucleotide sequence of 29 mer of nucleotides positioning from 117th guanine to 145th uridine,
(4) a nucleotide sequence of 40 mer of nucleotides positioning from 1241th adenine to 1280th guanine,
(5) a nucleotide sequence of 22 mer of nucleotides positioning from 1264th guanine to 1285th cytosine,
(6) a nucleotide sequence of 54 mer of nucleotides positioning from 1267th cytosine to 1320th adenine,
(7) a nucleotide sequence of 50 mer of nucleotides positioning from 1301th guanine to 1350th adenine,
(8) a nucleotide sequence of 20 mer of nucleotides positioning from 184th cytosine to 203th adenine,
(9) a nucleotide sequence of 20 mer of nucleotides positioning from 324th adenine to 343th cytosine,
(10) a nucleotide sequence of 20 mer of nucleotides positioning from 394th uridine to 413th guanine,
(11) a nucleotide sequence of 46 mer of nucleotides positioning from 444th cytosine to 489th cytosine,
(12) a nucleotide sequence of 20 mer of nucleotides positioning from 534th guanine to 553th uridine,
(13) a nucleotide sequence of 25 mer of nucleotides positioning from 644th uridine to 668th uridine,
(14) a nucleotide sequence of 75 mer of nucleotides positioning from 684th cytosine to 758th uridine,
(15) a nucleotide sequence of 35 mer of nucleotides positioning from 794th adenine to 828th guanine,
(16) a nucleotide sequence of 55 mer of nucleotides positioning from 864th cytosine to 918th guanine,
(17) a nucleotide sequence of 55 mer of nucleotides positioning from 994th guanine to 1048th cytosine,
(18) a nucleotide sequence of 45 mer of nucleotides positioning from 1064th guanine to 1108th uridine, and
(19) a nucleotide sequence of 30 mer of nucleotides positioning from 1194th guanine to 1223th guanine.

Of the above nucleotide sequences (1) to (19), the regions comprising nucleotide sequences (1), (2), (4), (5), (7), (8), (11), (16) and (19) respectively are considered to be particularly effective as target of the present oligonucleotides.

Accordingly, the preferred examples of the present oligonucleotides are oligonucleotides being capable of hybridizing with any of sequences selected from above (1) to (19), and oligonucleotides being capable of hybridizing with at least a part of any sequences selected from above (1) to (19). Preferably they are oligonucleotides being capable of hybridizing with any sequences selected from the nucleotide sequences (1), (2), (4), (5), (7), (8), (11), (16) and (19), and oligonucleotides being capable of hybridizing with at least a part of any sequences selected from the nucleotide sequences (1), (2), (4), (5), (7), (8), (11), (16) and (19). More preferably, the present oligonucleotides have nucleotide sequences complementary to any sequences selected from the above (1) to (19), or nucleotide sequences complementary to at least a part of any sequences selected from the above (1) to (19), preferably nucleotide sequences complementary to any sequences selected from the nucleotide sequences (1), (2), (4), (5), (7), (8), (11), (16) and (19), and nucleotide sequences complementary to at least a part of any sequences selected from the nucleotide sequences (1), (2), (4), (5), (7), (8), (11), (16) and (19). These oligonucleotides preferably comprises 10 to 50 nucleotides. A preferred example of the present oligonucleotides is an oligonucleotide having nucleotide sequences being capable of hybridizing with or complementary to at least 10 contiguous nucleotide within any nucleotide sequences selected from the above (1) to (19).

Of above sequences, sequences (1) to (3) locate within the region of 5' non-coding region to translation initiation site of mRNA encoding human CD14, and sequences (8) to (19) locate within coding region, and sequences (4) to (8) locate within 3' non-coding region.

The present oligonucleotides preferably exhibit inhibitory activity in the expression of human CD14. The present inventors discovered as indicated in Example 13 that the RNaseH cleavage experiment is effective as indicator for the selection of effective oligonucleotide inhibiting the expression of CD14. Accordingly, among the oligonucleotides hybridizing with, or having sequences complementary to at least a part of human CD14 mRNA, the preferred present oligonucleotides exhibit at least score 1, preferably at least 2, in an RNase H cleavage experiment. Furthermore, the oligonucleotides capable of inhibiting at least 20 %, preferably at least 40 %, of human CD14 expression in human CD14/luciferase fusion protein expression inhibition experiment, the oligonucleotides capable of inhibiting the TNFα production in TNFα production inhibition experiment, and the oligonucleotides capable of inhibiting at least 30 % of the CD14 translation in CD14 translation inhibition experiment are preferred.

Further, the present invention provides oligonucleotides having at least one nucleotide sequence selected from the group consisting of sequence Nos. 10, 11, 12, 13, 16, 19, 20, 21, 22, 23, 24, 25, 26, 27, 28, 29, 32, 33, 34, 35, 36, 37, 39, 40, 41, 42, 45, 46, 47, 48, 49, 50, 51, 52, 53, 54, 55, 56, 57, 58, 59, 61, 62, 63, 64, 70, 71, 72, 73, 74, 75, 76, 77, 78, 79, 81, 83, 85, 86, 87, 88, 89, 90, 102, 103, 109, 123, 124, 125, 130, 135, 136, 137, 138, 144, 155, 156, 159, 160, 161, 162, 163, 164, 165, 170, 171, 172, 177, 178, 179, 180, 181, 190, 191, 192, 193, 194, 196, 197, 198, 199, 209, 210, 215, 216, 220, 221, 224, 225, 226, 227, 228, 229, 230, 231, 232, 233, 234, 235, 236, 237, 238, 239, 240, 241, 242, 243, 244, 245, 246, 247 and 248 of sequence list. Phosphorothioate oligonucleotide and phosphodiester oligonucleotide are admixed in the above sequence list. However, the list indicates oligonucleotides having nucleotide sequences of above sequence Nos., herein regardless of the presence or absence of modification and of kinds of derivatives. The present oligonucleotides have the above nucleotide sequences, and are preferably of 30 mer or less.

With the development of antisense-technology, various derivatives have been discovered aiming for improvement of medical effect of oligonucleotides. At present, various oligonucleotide derivatives with high binding affinity to the target DNA or mRNA, histo-selectivity, ability of cellular uptake, nuclease resistance, and intracellular stability are obtained. As explained above, the present oligonucleotides include all kinds of derivatives including the ones composed of base, phosphate, backbone structure not present in nature. As examples of the derivatives included in the present invention, there are derivatives having phosphodiester linkage, phosphorothioate linkage, methylphosphonate linkage, phosphoroamidate linkage, phosphorodithioate linkage, and morpholino group as the whole or a part of backbone structure (Shôji Yôko, et al., "Gan to Kagakuryoho", Volume 20, pp. 1899 to 1907, 1993).

As examples of derivatives there are exemplified deoxyribonucleotide guanidine (DNG) (Robert P, et al., Proc. Natl. Acad. Sci. USA, Volume 92, page 6097, 1995), the one wherein 2'-position of sugar moiety is substituted by other atom or substituent, and the one wherein the sugar moiety is modified, such as $\alpha$-ribose (Bertrand JR. Biochem. Biophys. Res. Commun., Volume 164, page 311, 1989).

Further, the present invention includes oligonucleotide derivatives, such as the ones wherein the sugar moiety is substituted by other substance, the ones wherein parts of the bases are substituted by inosine or universal bases (a base capable of binding to any of A, T, C and G), the ones wherein cholesterol, acridine, poly-L-lysine, psoralen, or long chain alkyl is bound to 5'-end or 3'-end or inside of the oligonucleotide (G. Degols, et al., Nucleic Acid Research, Volume 17, page 9341, 1989; A. McConnaghie, et al., J. Med. Chem., Volume 38, page 3488, 1993; G. Godard, et al., Eur. J. Biochem., Volume 232, page 404, 1995).

As a preferred example of above derivatives, the present invention provides derivatives with phosphorothioate linkage as backbone structure, i.e. an oligonucleotide wherein at least one internucleotides linkage contains sulphur atom.

The suitable examples of such nucleotides are any oligonucleotides selected from SEQ. ID. Nos. 10, 11, 12, 13, 16, 19, 20, 21, 22, 23, 24, 25, 26, 27, 28, 29, 32, 33, 34, 35, 36, 37, 39, 40, 41, 42, 45, 46, 47, 48, 49, 50, 51, 52, 53, 54, 55, 56, 57, 58, 59, 225, 226, 227, 228, 229, 230, 231, 232, 233, 234, 235, 236, 237, 238, 239, 240, 241, 242, 243, 244, 245, 246, 247, and 248 (in other words, oligonucleotides with phosphorothioate linkage, and having any sequence selected from the sequences of above SEQ. ID numbers).

As explained above, as far as the present oligonucleotides are hybridized with said target sequences, they may not necessarily contain a sequence completely complementary to a part of base sequence of the target region. On the contrary, considering that the experiment using animal is indispensable for the research of pharmaceuticals, oligonucleotides, which are hybridized with a gene encoding human CD14 and hybridized with a gene encoding CD14 of model animal, are necessary. Such oligonucleotides are obtainable by targeting a region of high homology among the nucleotide sequences encoding human and model animal CD14. For example: SEQ. ID. No. 3 and No. 4 of the sequence listing indicate nucleotide sequences encoding mouse CD14. High homology regions between human and mouse are studied. And antisense oligonucleotide is designed to have complementary nucleotide bases regarding the consensus bases between human and mouse, and universal bases represented by inosine and 5-nitroindole are substituted for mismatched bases, whereby oligonucleotides to be hybridized with a gene encoding mouse CD14 and a gene encoding human CD14 both can be prepared. In the same manner, oligonucleotides being capable of hybridizing with a gene encoding human CD14 and also genes encoding CD14 of arbitrary at least two animals other than human can be prepared. As matter of course, if necessary, phosphorothioate linkage may be introduced to backbone. Among such oligonucleotides, the preferred ones, whose CD14 expression inhibitory activity is expectable, can be designed by targeting regions composed of any one of nucleotide sequences selected from (1) to (9). For the purpose improving the complementation of the oligonucleotides encoding human or other animals' CD14, the targeting may include several nucleotide of down stream and several nucleotide of up stream than said region. As embodiments of such antisens oligonucleotides, there are oligonucleotides having nucleotide sequence wherein at least one base is substituted by universal base in a nucleotide sequence complementary to any nucleotide sequence selected from the following (1) to (9). Alternatively, there are exemplified oligonucleotides with nucleotide sequence wherein at least one nucleotide is substituted by universal base in a nucleotide sequence complementary to arbitrary portion composed of at least 10 contiguous nucleotide sequence, within nucleotide sequence selected from the following (1) to (9).

(1) a nucleotide sequence of 29 mer of nucleotides positioning from 103th adenine to 131th cytosine in SEQ. ID. No.1,
(2) a nucleotide sequence of 20 mer of nucleotides positioning from 184th cytosine to 203th adenine in SEQ. ID. No.1,
(3) a nucleotide sequence of 20 mer of nucleotides positioning from 324th adenine to 343th cytosine in SEQ. ID. No.1,
(4) a nucleotide sequence of 46 mer of nucleotides positioning from 444th cytosine to 489th cytosine in SEQ. ID. No.1 ,
(5) a nucleotide sequence of 75 mer of nucleotides positioning from 684th cytosine to 758th uridine in SEQ. ID. No.1,
(6) a nucleotide sequence of 35 mer of nucleotides positioning from 794th adenine to 828th adenine in SEQ. ID. No.1,
(7) a nucleotide sequence of 45 mer of nucleotides positioning from 864th cytosine to 908th adenine in SEQ. ID. No.1 ,
(8) a nucleotide sequence of 53 mer of nucleotides positioning from 994th guanine to 1046th guanine and in SEQ. ID. No.1,
(9) a nucleotide sequence of 45 mer of nucleotides positioning from 1064th guanine to 1108th uridine in SEQ. ID. No.1.

Specifically, the oligonucleotides have whole of a nucleotide sequence selected from the following (10) to (18), or arbitrary partial sequence composed of at least 10 contiguous oligonucleotides. These sequences are designed so as to be hybridized with any of human, mouse or simian CD14 mRNA.

(10) CAA CAA GCX XXX XXC XCG CTC CAT GGT CGX TAX XT
(11) TTC XTC GTC XAG CTC XCA XGG
(12) ACT GCC XCX GXT CXG CXT CXG XXT CXA CXC GCX TTA GAA
(13) AGX TXX TCX AGX GTC AGT TCC TXG AGG CXG GAX XXC XCX AGX ACA CGC AXG GC
(14) GCX GXX ATC AGT CCX CXX TCG CCC AXT XCA GGA TTG TCA GAC AGG TCT AXG XTG GXX AGG GCX GGG AAX XCG CG
(15) GCA CAC GCC XXT GGG CGT CTC CAT XCC XGX GTT XCG CAG CGC TA
(16) TXC XGX XXC XCG CAG XGA XTT GTG XCT XAG GTC TAG XCX XTG
(17) CTG TTG XAX CTG AGA TCX AGC ACX CTG AGC TTG GCX GGC AGX CCT TTA GG
(18) CCA XXA AGG GAT TXC CXT XXA GTG XCA GGT TXX CCA CXT XGG GCA GCT C

(In the above sequences (10) to (18), X stands for a universal base.)

More specifically, there are oligonucleotides with nucleotide sequences of sequence Nos. 242, 243, 244, 245, 246, 247, 248, 249, 250, 251, 252, 253, 254, 255, 256 and 257.

Hereinafter, the process for the preparation of the present oligonucleotides is explained.

Oligonucleotides and derivatives thereof are prepared by known manner (e.g. S. Agrawal, et al., Protocol for oligonucleotides and Analogs, Method in Molecular Biology series, Volume 20, Humana Press; S. Agrawal, et al., Antisense Research and Development, Volume 4, page 185, 1994).

Of natural DNA and RNA, the present oligonucleotides are obtainable by chemical synthesis using synthesiser, or by PCR method using a gene encoding human CD14 as template. Some of derivatives, such as methylphosphonate modification and phosphorothioate modification, can be synthesized using a chemical synthesiser (e.g. model 394, manufactured by Perkin-Elmer Japan K.K.). In such case, the operation is conducted in accordance with a handbook attached to the chemical synthesiser, thus obtained product is purified by HPLC method using reverse phase chromatography, etc., so that the purpose oligonucleotide derivative is obtainable.

The inhibitory activities of the oligonucleotides synthesized by said procedure which hybridize with at least a part of the gene encoding human CD14 in the expression of human CD14 can be confirmed by translation inhibition experiment, using a human CD14/luciferase fusion protein expression system. Moreover, the effect inhibiting the expression of inflammatory factor induced via human CD14 can be confirmed using a cell based evaluation system. This cell based evaluation system elucidaes the effectivity of the oligonucleotide in such manner that THP-1 cell is differenciated into macrophage-like cell treating with PMA and vitamin D3 as inducer, and the cell are stimulated by LPS to produce TNF, various oligonucleotides are added and their effect is inspected by the inhibitory activity of TNF$\alpha$ as indicator. The present oligonucleotides are evaluated or chosen by its inhibitory activity of the human CD14 expression as indicator using recombinant cells expressing the human CD14. Alternatively, they are evaluated and chosen using biding activity values in RNaseH cleavage experiment.

Next, the use of the present oligonucleotides is explained.

Since the present oligonucleotides are characterized by the binding to a gene encoding human CD14, they can be employed as diagnosis probe aiming for the detection of the human CD14 gene in the specimen. In case of the use of the present oligonucleotides as diagnosis probe, they are labeled with radio isotope, enzyme, fluorescent substance, luminous substance, etc. Subsequently, DNA or mRNA from the cell of a patient, whose CD14 expression is to be inspected, is prepared in the known manner. A marker probe is added to this sample and the mixture is incubated, followed by washing to remove unreacted marker probe. If the specimen contains human CD14 DNA or RNA, the marker probe is bound to them. The presence of hybridization can be detected by luminescence, fluoreschence, radioactivity, etc. from labeled enzyme, fluoreschent substance, lumineschent substance as indicator.

Therefore, the present oligonucleotides as diagnosis probe are employable for the detection of increase or decrease of CD14 expression level in tissues or cells against external stimulation, for the diagnosis of disorders caused by inflammatory factor generated vis CD14, specifically such as systemic inflammatory response syndrom, sepsis and septic shock, ulcerative colitis, Crohn's disease, cancer, graft-versus-host reaction, periodontosis or osteoporosis. They are employable for the diagnosis determining inflammation degree, curing method and prognosis.

In the medical use, the present oligonucleotides with a purity suitable for medical use, if necessary, together with pharmacologically acceptable additives are employed in the preparation form suitable for human administration. The present pharmaceutical compositions are specifically explained below.

Next, the present pharmaceutical compositions are explained. The present pharmaceutical compositions comprise of the present oligonucleotides as above mentioned as an active ingredient. It was, the present pharmaceutical compositions comprise such oligonucleotide that is bound to an gene encoding human CD14, and is capable of inhibiting the human CD14 expression as an active ingredient. In the present pharmaceutical composition, an oligonucleotide with a pruirty suitable for the medical use may be directly dissolved or dispersed in a suitable solvent, or enclosed in liposome, or inserted into a suitable vector. Depending on the necessity, pharmaceutically acceptable addtives are added to the present oligonucleotide, and the mixture may be formed to suitable preparation, such as injection, tablet, capsule, collyrium, creme, suppository, spray, cataplasm, etc. The pharmacologically acceptable carrier includes solvent, base, stabiliser, antiseptic, dissolvent, excipient, buffer, etc.

As already mentioned, the CD14 is LPS receptor present on membrane of macrophage, monocyte, Kupffer cells, and neutrophil. It is estimated that, when bacterial infection is effected, the macrophage and neutrophil are activated via CD14, to induce inflammatory factor. Accordingly, the present pharmaceutical compositions comprising oligonucleotides inhibiting human CD14 expression as an active ingredient can be employed as prophylactic/therapeutic agent against disorders caused by inflammatory factor generated via CD14, specifically such as systemic inflammatory response syndrom, sepsis or endotoxin shock, septic shock, ulcerative colitis, Crohn's disease, autoimmune response or disease, allergy disease, cancer, peritonitis, graft-versus-host reaction, periodontosis or osteoporosis. Since it is assumed that the present pharmaceutical composition more selectively effect on the CD14 on liver Kupffer cells, a high effect as preventive or remedy against particularly sepsis and septic shock, and constitutional symptom and organ insufficiency caused by the sepsis and septic shock is expectable.

Of above disorders, the systemic inflammatory response syndrom (SIRS) is a condition triggered by bacteremia, trauma, burns, pancreatitis and operation invasion, and the grave SIRS lead to multiple organ dysfunction and multiple organ failure and to death. SIRS with bacteria infection is sepsis, and the representative is endotoxemia. In addition to exogenous LPS invasion by trauma, burns, operation invasion, there are reported some cases, i.e. that the invasion of endogeneous LPS from enterobacterial flord result from hyper permeability of intestinal mucosa (Ravin A., et al., Fed. Proc., Volume 21, page 65, 1962). For instance, it was reported that: if the infection is not documented, blood flow rate of mesenteric artery decreases due shock after injury, the physiological barrier of intestinal tract collapses, and bacterial translocation causes endotoxemica due to endogenous LPS (Surgery, Volume 110, page 154, 1991). In all cases of hepatitis with significantly decrease in liver function, such as alcoholic hepatitis, fulminating hepatitis or hepatocirrhosis: If endogenous LPS from intestine enters portal vein, without sufficiently removed by liver Kupffer cells with decrease of hepato-function, and is spilled over into systemic circulation, it cases DIC and multiple organ failure, which cause the death (Tanigawa Hisakazu, et al., Kan-Tan-Sui, Volume 27, page 381, 1993). In burns injury, it was reported that the infection is complicated at lesion, plasma LPS level elevates, inflammatory cytokines represented by TNF are produced, so that disorder is formed (Endô Shigeatsu, et al., Burns, Volume 19, page 124, 1993). In peritonitis, the majority of the cause is infection with Gram-negative bacteria, but sometimes peritonitis is derived from enterobacterium. The graft-versus-host disease is a disorder highly frequently occurred in bone marrow transplantation. It was reported that in the graft-versus-host disease, transplanted lymphocyte attacks the host tissue, in particular it is significant in intestine, LPS enters systemic circulation and causes endotoxemia (Moor KH., et al., Transplantation, Volume 44, page 249, 1987). As grave diseases due to endotoxemia, there are severe infectious disease, such as adult respiratory distress syndrome (ARDS), acute pyopoietic cholangeitis, pandemic peritonitis, postoperative celiac cystoma, etc.

In above preparation forms, administration method and dosage of the present oligonuleotides are adjusted depending on patient's age, sex, disorder kinds and degree. In other words, a suitable amount of the present oligonucleotides for adjustment of the CD14 expression level and improvement of disease condition is administered orally or parentally. For example, 0.001 to 2000 mg/kg are administered continuously or once or divided several portions per one day. In case of intravenous injection, 0.01 to 100 mg/kg are preferred. The present oligonucleotides are sufficiently safe in said dosage. The oral administration includes subglossal administration. The parenteral administration may be selected suitable one from aspiration, transdermal administration, collyrium, intravaginal administration, intra-articular administration, intrarectal administration, intra-artery administration, intravenous administration, topical administration, intramascular administration, subcutaneous administration, intraperitoneal administratoin.

Best mode for the application of the invention:

Hereinafter, the present invention is more specifically illustrated by examples. These are disclosed as examples, but do not intend to limit the invention. Abbreviations hereinafter are based on conventional abbreviations in this field. The operations in the examples were mainly in accordance with Molecular Cloning, A Laboratory Manual 2nd ed. (Sambrook J., et al., Cold Spring Harbor Laboratory, 1989). This is as a reference and included in the contents of the present specification.

The present invention is specifically explained by examples below.

Example 1: Cloning of human CD14 gene

THP-1 cells were inoculated into a 2 well and a 6 well plate at $7.1 \times 10^5$ cells/well, incubated at 37 °C over a day and night. 1$\alpha$,25-Dihydroxyvitamin $D_3$ (manufactured by BIOMOL Research) was added at the final concentration of 0.1 $\mu$M, and further the cells was cultured overnight. The THP-1 cells were collected, from which RNA was extracted using 1 ml of ISOGEN (manufactured by TELTEST) in accordance with protocol. Subsequently, cDNA library was prepared by Superscript Preamplification System (manufactured by GIBCO) from RNA as template which was extracted using oligo dT primer.

PCR was carried out by employing 1.5 $\mu$g of prepared cDNA library, sense primer (5' ACGCGTCGAC GAGTTCACAA GTGTGAAGCC TG 3': SEQ.ID. No. 5), antisense primer (5' ACATGCATGC TTAATAAAGG TGGGGCAAAG GG 3': SEQ.ID. No. 6), and Pfu DNA synthetic enzyme (manufactured by Stratagene). The reaction condition was 30 cycles of 94 °C for 30 seconds, of 55 °C for 30 seconds, and 72 °C for 180 seconds to effect PCR reaction. Amplified DNA fragment and pUC118 plasmid were digested with SalI restriction enzyme and SphI restriction enzyme, respectively, and purified by 1 % agarose gel electrophoresis. Subsequently, DNA fragment digested from pUC118 and the PCR product were mixed in a proportion of 2:1, and ligated using Ligation kit (manufactured by Takara). Subsequently, this reaction mixture was transfected to JM109 cell, plated on agar plate, and incubated at 37 °C overnight. The generated colonies were checked by PCR to identify recombinant clone (pUCH14P-4 plasmid).

Example 2: Construction of the expression plasmid for human CD14/luciferase fusion protein.

In order to obtain an expression vector necessary for the synthesis of RNA employed in vitro translation, DNA fragment digested at HindIII and BamHI sites of pUCH14P-4 plasmid were inserted into an expression vector (pGEMluc plasmid), and cloned to provide pGEMlucH14-9. Subsequently, PCR was carried out using pGEMlucH14-9 plasmid as template, as well as sense primer (5' CCCAAGCTTA AGTGTGAAGC CTGAAGCCGC CGG 3': SEQ. ID. No. 7) and antisense primer (5' ATGGCGCCGG GCCTTTCTTT ATGTTTTTGG CGTCTTCCAG TTGG 3': SEQ. ID. No. 8).

The reaction product was precipitated with ethanol, and digested with BbeI restriction enzyme and HindIII restriction enzyme, respectively. The DNA fragment from pGEMluc and PCR amplified product previously digested with the two restriction enzymes were ligated in the conventional manner, cloned using HB101 cells to provide pGEMluc(ctg)H14-3.

Example 3: Synthesis of oligonucleotides

Phosphodiester oligonucleotides and phosphorothioate oligonucleotides purified with OPC column obtained from Sawady Technology were employed in the following examples. Phosphorothioate oligonucleotides employed in Examples 10 and 11 purified with micro bondasphere C8 (300 Å) were obtained from Nisshinbô. Oligonucleotides complementary to human CD14 and oligonucleotides complementary to mouse CD14 are listed in Tables 1, 2, 3, 5 and 6. In Tables 1, 2, 3, 5 and 6, P=S stands for substitution of one oxygen atom (O) in phosphodiester linkage with a sulphur atom (S), and P=O stands for no substitution.

The mixture of random phosphodiester oligonucleotides or phosphorothioate oligonucleotides made by sequence undefined synthesis with the mixture of four kinds of amidite were used as control oligonucleotide in the following examples.

Oligonucleotides complementary to the gene encoding human CD14 (part 1)

Table 1-1

| oligonucleotide | sequence | base length | modification | SEQ. ID. No. |
|---|---|---|---|---|
| SH0013A | CGGCTTCCAGGCTTCACACT | 20mer | P=S | 9 |
| SH0023A | CGGCACCCGGCGGCTTCCAG | 20mer | P=S | 10 |
| SH0033A | TCCTACACAGCGGCACCCGC | 20mer | P=S | 11 |
| SH0038A | TTCTTTCCTACACAGCGGCA | 20mer | P=S | 12 |
| SH0043A | TTAGCTTCTTTCCTACACAG | 20mer | P=S | 13 |
| SH0048A | GTGCTTTAGCTTCTTTCCTA | 20mer | P=S | 14 |
| SH0053A | TGGAAGTGCTTTAGCTTCTT | 20mer | P=S | 15 |
| SH0063A | GGACAGGCTCTGGAAGTGCT | 20mer | P=S | 16 |
| SH0073A | TCTGAGCTCCGGACAGGCTC | 20mer | P=S | 17 |
| SH0083A | CTTCCGAACCTCTGAGCTCC | 20mer | P=S | 18 |
| SH0093A | GTCGATAAGTCTTCCGAACC | 20mer | P=S | 19 |
| SH0096A | ATGGTCGATAAGTCTTCCGA | 20mer | P=S | 20 |
| SH0099A | TCCATGGTCGATAAGTCTTC | 20mer | P=S | 21 |
| SH0102A | CGCTCCATGGTCGATAAGTC | 20mer | P=S | 22 |
| SH0104A | CGCGCTCCATGGTCGATAAG | 20mer | P=S | 23 |
| SH0105A | GCGCGCTCCATGGTCGATAA | 20mer | P=S | 24 |
| SH0106A | CGCGCGCTCCATGGTCGATA | 20mer | P=S | 25 |
| SH0107A | ACGCGCGCTCCATGGTCGAT | 20mer | P=S | 26 |
| SH0108A | GACGCGCGCTCCATGGTCGA | 20mer | P=S | 27 |
| SH0109A | GGACGCGCGCTCCATGGTCG | 20mer | P=S | 28 |
| SH0112A | GCAGGACGCGCGCTCCATGG | 20mer | P=S | 29 |
| SH0114A | AAGCAGGACGCGCGCTCCAT | 20mer | P=S | 30 |
| SH0116A | ACAAGCAGGACGCGCGCTCC | 20mer | P=S | 31 |

Oligonucleotides complementary to the gene encoding human CD14 (part 2)

Table 1-2

| Oligonucleotide | sequence | base length | modification | SEQ. ID. No. |
|---|---|---|---|---|
| SH0117A | AACAAGCAGGACGCGCGCTC | 20mer | P=S | 32 |
| SH0118A | CAACAAGCAGGACGCGCGCT | 20mer | P=S | 33 |
| SH0120A | AGCAACAAGCAGGACGCGCG | 20mer | P=S | 34 |
| SH0122A | GCAGCAACAAGCAGGACGCG | 20mer | P=S | 35 |
| SH0124A | CAGCAGCAACAAGCAGGACG | 20mer | P=S | 36 |
| SH0126A | AGCAGCAGCAACAAGCAGGA | 20mer | P=S | 37 |
| SH1231A | TCTTGGATCTTAGGCAAAGC | 20mer | P=S | 38 |
| SH1241A | CATTATTCTGTCTTGGATCT | 20mer | P=S | 39 |
| SH1256A | CAGTTTGAGTCCATTCATTA | 20mer | P=S | 40 |
| SH1259A | AGGCAGTTTGAGTCCATTCA | 20mer | P=S | 41 |
| SH1261A | CAAGGCAGTTTGAGTCCATT | 20mer | P=S | 42 |
| SH1262A | CCAAGGCAGTTTGAGTCCAT | 20mer | P=S | 43 |
| SH1263A | GCCAAGGCAGTTTGAGTCCA | 20mer | P=S | 44 |
| SH1264A | AGCCAAGGCAGTTTGAGTCC | 20mer | P=S | 45 |
| SH1265A | AAGCCAAGGCAGTTTGAGTC | 20mer | P=S | 46 |
| SH1266A | GAAGCCAAGGCAGTTTGAGT | 20mer | P=S | 47 |
| SH1267A | TGAAGCCAAGGCAGTTTGAG | 20mer | P=S | 48 |
| SH1268A | CTGAAGCCAAGGCAGTTTGA | 20mer | P=S | 49 |
| SH1269A | CCTGAAGCCAAGGCAGTTTG | 20mer | P=S | 50 |
| SH1270A | CCCTGAAGCCAAGGCAGTTT | 20mer | P=S | 51 |
| SH1271A | CCCCTGAAGCCAAGGCAGTT | 20mer | P=S | 52 |
| SH1273A | CTCCCCTGAAGCCAAGGCAG | 20mer | P=S | 53 |
| SH1276A | GCACTCCCCTGAAGCCAAGG | 20mer | P=S | 54 |
| SH1281A | TGACGGCACTCCCCTGAAGC | 20mer | P=S | 55 |

Oligonucleotides complementary to the gene encoding human CD14 (part 3)

Table 1-3

| Oligonucleotide | sequence | base length | modification | SEQ. ID. No. |
|---|---|---|---|---|
| SH1291A | CTCAACGTCCTGACGGCACT | 20mer | P=S | 56 |
| SH1301A | TCGAAAAGTCCTCAACGTCC | 20mer | P=S | 57 |
| SH1311A | GTTGAATTCGTCGAAAAGTC | 20mer | P=S | 58 |
| SH1331A | TAATAAAGGTGGGGCAAAGG | 20mer | P=S | 59 |
| OH0013A | CGGCTTCCAGGCTTCACACT | 20mer | P=O | 60 |
| OH0023A | CGGCACCCGGCGGCTTCCAG | 20mer | P=O | 61 |
| OH0033A | TCCTACACAGCGGCACCCGG | 20mer | P=O | 62 |
| OH0043A | TTAGCTTCTTTCCTACACAG | 20mer | P=O | 63 |
| OH0053A | TGGAAGTGCTTTAGCTTCTT | 20mer | P=O | 64 |
| OH0063A | GGACAGGCTCTGGAAGTGCT | 20mer | P=O | 65 |
| OH0073A | TCTGAGCTCCGGACAGGCTC | 20mer | P=O | 66 |
| OH0083A | CTTCCGAACCTCTGAGCTCC | 20mer | P=O | 67 |
| OH0092A | GTCGATAAGTCTTCCGAACC | 20mer | P=O | 68 |
| OH0096A | ATGGTCGATAAGTCTTCCGA | 20mer | P=O | 69 |
| OH0099A | TCCATGGTCGATAAGTCTTC | 20mer | P=O | 70 |
| OH0102A | CCCTCCATGGTCGATAAGTC | 20mer | P=O | 71 |
| OH0103A | GCCCTCCATGGTCGATAAGT | 20mer | P=O | 72 |
| OH0104A | CGCCCTCCATGGTCGATAAG | 20mer | P=O | 73 |
| OH0105A | GCGCCCTCCATGGTCGATAA | 20mer | P=O | 74 |
| OH0106A | CGCGCCCTCCATGGTCGATA | 20mer | P=O | 75 |
| OH0107A | ACGCGCCCTCCATGGTCGAT | 20mer | P=O | 76 |
| OH0108A | GACGCGCCCTCCATGGTCGA | 20mer | P=O | 77 |
| OH0109A | CGACGCGCCCTCCATGGTCG | 20mer | P=O | 78 |
| OH0110A | AGGACGCGCCCTCCATGGTC | 20mer | P=O | 79 |

Oligonucleotides complementary to the gene encoding human CD14 (part 4)

Table 1-4

| Oligonucleotide | sequence | base length | modification | SEQ. ID. No. |
|---|---|---|---|---|
| OHO111A | CAGGACGCGCGCTCCATGGT | 20mer | P=O | 80 |
| OHO112A | GCAGGACGCGCGCTCCATGG | 20mer | P=O | 81 |
| OHO113A | AGCAGGACGCGCGCTCCATG | 20mer | P=O | 82 |
| OHO114A | AAGCAGGACGCGCGCTCCAT | 20mer | P=O | 83 |
| OHO118A | CAACAAGCAGGACGCGCGCT | 20mer | P=O | 84 |
| OHO102A-15mer | CATGGTCGATAAGTC | 15mer | P=O | 85 |
| OHO102A-18mer | CTCCATGGTCGATAAGTC | 18mer | P=O | 86 |
| OHO102A-19mer | GCTCCATGGTCGATAAGTC | 19mer | P=O | 87 |
| OHO102A | CGCTCCATGGTCGATAAGTC | 20mer | P=O | 71 |
| OHO102A-21mer | GCGCTCCATGGTCGATAAGTC | 21mer | P=O | 88 |
| OHO102A-22mer | CGCGCTCCATGGTCGATAAGTC | 22mer | P=O | 89 |
| OHO102A-25mer | ACGCGCGCTCCATGGTCGATAAGTC | 25mer | P=O | 90 |
| OHO102A-30mer | GCAGGACGCGCGCTCCATGGTCGATAAGTC | 30mer | P=O | 224 |

Oligonucleotides complementary to the gene encoding mouse CD14

Table 2

| Oligonucleotide | sequence | base length | modification | SEQ. ID. No. |
|---|---|---|---|---|
| SM0097A | CATGCTCGGTAGATTCTGAA | 20mer | P=S | 91 |
| SM0101-0220A | CACACGCTCCATGGTCGGTAGATTC | 25mer | P=S | 92 |
| SM0102A-25mer | GCACACGCTCCATGGTCGGTAGATT | 25mer | P=S | 93 |
| SM0103A-25mer | AGCACACGCTCCATGGTCGGTAGAT | 25mer | P=S | 94 |
| SM0104A-25mer | AAGCACACGCTCCATGGTCGGTAGA | 25mer | P=S | 95 |
| SM0105A-25mer | CAAGCACACGCTCCATGGTCGGTAG | 25mer | P=S | 96 |
| SM0106A-25mer | CCAAGCACACGCTCCATGGTCGGTA | 25mer | P=S | 97 |
| SM0107-0226A -25mer | GCCAAGCACACGCTCCATGGTCGGT | 25mer | P=S | 98 |
| SM0109-25mer | AAGCCAAGCACACGCTCCATGGTCG | 25mer | P=S | 99 |
| SM0111-25mer | ACAAGCCAAGCACACGCTCCATGGT | 25mer | P=S | 100 |
| SM0105A-21mer | CACACGCTCCATGGTCGGTAG | 21mer | P=S | 258 |

Example 4: Synthesis of human CD14 RNA

In vitro transcription reaction was conducted using Ribo max system (manufactured by Promega) in line with attached protocol. pGEMluc(ctg)H14-3 plasmid was digested with XhoI, and blunted with Klenow fragment. Subsequently, in vitro transcription was performed employing 20 μg of this pGEMluc(ctg)H14-3 as template, and SP6 polymerase in the presence of 7-methyl guanine at 37 °C for 4 hours. The reaction product was treated with DNase, and extracted with phenol. The reaction mixture was subjected to ethanol precipitation, obtained RNA pellets were dried with air, and dissolved in distilled water. By denaturing agarose gel electrophoresis the RNA was exhibited as a single band of 1.4 kb.

Example 5: Detection of the inhibitory activities in CD14 translation by oligonucleotide complementary to human non-coding region

In vitro transcription reaction was performed using Rabbit Reticulocyte Lysate System (manufactured by Promega) in line with attached protocol. In other words, synthesized RNA from pGEMluc(ctg)H14-3 and unmodified oligonucleotides to be tested were mixed in a proportion of 1:10, and heated at 60 °C for 2 minutes. Subsequently, amino acids, and Rabbit Reticulocyte Lysate were added to the mixture, and incubated at 30 °C for 2 hours. 10 μl of reaction mixture and an equivalent amount of luminous substrate solution (luciferase assay system, manufactured by Promega) wore mixed, and allowed to react at room temperature for 5 seconds, the luminous intensity of the reaction solution was measured by a luminescence meter (Lumat LB96P). The result is shown in Fig. 1. The inhibitory activity of oligonucleotides was normalized by a fluorescent amount at control oligonucleotide (20mer phosphodiester oligonucleotide with random sequence) treatment as 100 %. sequences exhibiting at least 30 % of inhibitory activity were OH0013A, OH0023A, OH0033A, OH0043A, OH0053A, OH0099A, OH0102A, OH0103A, OH0104A, OH0105A, OH0106A, OH0107A, OH0108A, OH0109A, OH0110A, OH0112A and OH0114A. In particular, antisense oligonucleotides around translational initiation site showed the high inhibitory activity.

Example 6: The inhibitory activities in CD14 translation by oligonucleotides with different length

8 kinds of antisense oligonucleotides with different length (OH0102A-15mer, OH0102A-18mer, OH0102A-19mer, OH0102A, OH0102A-21mer, OH0102A-22mer, OH0102A-25mer, OH0102A-30mer, nucleotide lengths of which were 15mer, 18mer, 19mer, 20mer, 21mer, 22mer, 25mer and 30mer) and control oligonucleotide were tested, and the activity of translation arrest was reviewed in the manner of Example 5. As result, the inhibitory activity in the translation was detected in all nucleotides independent on the nucleotide length (Fig. 2).

Example 7: Measurement of the inhibitory activities in human TNFα production (5' non-coding region and neighbour region of translation initiational site)

THP-1 cells were suspended in RPMI1640 medium containing 10 % inactivated fetal bovine serum, inoculated at 1 X $10^5$ cells/well into the 24 well plates, and cultured in the presence of 10 ng/ml of Phorbol 12-Myristate 13-Acetate (manufactured by SIGMA) for 24 hours. After the medium was exchanged, the oligonucleotides were added at the final concentration of 100 nM. After incubation for 4 hours, the culture supernatant was removed and the cells were washed. The cells were again cultured in a RPMI1640 medium containing 10 % inactivated fetal bovine serum in the presence of 40 ng/ml of 1α, 25-Dihydroxyvitamin $D_3$ (manufactured by BIOMOL Research) for 20 hours. After washing the cells, the medium were replaced with RPMI1640 containing 2 % human serum to which 1 ng/ml of lipopolysuccharide (E. coli 055: B5, manufactured by Difco) was added. After incubation for 4 hours, the culture supernatant was collected. TNFα in the culture supernatant was measured with human TNFα ELISA SYSTEM (manufactured by Amersham).

The measurement of TNFα was performed in line with protocol attached to the human TNFα ELISA SYSTEM. In other words, 50 μl of suitably diluted culture supernatant were transferred to a reaction plate, 50 μl of biotinylated antibody solution were added, and left at room temperature stand for 2 hours. The reaction solution was removed, and wells were washed with 400 μl/well of wash buffer three times. 100 μl of suitably diluted streptavidin-peroxidose conjugate were added, and the mixture was further left to stand for 30 minutes. After washing, 100 μl of chromogenic solution were added, and reacted for 15 minutes. 100 μl of stop solution were added to terminate the reaction, and absorbance at 450 nm was measured in order to calculate the TNFα value in the sample. Fig. 3 indicates the results.

Inhibitory activity in the TNFα production was detected in SH0023A, SH0033A, SH0038A, SH0043A, SH0063A, SH0093A, SH0096A, SH0099A, SH0102A, SH0104A, SH0105A, SH0106A, SH0107A, SH0108A, SH0109A, SH0112A, SH0117A, SH0118A, SH0120A, SH0122A, SH0124A and SH0126A. The results are well related to the result of the inhibitory activity for translation in Example 4. It was found that the active sequences were complementary to namely 5' non-coding region and three regions in the neighbour of translation initiation site, roughly. The active region 1 was indicated by the oligonucleotides complementary to a part of the sequence CUGGAAGCCGCCGGGUGCCGCUGUGUAGGAAAGAAGCUAAA. The active region 2 was indicated by the oligonucleotides complementary to a part of the sequence GGUUCGGAAGACUUAUCGACCAUGGAGCGCGCGUCCUGC. The active region 3 overlapped with the active region 2, and was indicated by the oligonucleotides complementary to a part of the sequence GAGCGCGCGUCCUGCUUGUUGCUGCUGCU.

Example 8: Measurement of the inhibitory activities in human TNFα production (3' non-coding region)

In the same manner as Example 7, Fig. 4 indicates the result of the inhibitory assay TNFα production by oligonucleotides complementary to the 3' non-coding region of human CD14 mRNA.

Inhibitory activity in TNFα production was detected in SH1241A, SH1256A, SH1259A, SH1261A, SH1264A, SH1265A, SH1266A, SH1267A, SH1268A, SH1269A, SH1270A, SH1271A, SH1273A, SH1276A, SH1281A, SH1291A, SH1301A, SH1311A and SH1331A. It was found that the active sequences were complementary to roughly four regions. The active region 4 was indicated by the oligonucleotides complementary to a part of AGAUCCAAGACAGAAUAAUGAAUGGACUCAAACUGCCUUG. The active region 5 was indicated by the oligonucleotides complementary to a part of GGACUCAAACUGCCUUGGCUU. The active region 6 overlapped with the active region 5, and was indicated by the oligonucleotides complementary to a part of the sequence

CUCAAACUGCCUUGGCUUCAGGGGAGUCCCGUCAGGACGUUGAGGACUUUUCGA.

The active region 7 was indicated by the oligonucleotides complementary to a part of GGACGUUGAGGACUUUUCGACCAAUUCAACCCUUUGCCCCACCUUUAUUA.

Example 9: The measurement of inhibitory activities in mouse TNFα production (5' non-coding region and the neighbour region of translational initiation site)

J774A.1 cells were suspended in DMEM medium containing 10 % inactivated fetal bovine serum, inoculated in the 24 well plate at 0.5 X $10^5$ cells/well, and cultivated for 24 hours. After the medium was exchanged, the oligonucleotides were added to the culture medium at the final concentration of 100 nM. After incubation for 4 hours, the culture supernatant was removed, and the cells were washed. Then cells were again cultured in RPMI1640 medium containing 10 % inactivated fetal bovine serum for 20 hours. After washing the cells, the medium was substituted with DMEM containing 2 % mous serum to which lipopolysuccharide (LPS) (E. coli 0111: B4, manufactured by DIFCO) was added at the final concentration of 100 ng/ml. After incubation for 4 hours, the culture supernatant was collected. TNFα in the culture supernatant was determined with mouse TNFα ELISA SYSTEM (manufactured by Amersham).

The measurement of TNFα was carried out in line with protocol attached to mouse TNFα ELISA SYSTEM. In other words, 50 μl of suitably diluted culture supernatant were transferred to a reaction plate, 50 μl of biotinylated antibody solution were added, and left to stand at room temperature for 2 hours. The reaction solution was removed, wells were washed with wash buffer fluid of 400 μl/well three times. 100 μl of suitably diluted streptavidin-peroxidase conjugate were added, and the mixture was further left to stand for 30 minutes. After washing, 100 μl of chromogenic solution were added, and reacted for 15 minutes. 100 μl of stop solution were added to terminate the reaction, and absorbance at 450 nm was determined in order to calculate the TNFα value in the sample. Fig. 5 indicates the results.

The high inhibitory activity of mouse TNFα production was detected in antisense compounds having complementary sequence to the neighbour of mouse CD14 mRNA translation inititation site, e.g. SM0101-0220A, SM0102A-25mer, SM0103A-25mer, SM0104A-25mer, SM0105A-25mer, SM0106A-25mer, SM0107-0226A-25mer and SM0109-25mer.

Example 10: Effect of SM0105A in mouse shock model.

The following experiment using antisense oligonucleotide SM0105A-21mer to a gene encoding mouse CD14 was carried out.

(1) Effect in mortal endotoxin shock model:

Balb/c male mouse of 6 week age (manufactured by Charles River Japan) were grouped into 7 (each group consisting of 10 animals) based on body weight. Subsequently, 3 mg/kg to 0.3 mg/kg of SM0105A oligonucleotide, 3 mg/kg to 0.3 mg/kg of control oligonucleotide (a 21mer phosphorothioate oligonucleotide with random sequence), or 10 ml/kg of saline (for negative control, manufactured by Ôtsuka) were administered to tail vein once.

At 24 hours after the administration, 5 μg/kg of LPS (E. coli 055: B5, manufactured by Difco) and 700 mg/kg of galactosamine (D-Galactosamine hydrochloride, manufactured by Wakô) were administered to tail vein to induce shock. 0.3 mg/kg of methyl prednisolone were administered immediately before the LPS injection. The survival rate was periodically evaluated until 24 hours after the shock induction.

Fig. 6 indicates the results. All animals of saline-administered group as negative control group were dead until 9 hours after the shock induction. All animals of control oligonucleotide administered group were dead until 10 hours after the shock induction, in every dosage amount. On the other hand, in SM0105A oligonucleotide-administered group, all animals of 3 mg/kg dosage group survived after 24 hours, 9 animals of 1 mg/kg dosage group survived, and 2 animals of 0.3 mg/kg dosage group survived. Survival rate of 0.3 mg/kg of SM0105A-administered was equivalent with the survival rate of the same amount of methyl prednisolone-administered. By this result, dosage-dependent survival rate improvement effect of SM0105A was confirmed

(2) Effect of SM0105A in mortal endotoxin pre-shock model.

It was conducted in accordance with method of Matsumoto, T., et al. (FEMS Immunology and Medical Microbiology 17, 171-178 (1997)). 200 mg/kg of cyclophosphamide (hereinafter designated as "CPA") were administered to tail vein of 6 weeks age male Balb/c mouse freely water-fed and dieted. 7 days after CPA administeration, 5 mg of iota carrageenan (manufactured by Sigma) dissolved in a saline were intraperitonally administered. 12 hours after the iota carrageenan injection, 30 μg/kg dosage of LPS (E. coli 127: B8, manufactured by Difco) were administered from tail vein. At 1 hour and 24 hours after LPS administration, blood was collected from eyegroud vein using a glass capillary pretreated with heparin solution (1000 IU/ml) (manufactured by Mochida), 50 μl of the blood were centrifuged to collect plasma, and GPT activity was determined using GPT in blood activity measurement slide GPT/ALT-P, (manufactured by Fuji Film) and Fuji DRI-CHEM 5000 (manufactured by Fuji Film). Control oligonucleotide and SM0105A designed were in a volume of 10 ml/kg saline were administered to tail vein 24 hour before, and water-soluble prednisolone was administered immediately before the LPS administration, in the same manner.

As result, in comparison with the 50 % survival rate of solvent administration group, SM0105A administration group and prednisolone administration group exhibited 100 % of survival rate. Suppression of a significant GTP raise was observed in liver of SM0105A administration group, whereas such effect was not recognised in prednisolone administered group (Fig. 7).

Example 11: Acute toxicity in mouse

The following experiment was carried out using SM105A-21mer.

Balb/c male mice (supplied by Charlse-Liver Japan) of 6 weeks age were divided into 2 groups (4 animals per group).

Subsequently, SM0105A and control oligonucleotide (21mer phosphorothioate oligonucleotide with a random sequence) in an amount of 30 mg/kg, or 10 ml/kg of saline (for negative control, manufactured by Ôtsuka) were administered to tail vein once. The survival rate and GOT value in blood were determined until 7 days after.

All animals were alive and the GOT value in blood was normal of saline administered group and oligonucleotide administered group both, and there was no difference in both groups.

Example 12: Measurement of the inhibitory activities in the expression of human CD14/luciferase fusion protein

(1) Establishing of a HeLa transformant expressing human CD14/luciferase fusion protein

In order to establish a HeLa transformant using for the inhibitory assay of human CD14/luciferase fusion protein expression, the expression plasmid for a human CD14/luciferase fusion protein (pM1651) was constructed. In other words, the pGEMlucH14-9 prepared in Example 2 was digested with HindIII and XhoI to provide a DNA fragment, which was inserted to HindIII/XhoI site of pcDNA3.1(+) (manufactured by Invitrogen) in the conventional manner, cloned by JM109 cell to provide pM1651.

The pM1651 was transfected into HeLa cell, i.e. human endocervix cancer-derived cell, to establish a HeLa transformant expressing human CD14/luciferase fusion protein. In other words, $5 \times 10^5$ of HeLa cell were inoculated onto a dish with 100 mm diameter, cultured for one night, subsequently 10 $\mu$g of pM1651 were transfected by calcium phosphate method. The cell was cultured in the DMEM medium containing 10 % fetal bovine serum for one night. The cells were seeded to a 96 well plate at 100 to 500 cells/well. From the next day, the transformants were chosen in the medium containing G-418. Among obtained G-418-resistant strains, a He1651d3-20 clone exhibiting luciferase activity was employed for the inhibitory assay of fusion protein expression by antisense oligonucleotides.

(2) Measurement of the inhibitory activity in the expression of human CD14/luciferase fusion protein (5' non-coding region, neighbour region of translational initiation site and 5'-coding region)

HeLa transformant (He1651d3-20) prepared in (1) were suspended in the DMEM medium containing 10 % fetal bovine serum and 0.6 mg/mL of G-418, were seeded into the 24 well plate at $1 \times 10^5$ cells/well, and cultured for one night. They were washed with saline (manufactured by Ôtsuka) twice, subsequently 450 $\mu$L/well of Opti-MEM medium (manufactured by Gibco BRL) were added. Subsequently, in line with a handbock of Gibco BRL, lipofectin reagent and an oligonucleotide of SEQ. ID. No. 9, 10, 11, 12, 13, 14, 15, 16, 17, 18, 19, 21, 22, 23, 24, 25, 26, 27, 28, 29, 30, 32, 33, 35, 37 were added at the final concentration 100 nM. The cells were incubated at 37 °C for 6 hours, culture supernatant was removed, and the cells were washed. The cells were again cultured in the DMEM medium containing 10 % fetal bovine serum and 0.6 mg/mL of G418 further for one night. After washing of the cells, the cells were dissolved in Passive Lysis Buffer (manufactured by Promega). Employing 20 $\mu$L of the solution, the luciferase activity in the cell solution was measured. The measurement of luciferase activity was conducted in line with protocol of Promega. In other words, the cell solution and Luciferase Assay Reagent II (manufactured by Promega) were mixed in a plate for fluoroeschence measurement (manufactured by DYNEX; Microlite 2 plate) to initiate reaction, and luminescence intensity for 10 seconds was determined. Luminometer manufactured by berthold (LB96P) was employed for the measurement. In the inhibitory activities of protein expression, oligonucleotides were calculated based on 100% by the luminescence intensity of control sample without oligonucleotide. Fig. 8 indicates the results. Antisense oligonucleotides exhibiting at least 40 % of inhibitory activity in 5, non-coding region were SH0023A, SH0033A, SH0038A and SH0043A. On the other hand, antisense oligonucleotides exhibiting at least 40 % of inhibitory activity in the region containing translation initiation site were SH0102A, SH0104A, SH0105A, SH0106A, SH0107A, SH0108A, SH0109A, SH0112A, SH0114A, SH0117A, SH0118A, SH0122A and SH0126A. These results of the inhibitory activity in protein expression were well consistent with the results of inhibitory activity in CD14 translation by in vitro translation in Example 5.

Example 13: Measurement of antisense oligo-binding activity by Rnase H cleavage test

2 µg of human CD14 RNA obtained in Example 4 and unmodified oligonucleotides to be tested, which are listed in Table 3, were mixed in a molar ratio of 1:1, 1 µl of RNaseH buffer of 5-fold concentration and a suitable amount of distilled water were added to prepare 4 µl of mixture solution. This mixture was heated at 75 °C, then cooled, 0.05 U of RNaseH were added, and reaction was performed at 37 °C for 15 minutes. 10 µl of stop solution of 2-fold concentration (95 % formamide, 0.5 mM EDTA with pH 8.0, 0.025 % of SDS, 0.025 % of Xylene Cyanol, 0.025 % of BPB) were added to terminate the reaction. 4 µl of the sample were pre-treated at 65 °C, and electrophoresed using 6 M urea-denaturing 5 % polyacrylamide gel (160 mm width, 330 mm height, 0.35 mm thickness) at 15 mA/plate. The band generated by staining with 5000-fold diluted SYBER Green II (manufactured by Wakô Pure Chemicals Industries ltd.) was measured with Fluor Imager SI (manufactured by Molecular Dynamics). The score of binding activity was calculated by the following formula.

Binding value= (fluorescence value of sample oligonucleotide - fluorescence value of control oligonucleotide) / (fluorescence value of SH0102A - fluoroscence value of control oligonucleotide )

| score | binding value |
|---|---|
| 0 | $0.5 > X$ |
| 1 | $0.9 > X \geqq 0.5$ |
| 2 | $1.3 > X \geqq 0.9$ |
| 3 | $X \geqq 1.3$ |

Table 3-1

| oligonucleotide | sequence | modification | base length | sequence ID No. |
|---|---|---|---|---|
| OH0083A-15mer | GAACCTCTGAGCTCC | P=0 | 15mer | 101 |
| OH0102A-15mer | CATGGTCGATAAGTC | P=0 | 15mer | 85 |
| OH0104A-15mer | TCCATGGTCGATAAG | P=0 | 15mer | 102 |
| OH0114A-15mer | GGACGCGCGCTCCAT | P=0 | 15mer | 103 |
| OH0134A-15mer | AGCAGCAGCAGCAAC | P=0 | 15mer | 104 |
| OH0144A-15mer | CACCAGCGGCAGCAG | P=0 | 15mer | 105 |
| OH0154A-15mer | CAGAGACGTGCACCA | P=0 | 15mer | 106 |
| OH0164A-15mer | GGCGTGGTCGCAGAG | P=0 | 15mer | 107 |
| OH0174A-15mer | ACAAGGTTCTGGCGT | P=0 | 15mer | 108 |
| OH0184A-15mer | CGTCCAGCTCACAAG | P=0 | 15mer | 109 |
| OH0194A-15mer | AAATCTTCATCGTCC | P=0 | 15mer | 110 |
| OH0204A-15mer | GACGCAGCGGAAATC | P=0 | 15mer | 111 |
| OH0214A-15mer | AGAAGTTGCAGACGC | P=0 | 15mer | 112 |
| OH0224A-15mer | TGAGGTTCGGAGAAG | P=0 | 15mer | 113 |
| OH0234A-15mer | CCAGTCGGGCTGAGG | P=0 | 15mer | 114 |
| OH0244A-15mer | AGGCTTCGGACCAGT | P=0 | 15mer | 115 |
| OH0254A-15mer | ACACACTGGAAGGCT | P=0 | 15mer | 116 |
| OH0264A-15mer | TACTGCAGACACACA | P=0 | 15mer | 117 |
| OH0274A-15mer | TCTCCACCTCTACTG | P=0 | 15mer | 118 |
| OH0284A-15mer | CCGGCATGGATCTCC | P=0 | 15mer | 119 |
| OH0294A-15mer | GTTGAGACCGCCGGC | P=0 | 15mer | 120 |
| OH0304A-15mer | ACGCCTCTAGGTTGA | P=0 | 15mer | 121 |

Table 3-2

| oligonucleotide | sequence | modification | base length | sequence ID No. |
|---|---|---|---|---|
| OH0314A-15mer | CGCTTTAGAAACGGC | P=0 | 15mer | 122 |
| OH0324A-15mer | CGCATCGACGCGCTT | P=0 | 15mer | 123 |
| OH0334A-15mer | CGTCCGCGTCCGCAT | P=0 | 15mer | 124 |
| OH0344A-15mer | TACTGCCGCGGGTCG | P=0 | 15mer | 125 |
| OH0354A-15mer | CGTGTCAGCATACTG | P=0 | 15mer | 126 |
| OH0364A-15mer | GAGCCTTGACCGTGT | P=0 | 15mer | 127 |
| OH0374A-15mer | CGCACGCGGAGAGCC | P=0 | 15mer | 128 |
| OH0384A-15mer | TGTGAGCCGCCGCAC | P=0 | 15mer | 129 |
| OH0394A-15mer | CGGCTCCCACTGTGA | P=0 | 15mer | 130 |
| OH0404A-15mer | GGAACCTGTGCGGCT | P=0 | 15mer | 131 |
| OH0414A-15mer | TACCTGAGCAGGAAC | P=0 | 15mer | 132 |
| OH0424A-15mer | CGCCTACCAGTAGCT | P=0 | 15mer | 133 |
| OH0434A-15mer | ACACGCAGGGCGCCT | P=0 | 15mer | 134 |
| OH0444A-15mer | GTACGCTAGCACACG | P=0 | 15mer | 135 |
| OH0454A-15mer | TGAGGCGGGAGTACG | P=0 | 15mer | 136 |
| OH0464A-15mer | GTCAGTTCCTTGAGG | P=0 | 15mer | 137 |
| OH0474A-15mer | GTCCTCGAGCGTCAG | P=0 | 15mer | 138 |
| OH0484A-15mer | TTATCTTTAGGTCCT | P=0 | 15mer | 139 |
| OH0494A-15mer | ATGGTGCCGGTTATC | P=0 | 15mer | 140 |
| OH0504A-15mer | CAGCGGAGGCATGGT | P=0 | 15mer | 141 |

Table 3-3

| oligonucleotide | sequence | modification | base length | sequence ID No. |
|---|---|---|---|---|
| OH0514A-15mer | CTTCCAGAGGCAGCG | P=O | 15mer | 142 |
| OH0524A-15mer | AGTCCTGTGGCTTCC | P=O | 15mer | 143 |
| OH0534A-15mer | GGAAAGTGCAAGTCC | P=O | 15mer | 144 |
| OH0544A-15mer | GCGCAAGCTGGAAA | P=O | 15mer | 145 |
| OH0554A-15mer | ACGTTGCGTAGGCGC | P=O | 15mer | 146 |
| OH0564A-15mer | CGCCCACGACACGTT | P=O | 15mer | 147 |
| OH0574A-15mer | AACGCCCTGTCGCCC | P=O | 15mer | 148 |
| OH0584A-15mer | GCGAGCCAAGAACGC | P=O | 15mer | 149 |
| OH0594A-15mer | CTGCAGCTCGGCGAG | P=O | 15mer | 150 |
| OH0604A-15mer | TGAGCCACTGCTGCA | P=O | 15mer | 151 |
| OH0614A-15mer | AGGCCTGGCTTGAGC | P=O | 15mer | 152 |
| OH0624A-15mer | CAGTACCTTGAGGCC | P=O | 15mer | 153 |
| OH0634A-15mer | GGGCAATGCTCAGTA | P=O | 15mer | 154 |
| OH0644A-15mer | GAGTGTGCTTGGGCA | P=O | 15mer | 155 |
| OH0654A-15mer | AAAGGCAGGCGAGTG | P=O | 15mer | 156 |
| OH0664A-15mer | GTTCGTAGGAAAAGG | P=O | 15mer | 157 |
| OH0674A-15mer | GCGCGAACCTGTTCG | P=O | 15mer | 158 |
| OH0684A-15mer | GGCCGGGAAGGCGCG | P=O | 15mer | 159 |
| OH0694A-15mer | GGCTGGTAAGGGCCG | P=O | 15mer | 160 |
| OH0704A-15mer | GACAGGTCTAGGCTG | P=O | 15mer | 161 |

Table 3-4

| oligonucleotide | sequence | modification | base length | sequence ID No. |
|---|---|---|---|---|
| OH0714A-15mer | AGGATTGTCAGACAG | P=O | 15mer | 162 |
| OH0724A-15mer | CGCCCACTCCAGGAT | P=O | 15mer | 163 |
| OH0734A-15mer | AGTCCGCGTTCGCCC | P=O | 15mer | 164 |
| OH0744A-15mer | AGCCGCCATCAGTCC | P=O | 15mer | 165 |
| OH0754A-15mer | GGGGACAGAGAGCCG | P=O | 15mer | 166 |
| OH0764A-15mer | GGGAACTTGTGGGGA | P=O | 15mer | 167 |
| OH0774A-15mer | CTGGATGGCCGGGAA | P=O | 15mer | 168 |
| OH0784A-15mer | GCGCTAGATTCTGGA | P=O | 15mer | 169 |
| OH0794A-15mer | GTGTTGCGCAGCGCT | P=O | 15mer | 170 |
| OH0804A-15mer | CTCCATTCCTGTGTT | P=O | 15mer | 171 |
| OH0814A-15mer | CTGTGGGCGTCTCCA | P=O | 15mer | 172 |
| OH0824A-15mer | GCGCACACGCCTGTG | P=O | 15mer | 173 |
| OH0834A-15mer | CGCCAGTGCGGCGCA | P=O | 15mer | 174 |
| OH0844A-15mer | CACCTGCCGCCGCCA | P=O | 15mer | 175 |
| OH0854A-15mer | TGGGGCTGCACACCT | P=O | 15mer | 176 |
| OH0864A-15mer | GTCTAGGCTGTGGGG | P=O | 15mer | 177 |
| OH0874A-15mer | TGTGGCTGAGGTCTA | P=O | 15mer | 178 |
| OH0884A-15mer | CGCAGCGAGTTGTGG | P=O | 15mer | 179 |
| OH0894A-15mer | TACGGTGGCGCGCAG | P=O | 15mer | 180 |
| OH0904A-15mer | CGCTAGGGTTTACGG | P=O | 15mer | 181 |
| OH0914A-15mer | CATCTCGGAGCGCTA | P=O | 15mer | 182 |
| OH0924A-15mer | GGACCACATGCATCT | P=O | 15mer | 183 |
| OH0934A-15mer | TCAGGGCGCTGGACC | P=O | 15mer | 184 |
| OH0944A-15mer | TTGAGGGAGTTCAGG | P=O | 15mer | 185 |
| OH0954A-15mer | GAACGACACAGATTGAG | P=O | 15mer | 186 |

Table 3-5

| oligonucleotide | sequence | modification | base length | sequence ID No. |
|---|---|---|---|---|
| OH0964A-15mer | CCAGCCCAGCGAACG | P=O | 15mer | 187 |
| OH0974A-15mer | GGCACCTGTTCCAGC | P=O | 15mer | 188 |
| OH0984A-15mer | CAGTCCTTTAGGCAC | P=O | 15mer | 189 |
| OH0994A-15mer | GCTTGGCTGGCAGTC | P=O | 15mer | 190 |
| OH1004A-15mer | AGCACTCTGAGCTTG | P=O | 15mer | 191 |
| OH1014A-15mer | GCTGAGATCGAGCAC | P=O | 15mer | 192 |
| OH1024A-15mer | GTCTGTTGCAGCTGA | P=O | 15mer | 193 |
| OH1034A-15mer | GCCCTGTTCAGTCTG | P=O | 15mer | 194 |
| OH1054A-15mer | GCAGCTCGTCAGGCT | P=O | 15mer | 195 |
| OH1064A-15mer | TCCACCTCGGGCAGC | P=O | 15mer | 196 |
| OH1074A-15mer | TGTCAGGTTATCCAC | P=O | 15mer | 197 |
| OH1084A-15mer | TCCCGTCCAGTGTCA | P=O | 15mer | 198 |
| OH1094A-15mer | AGGAAGGGATTCCCG | P=O | 15mer | 199 |
| OH1104A-15mer | TCCAGGGACCAGGAA | P=O | 15mer | 200 |
| OH1114A-15mer | GGAGGGCAGTTCCAG | P=O | 15mer | 201 |
| OH1124A-15mer | CCCTCGTGGGGGAGG | P=O | 15mer | 202 |
| OH1134A-15mer | GTTCATTGAGCCCTC | P=O | 15mer | 203 |
| OH1144A-15mer | CCACGCCGGAGTTCA | P=O | 15mer | 204 |
| OH1154A-15mer | CAGGCTGGGACCACG | P=O | 15mer | 205 |
| OH1164A-15mer | CGAACGTGCACAGGC | P=O | 15mer | 206 |
| OH1174A-15mer | CCGACAGGGTCGAAC | P=O | 15mer | 207 |
| OH1184A-15mer | GACACCCCCACCGAC | P=O | 15mer | 208 |
| OH1194A-15mer | CAGGGTTCCCGACAC | P=O | 15mer | 209 |
| OH1204A-15mer | GGAGCAGCACCAGCG | P=O | 15mer | 210 |

Table 3-6

| oligonucleotide | sequence | modification | base length | sequence ID No. |
|---|---|---|---|---|
| OH1214A-15mer | CGCGCCCCTTGGAGC | P=O | 15mer | 211 |
| OH1224A-15mer | GGCAAAGCCCCGCGC | P=O | 15mer | 212 |
| OH1234A-15mer | TTGGATCTTAGGCAA | P=O | 15mer | 213 |
| OH1244A-15mer | TTATTCTGTCTTGGA | P=O | 15mer | 214 |
| OH1254A-15mer | AGTCCATTCATTATT | P=O | 15mer | 215 |
| OH1264A-15mer | AGGCAGTTTGAGTCC | P=O | 15mer | 216 |
| OH1274A-15mer | CCTGAAGCCAAGGCA | P=O | 15mer | 217 |
| OH1284A-15mer | ACGGGACTCCCCTGA | P=O | 15mer | 218 |
| OH1294A-15mer | CAACGTCCTGACGGG | P=O | 15mer | 219 |
| OH1304A-15mer | GAAAAGTCCTCAACG | P=O | 15mer | 220 |
| OH1314A-15mer | TGAATTGGTCGAAAA | P=O | 15mer | 221 |
| OH1324A-15mer | GGCAAAGGGTTGAAT | P=O | 15mer | 222 |
| OH1334A-15mer | ATAAAGGTGGGGCAA | P=O | 15mer | 223 |

Table 4-1

| oligonucleotide | sequence ID No. | binding activity (score) |
|---|---|---|
| OH0083A-15mer | 101 | 0 |
| OH0102A-15mer | 85 | 1 |
| OH0104A-15mer | 102 | 2 |
| OH0114A-15mer | 103 | 1 |
| OH0134A-15mer | 104 | 0 |
| OH0144A-15mer | 105 | 0 |
| OH0154A-15mer | 106 | 0 |
| OH0164A-15mer | 107 | 0 |
| OH0174A-15mer | 108 | 0 |
| OH0184A-15mer | 109 | 2 |
| OH0194A-15mer | 110 | 0 |
| OH0204A-15mer | 111 | 0 |
| OH0214A-15mer | 112 | 0 |
| OH0224A-15mer | 113 | 0 |
| OH0234A-15mer | 114 | 0 |
| OH0244A-15mer | 115 | 0 |
| OH0254A-15mer | 116 | 0 |
| OH0264A-15mer | 117 | 0 |
| OH0274A-15mer | 118 | 0 |
| OH0284A-15mer | 119 | 1 |
| OH0294A-15mer | 120 | 0 |
| OH0304A-15mer | 121 | 0 |

Table 4-2

| oligonucleotide | sequence ID No. | binding activity (score) |
|---|---|---|
| OH0314A-15mer | 122 | 0 |
| OH0324A-15mer | 123 | 1 |
| OH0334A-15mer | 124 | 1 |
| OH0344A-15mer | 125 | 2 |
| OH0354A-15mer | 126 | 0 |
| OH0364A-15mer | 127 | 0 |
| OH0374A-15mer | 128 | 0 |
| OH0384A-15mer | 129 | 0 |
| OH0394A-15mer | 130 | 1 |
| OH0404A-15mer | 131 | 0 |
| OH0414A-15mer | 132 | 0 |
| OH0424A-15mer | 133 | 0 |
| OH0434A-15mer | 134 | 0 |
| OH0444A-15mer | 135 | 1 |
| OH0454A-15mer | 136 | 1 |
| OH0464A-15mer | 137 | 2 |
| OH0474A-15mer | 138 | 2 |
| OH0484A-15mer | 139 | 0 |
| OH0494A-15mer | 140 | 0 |
| OH0504A-15mer | 141 | 0 |

Table 4-3

| oligonucleotide | sequence ID No. | binding activity (score) |
|---|---|---|
| OH0514A-15mer | 142 | 0 |
| OH0524A-15mer | 143 | 0 |
| OH0534A-15mer | 144 | 1 |
| OH0544A-15mer | 145 | 0 |
| OH0554A-15mer | 146 | 0 |
| OH0564A-15mer | 147 | 0 |
| OH0574A-15mer | 148 | 0 |
| OH0584A-15mer | 149 | 0 |
| OH0594A-15mer | 150 | 0 |
| OH0604A-15mer | 151 | 0 |
| OH0614A-15mer | 152 | 0 |
| OH0624A-15mer | 153 | 0 |
| OH0634A-15mer | 154 | 0 |
| OH0644A-15mer | 155 | 1 |
| OH0654A-15mer | 156 | 1 |
| OH0664A-15mer | 157 | 0 |
| OH0674A-15mer | 158 | 0 |
| OH0684A-15mer | 159 | 1 |
| OH0694A-15mer | 160 | 1 |

Table 4-4

| oligonucleotide | sequence ID No. | binding activity (score) |
|---|---|---|
| OH0704A-15mer | 161 | 2 |
| OH0714A-15mer | 162 | 2 |
| OH0724A-15mer | 163 | 2 |
| OH0734A-15mer | 164 | 1 |
| OH0744A-15mer | 165 | 1 |
| OH0754A-15mer | 166 | 0 |
| OH0764A-15mer | 167 | 0 |
| OH0774A-15mer | 168 | 0 |
| OH0784A-15mer | 169 | 0 |
| OH0794A-15mer | 170 | 1 |
| OH0804A-15mer | 171 | 2 |
| OH0814A-15mer | 172 | 2 |
| OH0824A-15mer | 173 | 0 |
| OH0834A-15mer | 174 | 0 |
| OH0844A-15mer | 175 | 0 |
| OH0854A-15mer | 176 | 0 |
| OH0864A-15mer | 177 | 2 |
| OH0874A-15mer | 178 | 2 |
| OH0884A-15mer | 179 | 2 |
| OH0894A-15mer | 180 | 2 |
| OH0904A-15mer | 181 | 2 |

Table 4-5

| oligonucleotide | sequence ID No. | binding activity (score) |
|---|---|---|
| OH0914A-15mer | 182 | 0 |
| OH0924A-15mer | 183 | 1 |
| OH0934A-15mer | 184 | 0 |
| OH0944A-15mer | 185 | 1 |
| OH0954A-15mer | 186 | 0 |
| OH0964A-15mer | 187 | 0 |
| OH0974A-15mer | 188 | 1 |
| OH0984A-15mer | 189 | 0 |
| OH0994A-15mer | 190 | 1 |
| OH1004A-15mer | 191 | 1 |
| OH1014A-15mer | 192 | 1 |
| OH1024A-15mer | 193 | 2 |
| OH1034A-15mer | 194 | 2 |
| OH1054A-15mer | 195 | 0 |
| OH1064A-15mer | 196 | 2 |
| OH1074A-15mer | 197 | 2 |
| OH1084A-15mer | 198 | 1 |
| OH1094A-15mer | 199 | 1 |
| OH1104A-15mer | 200 | 0 |
| OH1114A-15mer | 201 | 0 |
| OH1124A-15mer | 202 | 0 |
| OH1134A-15mer | 203 | 0 |
| OH1144A-15mer | 204 | 0 |

Table 4-6

| oligonucleotide | sequence ID No. | binding activity (score) |
|---|---|---|
| OH1154A-15mer | 205 | 0 |
| OH1164A-15mer | 206 | 1 |
| OH1174A-15mer | 207 | 0 |
| OH1184A-15mer | 208 | 0 |
| OH1194A-15mer | 209 | 2 |
| OH1204A-15mer | 210 | 3 |
| OH1214A-15mer | 211 | 0 |
| OH1224A-15mer | 212 | 0 |
| OH1234A-15mer | 213 | 0 |
| OH1244A-15mer | 214 | 0 |
| OH1254A-15mer | 215 | 3 |
| OH1264A-15mer | 216 | 3 |
| OH1274A-15mer | 217 | 0 |
| OH1284A-15mer | 218 | 0 |
| OH1294A-15mer | 219 | 0 |
| OH1304A-15mer | 220 | 2 |
| OH1314A-15mer | 221 | 2 |
| OH1324A-15mer | 222 | 0 |
| OH1334A-15mer | 223 | 0 |

Of the antisense oligonucleotides indicating cleavage activity, antisense oligonucleotides to the neighbor region of translation initiation site were OH0102A-15mer, OH0104A-15mer, OH0114A-15mer, and antisense oligonucleotides in 3' non-coding region were OH1254A-15mer, OH1264A-15mer, OH1304A-15mer and OH1314A-15mer. These oligonucleotides have the sequences complementary to parts of active regions 2, 4 and 7, which were considered to be inhibitory activity of TNFα production according to measurement results of inhibitory activity in human TNFα production in Examples 7 and 8. Accordingly, the results of RNaseH cleavage test were well consistent with the results of inhibitory activity in TNFα production.

Example 14: Measurement of inhibitory activity in human TNFα production (coding region)

Concerning the antisense oligonucleotide-binding regions clarified in Example 13, representative antisense oligonucleotides in the each region (see Table 5) were synthesized by the manner of Example 3, and evaluated by the method of Example 7. In other words, THP-1 cell was treated with SH0108A, SH0184A, SH0324A, SH0394A, SH0444A, SH0457A, SH0470A, SH0534A, SH0649A, SH0714A, SH0720A, SH0809A, SH0864A, SH0899A, SH1014A, SN1074A, SH1199A, SH1204A, SH1259A, SH1311A and control oligonucleotide at the final concentration of 30 nM. After incubation for 4 hours the culture supernatant was collected. TNFα in the culture supernatant was measured by human TNFα ELISA SYSTEM (manufactured by Amersham).

Table 5

| oligonucleotide | sequence | base length | modificatio n | sequence No. |
|---|---|---|---|---|
| SH0108A | GACGCGCGCTCCATGGTCGA | 20mer | P=S | 27 |
| SH0184A | TTCATCGTCCAGCTCACAAG | 20mer | P=S | 225 |
| SH0324A | GCGTCCGCATCGACGCGCTT | 20mer | P=S | 226 |
| SH0394A | CTGTGCGGCTCCCACTGTGA | 20mer | P=S | 227 |
| SH0444A | CGGGAGTACGCTAGCACACG | 20mer | P=S | 228 |
| SH0457A | CAGTTCCTTGAGGCGGGAGT | 20mer | P=S | 229 |
| SH0470A | GGTCCTCGAGCGTCAGTTCC | 20mer | P=S | 230 |
| SH0534A | AAGCTGGAAAGTGCAAGTCC | 20mer | P=S | 231 |
| SH0649A | AAAGGCAGGCGAGTGTGCTT | 20mer | P=S | 232 |
| SH0714A | AGTCCAGGATTGTCAGACAG | 20mer | P=S | 233 |
| SH0720A | TCGCCCAGTCCAGGATTGTC | 20mer | P=S | 234 |
| SH0809A | CTGTGGGCGTCTCCATTCCT | 20mer | P=S | 235 |
| SH0864A | CTGAGGTCTAGGCTGTGGGG | 20mer | P=S | 236 |
| SH0899A | CGCTAGGGTTTACGGTGGCG | 20mer | P=S | 237 |
| SH1014A | TTGCAGCTGAGATCGAGCAC | 20mer | P=S | 238 |
| SH1074A | TCCAGTGTCAGGTTATCCAC | 20mer | P=S | 239 |
| SH1199A | GGAGCAGCACCAGGGTTCCC | 20mer | P=S | 240 |
| SH1204A | CCCTTGGAGCAGCACCAGGG | 20mer | P=S | 241 |
| SH1259A | AGGCAGTTTGAGTCCATTCA | 20mer | P=S | 41 |
| SH1311A | GTTGAATTGGTCGAAAAGTC | 20mer | P=S | 58 |

Fig. 9 indicates the results, by which an inhibitory activity was confirmed in twelve regions below.

Active region 8 was indicated by oligonucleotide SH0184A complementary to a part of the sequence :CUUGUGAGCUGGACGA

Active region 9 was indicated by oligonucleotide SH0324A complementary to a part of the sequence AAGCGCGUCGAUGCGGACGCCGACCCGCGGCAGUA

Active region 10 was indicated by oligonucleotide SH0394A complementary to a part of the sequence :UCACAGUGGGAGCCG

Active region 11 was indicated by oligonucleotides SH0444A, SH0457A and SH0470A complementary to a part of the sequence :CGUGUGCUAGCGUACUCCCGCCUCAAGGAACUGACGCUCGAGGAC

Active region 12 was indicated by oligonucleotide SH0534A complementary to a part of the sequence GGACUUGCACUUUCC

Active region 13 was indicated by oligonucleotide SH0649A complementary to a part of the sequence :UACUGAGCAUUGCCCAAGCACACUCGCCUGCCUUU

Active region 14 was indicated by oligonucleotides SH0714A and SH0720A complementary to a part of the sequence

CGCGCCUUCCCGGCCCUUACCAGCCUAGACCUGUCUGACAAUCCUGGACUGGCCGA
ACGCGGACUGAUGGCCGCU .

Active region 15 was indicated by oligonucleotide SH0809A complementary to a part of the sequence UCCAGAAUCUAGCGCUGCGCAACACAGGAAUGGAGACGCCCACAG

Active region 16 was indicated by oligonucleotide SH0899A complementary to a part of the sequence

CCCCACAGCCUAGACCUCAGCCACAACUCGCUGCGCGCCACCGUAAACCCUAGCG

Active region 17 was indicated by oligonucleotide SH1014A complementary to a part of the sequence

GACUGCCAGCCAAGCUCAGAGUGCUCGAUCUCAGCUGCAACAGACUGAACAGGGC

Active region 18 was indicated by oligonucleotide SH1014A complementary to a part of the sequence :GCUGCCCGAGGUGGAUAACCUGAACCACUGGACGGGAAUCCCUUCCU

Active region 19 was indicated by oligonucleotides SH1199A and SH1204A complementary to a part of the sequence GUGUUCGGGAACCCUGGUGCUGCUCC

Example 15: Design of consensus oligonucleotides, and measurement of the inhibitory activities in the CD14 expression.

(1) Design of consensus oligonucleotides:

Oligonucleotides, which are bound to both a gene encoding human CD14 and a gene encoding CD14 of animals other than human, (hereinafter called "consensus oligonucleotide") were prepared by the following manner. First, a region of SEQ ID No. 1 from 93th guanine to 145th uridine, which is considered to be accessible region to bond, was remarked, and sequences of human and mouse were compared. There was designed a 21mer antisense oligonucleotide complementary to the sequence from 103th uridine to 137th uridine which exhibited a high activity in Example 8 and Example 12, in said region, so that all bases wherein sequences were not consistent between human and mouse (bases indicated as X in Fig. 10) were pyrimidine substitution of cytosine or uridine. Thus, an antisense oligonucleotide the bases indicated as X were substituted by inosine which is a base to be bound to pyrimidine base, was designed, and synthesized in the manner according to Example 3. Table 6 indicates the synthesized consensus oligonucleotides.

Table 6

| oligonucleotide | sequence | base length | modification | SEQ. ID. No. |
|---|---|---|---|---|
| SU0103A-21mer | CICGCTCCATGGTCGITAIIT | 21mer | P=S | 242 |
| SU0104A-21mer | ICICGCTCCATGGTCGITAII | 21mer | P=S | 243 |
| SU0105A-21mer | CICICGCTCCATGGTCGITAI | 21mer | P=S | 244 |
| SU0106A-21mer | ICICICGCTCCATGGTCGITA | 21mer | P=S | 245 |
| SU0107A-21mer | IICICICGCTCCATGGTCGIT | 21mer | P=S | 246 |
| SU0108A-21mer | IIICICICGCTCCATGGTCGI | 21mer | P=S | 247 |
| SU0109A-21mer | IIIICICICGCTCCATGGTCG | 21mer | P=S | 248 |
| SU0110A-21mer | CIIIICICICGCTCCATGGTC | 21mer | P=S | 249 |
| SU0111A-21mer | GCIIIICICICGCTCCATGGT | 21mer | P=S | 250 |
| SU0112A-21mer | AGCIIIICICICGCTCCATGG | 21mer | P=S | 251 |
| SU0113A-21mer | AAGCIIIICICICGCTCCATG | 21mer | P=S | 252 |
| SU0114A-21mer | CAAGCIIIICICICGCTCCAT | 21mer | P=S | 253 |
| SU0115A-21mer | ACAAGCIIIICICICGCTCCA | 21mer | P=S | 254 |
| SU0116A-21mer | AACAAGCIIIICICICGCTCC | 21mer | P=S | 255 |
| SU0117A-21mer | CAACAAGCIIIICICICGCTC | 21mer | P=S | 256 |
| SU0118A-21mer | GCAACAAGCIIIICICICGCT | 21mer | P=S | 257 |

(2) Measurement of inhibitory activities of consensus oligonucleotides in expression of human CD14/luciferase fusion protein and production of mouse TNF$\alpha$:

According to Example 12, inhibitory activities of following oligonucleotides SU103A-21mer, SU0104A-21mer, SU0105A-21mer, SU0106A-21mer, SU0107A-21mer, SU0108A-21mer, SU0109A-21mer, SU0110A-21mer, SU0111A-21mer, SU0112A-21mer, SU0113A-21mer, SU0114A-21mer, SU0115A-21mer, SU0116A-21mer, SU0117-21mer and SU0118A-21mer in expression of CD14/luciferase fusion protein were compared using HeLa transformant cell expressing human CD14/luciferase fusion protein. Fig. 10 indicates the results. Consensus oligonucleotides exhibiting at least 40 % of inhibitory activity were SU0103A-21mer, SU0104A-21mer, SU0105A-21mer, SU0106A-21mer, SU0107A-21mer, SU0108A-21mer, SU0109A-21mer.

Next, inhibitory activities of SU0104A-21mer, SU0105A-21mer, SU0106A-21mer and SU0108A-21mer in mouse TNF$\alpha$ production were determined. The measurement was performed in accordance with the manner of Example 9, using RAW264.7 cell in stead of J774A.1 cell. TNF$\alpha$ in the culture supernatant was measured by mouse TNF$\alpha$ ELISA SYSTEM (manufactured by Amersham). Fig. 11 indicates the results.

Inhibitory activities of SU0104A-21mer, SU0105A-21mer, SU0106A-21mer and SU0108A-21mer in mouse TNF$\alpha$ production were 24 %, 33 %, 54 % and 69%, respectively. Control oligonucleotide indicated an inhibition of 3 %.

Based on the results, it was found that the oligonucleotides SU0104A-21mer, SU0105A-21mer, SU0106A-21mer and SU0108A-21mer work on mouse and human.

Industrial Application:

The present invention provides oligonucleotides containing sequences hybridized with a part of the gene encoding human CD14. Further, it provides pharmaceutical compositions comprising the oligonucleotide and pharmacologically acceptable carriers. By this, inflammatory factor can be effectively suppressed. In other words, the oligonucleotide inhibiting the human CD14 expression is useful as prophylactic/therapeutic agent against disorders caused by inflammatory factor induced via CD14, specifically such as system inflammatory reaction symptom, endotoxemia and endotoxic shock, ulcerative colitis, Crohn's disease, autoimmune response, allergy disease, cancer, graft-versus-host reaction, peritonitis, or osteoporosis.

List of sequences

Sequence No. 1

Sequence length: 1351

Sequence type: nucleic acid

Strand number: single-stranded

Topology: linear

Sequence variety: mRNA

Origin: human

Sequence

```
GAAGAGUUCA CAAGUGUGAA GCCUGGAAGC CGCCGGGUGC CGCUGUGUAG GAAAGAAGCU   60
AAAGCACUUC CAGAGCCUGU CCGGAGCUCA GAGGUUCGGA AGACUUAUCG ACCAUGGAGC  120
GCGCGUCCUG CUUGUUGCUG CUGCUGCUGC CGCUGGUGCA CGUCUCUGCG ACCACGCCAG  180
AACCUUGUGA GCUGGACGAU GAAGAUUUCC GCUGCGUCUG CAACUUCUCC GAACCUCAGC  240
CCGACUGGUC CGAAGCCUUC CAGUGUGUGU CUGCAGUAGA GGUGGAGAUC CAUGCCGGCG  300
GUCUCAACCU AGAGCCGUUU CUAAAGCGCG UCGAUGCGGA CGCCGACCCG CGGCAGUAUG  360
CUGACACGGU CAAGGCUCUC CGCGUGCGGC GGCUCACAGU GGGAGCCGCA CAGGUUCCUG  420
CUCAGCUACU GGUAGGCGCC CUGCGUGUGC UAGCGUACUC CCGCCUCAAG GAACUGACGC  480
UCGAGGACCU AAAGAUAACC GGCACCAUGC CUCCGCUGCC UCUGGAAGCC ACAGGACUUG  540
CACUUUCCAG CUUGCGCCUA CGCAACGUGU CGUGGGCGAC AGGGCGUUCU UGGCUCGCCC  600
AGCUGCAGCA GUGGCUCAAG CCAGGCCUCA AGGUACUGAG CAUUGCCCAA GCACACUCGC  660
```

CUGCCUUUUC CUACGAACAG GUUCGCGCCU UCCCGGCCCU UACCAGCCUA GACCUGUCUG 720

ACAAUCCUGG ACUGGGCGAA CGCGGACUGA UGGCGGCUCU CUGUCCCCAC AAGUUCCCGG 780

CCAUCCAGAA UCUAGCGCUG CGCAACACAG GAAUGGAGAC GCCCACAGGC GUGUGCGCCG 840

CACUGGCGGC GGCAGGUGUG CAGCCCCACA GCCUAGACCU CAGCCACAAC UCGCUGCGCG 900

CCACCGUAAA CCCUAGCGCU CCGAGAUGCA UGUGGUCCAG CGCCCUGAAC UCCCUCAAUC 960

UGUCGUUCGC UGGGCUGGAA CAGGUGCCUA AAGGACUGCC AGCCAAGCUC AGAGUGCUCG 1020

AUCUCAGCUG CAACAGACUG AACAGGGCGC CGCAGCCUGA CGAGCUGCCC GAGGUGGAUA 1080

ACCUGACACU GGACGGGAAU CCCUUCCUGG UCCCUGGAAC UGCCCUCCCC CACGAGGGCU 1140

CAAUGAACUC CGGCGUGGUC CCAGCCUGUG CACGUUCGAC CCUGUCGGUG GGGGUGUCGG 1200

GAACCCUGGU GCUGCUCCAA GGGGCCCGGG GCUUUGCCUA AGAUCCAAGA CAGAAUAAUG 1260

AAUGGACUCA AACUGCCUUG GCUUCAGGGG AGUCCCGUCA GGACGUUGAG GACUUUUCGA 1320

CCAAUUCAAC CCUUUGCCCC ACCUUUAUUA A 1351

Sequence No.: 2

Sequence length: 1570

Sequence type: nucleic acid

Strand number: double-stranded

Topology: linear

Sequence variety: genomic DNA

Origin: human

Sequence

CAGAATGACA TCCCAGGATT ACATAAACTG TCAGAGGCAG CCGAAGAGTT CACAAGTGTG 60
AAGCCTGGAA GCCGCCGGGT GCCGCTGTGT AGGAAAGAAG CTAAAGCACT TCCAGAGCCT 120
GTCCGGAGCT CAGAGGTTCG GAAGACTTAT CGACCATGGT GAGTGTAGGG TCTTGGGGTC 180
GAACGCGTGC CACTCGGGAG CCACAGGGGT TGGATGGGGC CTCCTAGACC TCTGCTCTCT 240
CCCCAGGAGC GCGCGTCCTG CTTGTTGCTG CTGCTGCTGC CGCTGGTGCA CGTCTCTGCG 300
ACCACGCCAG AACCTTGTGA GCTGGACGAT GAAGATTTCC GCTGCGTCTG CAACTTCTCC 360
GAACCTCAGC CCGACTGGTC CGAAGCCTTC CAGTGTGTGT CTGCAGTAGA GGTGGAGATC 420
CATGCCGGCG GTCTCAACCT AGAGCCGTTT CTAAAGCGCG TCGATGCGGA CGCCGACCCG 480
CGGCAGTATG CTGACACGGT CAAGGCTCTC CGCGTGCGGC GGCTCACAGT GGGAGCCGCA 540
CAGGTTCCTG CTCAGCTACT GGTAGGCGCC CTGCGTGTGC TAGCGTACTC CCGCCTCAAG 600
GAACTGACGC TCGAGGACCT AAAGATAACC GGCACCATGC CTCCGCTGCC TCTGGAAGCC 660
ACAGGACTTG CACTTTCCAG CTTGCGCCTA CGCAACGTGT CGTGGGCGAC AGGGCGTTCT 720
TGGCTCGCCG AGCTGCAGCA GTGGCTCAAG CCAGGCCTCA AGGTACTGAG CATTGCCCAA 780
GCACACTCGC CTGCCTTTTC CTACGAACAG GTTCGCGCCT TCCCGGCCCT TACCAGCCTA 840
GACCTGTCTG ACAATCCTGG ACTGGGCGAA CGCGGACTGA TGGCGGCTCT CTGTCCCCAC 900
AAGTTCCCGG CCATCCAGAA TCTAGCGCTG CGCAACACAG GAATGGAGAC GCCCACAGGC 960
GTGTGCGCCG CACTGGCGGC GGCAGGTGTG CAGCCCCACA GCCTAGACCT CAGCCACAAC 1020
TCGCTGCGCG CCACCGTAAA CCCTAGCGCT CCGAGATGCA TGTGGTCCAG CGCCCTGAAC 1080
TCCCTCAATC TGTCGTTCGC TGGGCTGGAA CAGGTGCCTA AAGGACTGCC AGCCAAGCTC 1140
AGAGTGCTCC ATCTCAGCTG CAACAGACTG AACAGGGCGC CGCAGCCTGA CGAGCTGCCC 1200
GAGGTGGATA ACCTGACACT GGACGGGAAT CCCTTCCTGG TCCCTGGAAC TGCCCTCCCC 1260
CACGAGGGCT CAATGAACTC CGGCCGTGGTC CCAGCCTGTG CACGTTCGAC CCTGTCGGTG 1320

GGGGTGTCGG GAACCCTGGT GCTGCTCCAA GGGGCCCGGG GCTTTGCCTA AGATCCAAGA 1380

CAGAATAATG AATGGACTCA AACTGCCTTG GCTTCAGGGG AGTCCCGTCA GGACGTTGAG 1440

GACTTTTCGA CCAATTCAAC CCTTTGCCCC ACCTTTATTA AAATCTTAAA CAACGGTTCC 1500

GTGTCATTCA TTTAACAGAC CTTTATTGGA TGTCTGCTAT GTGCTGGGCA CAGTACTGGA 1560

TGGGGAATTC 1570

Sequence No.: 3

Sequence length: 1447

Sequence type: nucleic acid

Strand number: single-stranded

Topology: linear

Sequence variety: mRNA

Origin: mouse

Sequence

CGAACAAGCC CGUGGAACCU GGAAGCCAGA GAACACCACC GCUGUAAAGG AAAGAAACUG 60

AAGCCUUUCU CGGAGCCUAU CUGGGCUGCU CAAACUUUCA GAAUCUACCG ACCAUGGAGC 120

GCUGCUUGG CUUGUUGCUG UUGCUUCUGG UGCACGCCUC UCCCGCCCCA CCAGAGCCCU 180

GCCGAGCUAGA CGAGGAAAGU UGUUCCUGCA ACUUCUCAGA UCCGAAGCCA GAUUGGUCCA 240

GCCCUUUCAA UUGUUUGGGG GCGGCAGAUG UGGAAUUGUA CGGCGGCGGC CGCAGCCUGG 300

AAUACCUUCU AAAGCGUGUG GACACGGAAG CAGAUCUGGG GCAGUUCACU GAUAUUAUCA 360

AGUCUCUGUC CUUAAAGCGG CUUACGGUGC GGGCCGCGCG GAUUCCUAGU CGGAUUCUAU 420

UCGGAGCCCU GCGUGUGCUC GGGAUUUCCG GCCUCCAGGA ACUGACUCUU GAAAAUCUCG 480
AGGUAACCGG CACCGCGCCG CCACCGCUUC UGGAAGCCAC CGGACCCGAU CUCAACAUCU 540
UGAACCUCCG CAACGUGUCG UGGGCAACAA GGGAUGCCUG GCUCGCAGAA CUGCAGCAGU 600
GGCUAAAGCC UGGACUCAAG GUACUGAGUA UUGCCCAAGC ACACUCACUC AACUUUUCCU 660
GCGAACAGGU CCGCGUCUUC CCUGCCCUCU CCACCUUAGA CCUGUCUGAC AAUCCUGAAU 720
UGGGCGAGAG AGGACUGAUC UCAGCCCUCU GUCCCCUCAA GUUCCCGACC CUCCAAGUUU 780
UAGCGCUGCG UAACGCGGGG AUGGAGACGC CCAGCGGCGU GUGCUCUGCG CUGGCCGCAG 840
CAAGGGUACA GCUGCAAGGA CUAGACCUUA GUCACAAUUC ACUGCGGGAU GCUGCAGGCG 900
CUCCGAGUUG UGACUGGCCC AGUCAGCUAA ACUCGCUCAA UCUGUCUUUC ACUGGGCUGA 960
AGCAGGUACC UAAAGGGCUG CCAGCCAAGC UCAGCGUGCU GGAUCUCAGU UACAACAGGC 1020
UGGAUAGCAA CCCUAGCCCA GAUGAGCUGC CCCAAGUGGG GAACCUGUCA CUUAAAGGAA 1080
AUCCCUUUUU GGACUCUGAA UCCCACUCGG AGAAGUUUAA CUCUGGCGUA GUCACCGCCG 1140
GAGCUCCAUC AUCCCAAGCA GUGGCCUUGU CAGGAACUCU GGCUUUGCUC CUAGGAGAUC 1200
GCCUCUUUGU UUAAGGAACA UUUGCAUCCU CCUGGUUUCU GAGGGUCCUC GUCAACGAAU 1260
CCUCUGCUUU AAAUUUAUUA AAAUCUUAAU CCACGAUGUA AGGAAAGAAA GGCAGUCAAG 1320
AUGGUUCAGU GGGUAAAAGC CAGCAAACUU GACCCCUGAU UUUAACCCUC AGGAUCCACA 1380
CGGAAGGGGA AAACUCACUC CUGAAAGUUG UCCAUCUGUG CUCACAAAUA AAUAUUUUUU 1440
AAAAUAA 1447

Sequence No.: 4

Sequence length: 2404

Sequence type: nucleic acid

Strand number: double-stranded

Topology: linear

Sequence variety: genomic DNA

Origin: mouse

Sequence

```
CCTAGCATTT GGGAGCCAGA GGCAGGAGGA AAATCATGCG TTTCAGGCTA GGCTAGATTG   60
GGTTACTAGA CTGAGATATC ATGGGGAGAA TGGAGAGGTA GAGACTGGGA GAAGAATGAA  120
TTAATAAAGA ACTGAATAAG ATGGGAAGAA GGGAGAATTA TTTTTCATAT TAACTCTCAA  180
CTTTGAGCTT TATTCTCTGC CTGGAATCTA TAGATAAGTT CACAATCTTT CCACAAATGT  240
CCAATTACAT TCAAAGAAAA TCAAGAGCTG GATTTGAACG GTGGGAAATT GCTAGCAACT  300
AAGACTAGGG GAAATGGAGG TGAATCAATG GGACTGAGCA ACAGAATAAT GATCTAAGGC  360
ACTAGCTGTG ATTCACTCTT TTCCTGTACG CACCAGACAA GTCCGGGGCT CATAGGTCAT  420
CCTCCTGGCA CAGAATGCCC TAATGCCACT CTGAATTCTT CCTGTTTTTC GTCCCTCCCT  480
AAAAAACACT TCCTTGCAAT ATTTACTAGA AGTGAGTAGG GCTGTTAGGA GGAAGAGAAG  540
TGGAGACGCA ATTAGAATTC ACAGAGGAAG GGACAGGGTG ACACCCCAGG ATTACATAAA  600
TTTACAGGGG CTGCCGAATT GGTCGAACAA GCCCGTGGAA CCTGGAAGCC AGAGAACACC  660
ATCGCTGTAA AGGAAAGAAA CTGAAGCTTT TCTCGGAGCC TATCTGGGCT GCTCAAACTT  720
TCAGAATCTA CCGACCATGG TGAGTCAGAC AGACTGTCTT GGGGTGGAAC TGGAGCCAAC  780
CTGAGGAATC TCAGGGTCTG GCAGGAGTCT CCCTGACCCC TACTTTCTCC TCAGGAGCGT  840
GTGCTTGGCT TGTTGCTGTT GCTTCTGGTG CACGCCTCTC CCGCCCCACC AGAGCCCTGC  900
GAGCTAGACG AGGAAAGTTG TTCCTGCAAC TTCTCAGATC CGAAGCCAGA TTGGTCCAGC  960
```

GCTTTCAATT GTTTGGGGGC GGCAGATGTG GAATTGTACG GCGGCGGCCG CAGCCTGGAA 1020
TACCTTCTAA AGCGTGTGGA CACGGAAGCA GATCTGGGGC AGTTCACTGA TATTATCAAG 1080
TCTCTGTCCT TAAAGCGGCT TACGGTGCGG GCCGCGCGGA TTCCTAGTCG GATTCTATTC 1140
GGAGCCCTGC GTGTCCTCGG GATTTCGGGC CTCCAGGAAC TGACTCTTGA AAATCTCGAG 1200
GTAACCGGCA CCGCGCCGCC ACCGCTTCTG GAAGCCACCG GACCCGATCT CAACATCTTG 1260
AACCTCCGCA ACGTGTCGTG GGCAACAAGG GATGCCTGGC TCGCAGAACT GCAGCAGTGG 1320
CTAAAGCCTG GACTCAAGGT ACTGAGTATT GCCCAAGCAC ACTCACTCAA CTTTTCCTGC 1380
GAACAGGTCC GCGTCTTCCC TGCCCTCTCC ACCTTAGACC TGTCTGACAA TCCTGAATTG 1440
GGCGAGAGAG GACTGATCTC AGCCCTCTGT CCCCTCAAGT TCCCGACCCT CCAAGTTTTA 1500
GCGCTGCGTA ACGCGGGGAT GGAGACGCCC AGCGGCGTGT GCTCTGCGCT GGCCGCAGCA 1560
AGGGTACAGC TGCAAGGACT AGACCTTAGT CACAATTCAC TGCGGGATGC TGCAGGCGCT 1620
CCGAGTTGTG ACTGGCCCAG TCAGCTAAAC TCGCTCAATC TGTCTTTCAC TGGGCTGAAG 1680
CAGGTACCTA AAGGGCTGCC AGCCAAGCTC AGCGTGCTGG ATCTCAGTTA CAACAGGCTG 1740
GATAGGAACC CTAGCCCAGA TGAGCTGCCC CAAGTGGGGA ACCTGTCACT TAAAGGAAAT 1800
CCCTTTTTGG ACTCTGAATC CCACTCGGAG AAGTTTAACT CTGGCGTAGT CACCGCCGGA 1860
GCTCCATCAT CCCAAGCAGT GGCCTTGTCA GGAACTCTGG CTTTGCTCCT AGGAGATCGC 1920
CTCTTTGTTT AAGGAACATT TGCATCCTCC TGGTTTCTGA GGGTCCTCGT CAACGAATCC 1980
TCTGCTTTAA ATTTATTAAA ATCTTAATCC ACGATGTAAG GAAAGAAACG CAGTCAAGAT 2040
GGTTCAGTGG GTAAAAGCCA GCAAACTTGA CCCCTGATTT TAACCCTCAG GATCCACACG 2100
GAAGGGGAAA ACTCACTCCT GAAAGTTGTC CATCTGTGCT CACAAATAAA TATTTTTTAA 2160
AATAACAATG TGTTTGTTGG TTTTGTTTTT GTTTGGGTTT TGTTGTGGTT TTGTTTGTTT 2220
TGTTTTGTTT TTGAGACAGT CTGGCTATGT ATCCTTGGCT GGCCTCAAAC TCATAAAGAT 2280

CAAGATCGGC CTGCCTCTAC CTCCAAATGC TCTGGTTAAA GGGATGTGCC TCCATGCCCA 2340

GTTGAAGTCA TCCTGAACCA CGAGTCCAGG CCACTCACTC TTTACTAAGA TCTTTACTAA 2400

GTAT 2404

Sequence No.: 5

Sequence length: 32

Sequence type: nucleic acid

Strand number: single-stranded

Topology: linear

Sequence variety: other nucleic acid, synthetic DNA

Sequence

ACGCGTCGAC GAGTTCACAA GTGTGAAGCC TG 32

Sequence No.: 6

Sequence length: 32

Sequence type: nucleic acid

Strand number: single-stranded

Topology: linear

Sequence variety: other nucleic acid, synthetic DNA

Sequence

ACATGCATGC TTAATAAAGG TGGGGCAAAG GG 32

Sequence No.: 7

Sequence length: 33

Sequence type: nucleic acid

Strand number: single-stranded

Topology: linear

Sequence variety: other nucleic acid, synthetic DNA

Sequence

CCCAAGCTTA AGTGTGAAGC CTGAAGCCGC CGG 33

Sequence No.: 8

Sequence length: 44

Sequence type: nucleic acid

Strand number: single-stranded

Topology: linear

Sequence variety: other nucleic acid, synthetic DNA

Sequence

ATGGCGCCGG GCCTTTCTTT ATGTTTTTGG CGTCTTCCAG TTGG 44

Sequence No.: 9

Sequence length: 20

Sequence type: nucleic acid

Strand number: single-stranded

Topology: linear

Sequence variety: other nucleic acid, synthetic DNA

Sequence

CGGCTTCCAG GCTTCACACT 20

Sequence No.: 10

Sequence length: 20

Sequence type: nucleic acid

Strand number: single-stranded

Topology: linear

Sequence variety: other nucleic acid, synthetic DNA

Sequence

CGGCACCCGG CGGCTTCCAG 20

Sequence No.: 11

Sequence length: 20

Sequence type: nucleic acid

Strand number: single-stranded

Topology: linear

Sequence variety: other nucleic acid, synthetic DNA

Sequence

TCCTACACAG CGGCACCCGG 20

Sequence No.: 12

Sequence length: 20

Sequence type: nucleic acid

Strand number: single-stranded

Topology: linear

Sequence variety: other nucleic acid, synthetic DNA

Sequence

TTCTTTCCTA CACAGCGGCA 20

Sequence No.: 13

Sequence length: 20

Sequence type: nucleic acid

Strand number: single-stranded

Topology: linear

Sequence variety: other nucleic acid, synthetic DNA

Sequence

TTAGCTTCTT TCCTACACAG 20

Sequence No.: 14

Sequence length: 20

Sequence type: nucleic acid

Strand number: single-stranded

Topology: linear

Sequence variety: other nucleic acid, synthetic DNA

Sequence

GTGCTTTAGC TTCTTTCCTA 20

Sequence No.: 15

Sequence length: 20

Sequence type: nucleic acid

Strand number: single-stranded

Topology: linear

Sequence variety: other nucleic acid, synthetic DNA

Sequence

TGGAAGTGCT TTAGCTTCTT 20

Sequence No.: 16

Sequence length: 20

Sequence type: nucleic acid

Strand number: single-stranded

Topology: linear

Sequence variety: other nucleic acid, synthetic DNA

Sequence

GGACAGGCTC TGGAAGTGCT 20

Sequence No.: 17

Sequence length: 20

Sequence type: nucleic acid

Strand number: single-stranded

Topology: linear

Sequence variety: other nucleic acid, synthetic DNA

Sequence

TCTGAGCTCC GGACAGGCTC 20

Sequence No.: 18

Sequence length: 20

Sequence type: nucleic acid

Strand number: single-stranded

Topology: linear

Sequence variety: other nucleic acid, synthetic DNA

Sequence

CTTCCGAACC TCTGAGCTCC 20

Sequence No.: 19

Sequence length: 20

Sequence type: nucleic acid

Strand number: single-stranded

Topology: linear

Sequence variety: other nucleic acid, synthetic DNA

Sequence

GTCGATAAGT CTTCCGAACC 20

Sequence No.: 20

Sequence length: 20

Sequence type: nucleic acid

Strand number: single-stranded

Topology: linear

Sequence variety: other nucleic acid, synthetic DNA

Sequence

ATGGTCGATA AGTCTTCCGA 20

Sequence No.: 21

Sequence length: 20

Sequence type: nucleic acid

Strand number: single-stranded

Topology: linear

Sequence variety: other nucleic acid, synthetic DNA

Sequence

TCCATGGTCG ATAAGTCTTC 20

Sequence No.: 22

Sequence length: 20

Sequence type: nucleic acid

Strand number: single-stranded

Topology: linear

Sequence variety: other nucleic acid, synthetic DNA

Sequence

CGCTCCATGG TCGATAAGTC 20

Sequence No.: 23

Sequence length: 20

Sequence type: nucleic acid

Strand number: single-stranded

Topology: linear

Sequence variety: other nucleic acid, synthetic DNA

Sequence

CGCGCTCCAT GGTCGATAAG 20

Sequence No.: 24

Sequence length: 20

Sequence type: nucleic acid

Strand number: single-stranded

Topology: linear

Sequence variety: other nucleic acid, synthetic DNA

Sequence

GCGCGCTCCA TGGTCGATAA 20

Sequence No.: 25

Sequence length: 20

Sequence type: nucleic acid

Strand number: single-stranded

Topology: linear

Sequence variety: other nucleic acid, synthetic DNA

Sequence

CGCGCGCTCC ATGGTCGATA 20

Sequence No.: 26

Sequence length: 20

Sequence type: nucleic acid

Strand number: single-stranded

Topology: linear

Sequence variety: other nucleic acid, synthetic DNA

Sequence

ACGCGCGCTC CATGGTCGAT 20

Sequence No.: 27

Sequence length: 20

Sequence type: nucleic acid

Strand number: single-stranded

Topology: linear

Sequence variety: other nucleic acid, synthetic DNA

Sequence

GACGCGCGCT CCATGGTCGA 20

Sequence No.: 28

Sequence length: 20

Sequence type: nucleic acid

Strand number: single-stranded

Topology: linear

Sequence variety: other nucleic acid, synthetic DNA

Sequence

GGACGCGCGC TCCATGGTCG 20

Sequence No.: 29

Sequence length: 20

Sequence type: nucleic acid

Strand number: single-stranded

Topology: linear

Sequence variety: other nucleic acid, synthetic DNA

Sequence

GCAGGACGCG CGCTCCATGG 20

Sequence No.: 30

Sequence length: 20

Sequence type: nucleic acid

Strand number: single-stranded

Topology: linear

Sequence variety: other nucleic acid, synthetic DNA

Sequence

AAGCAGGACG CGCGCTCCAT 20

Sequence No.: 31

Sequence length: 20

Sequence type: nucleic acid

Strand number: single-stranded

Topology: linear

Sequence variety: other nucleic acid, synthetic DNA

Sequence

ACAAGCAGGA CGCGCGCTCC 20

Sequence No.: 32

Sequence length: 20

Sequence type: nucleic acid

Strand number: single-stranded

Topology: linear

Sequence variety: other nucleic acid, synthetic DNA

Sequence

AACAAGCAGG ACGCGCGCTC 20

Sequence No.: 33

Sequence length: 20

Sequence type: nucleic acid

Strand number: single-stranded

Topology: linear

Sequence variety: other nucleic acid, synthetic DNA

Sequence

CAACAAGCAG GACGCGCGCT 20

Sequence No.: 34

Sequence length: 20

Sequence type: nucleic acid

Strand number: single-stranded

Topology: linear

Sequence variety: other nucleic acid, synthetic DNA

Sequence

AGCAACAAGC AGGACGCGCG 20

Sequence No.: 35

Sequence length: 20

Sequence type: nucleic acid

Strand number: single-stranded

Topology: linear

Sequence variety: other nucleic acid, synthetic DNA

Sequence

GCAGCAACAA GCAGGACGCG 20

Sequence No.: 36

Sequence length: 20

Sequence type: nucleic acid

Strand number: single-stranded

Topology: linear

Sequence variety: other nucleic acid, synthetic DNA

Sequence

CAGCAGCAAC AAGCAGGACG 20

Sequence No.: 37

Sequence length: 20

Sequence type: nucleic acid

Strand number: single-stranded

Topology: linear

Sequence variety: other nucleic acid, synthetic DNA

Sequence

AGCAGCAGCA ACAAGCAGGA 20

Sequence No.: 38

Sequence length: 20

Sequence type: nucleic acid

Strand number: single-stranded

Topology: linear

Sequence variety: other nucleic acid, synthetic DNA

Sequence

TCTTGGATCT TAGGCAAAGC 20

Sequence No.: 39

Sequence length: 20

Sequence type: nucleic acid

Strand number: single-stranded

Topology: linear

Sequence variety: other nucleic acid, synthetic DNA

Sequence

CATTATTCTG TCTTGGATCT 20

Sequence No.: 40

Sequence length: 20

Sequence type: nucleic acid

Strand number: single-stranded

Topology: linear

Sequence variety: other nucleic acid, synthetic DNA

Sequence

CAGTTTGAGT CCATTCATTA 20

Sequence No.: 41

Sequence length: 20

Sequence type: nucleic acid

Strand number: single-stranded

Topology: linear

Sequence variety: other nucleic acid, synthetic DNA

Sequence

AGGCAGTTTG AGTCCATTCA 20

Sequence No.: 42

Sequence length: 20

Sequence type: nucleic acid

Strand number: single-stranded

Topology: linear

Sequence variety: other nucleic acid, synthetic DNA

Sequence

CAAGGCAGTT TGAGTCCATT 20

Sequence No.: 43

Sequence length: 20

Sequence type: nucleic acid

Strand number: single-stranded

Topology: linear

Sequence variety: other nucleic acid, synthetic DNA

Sequence

CCAAGGCAGT TTGAGTCCAT 20

Sequence No.: 44

Sequence length: 20

Sequence type: nucleic acid

Strand number: single-stranded

Topology: linear

Sequence variety: other nucleic acid, synthetic DNA

Sequence

GCCAAGGCAG TTTGAGTCCA 20

Sequence No.: 45

Sequence length: 20

Sequence type: nucleic acid

Strand number: single-stranded

Topology: linear

Sequence variety: other nucleic acid, synthetic DNA

Sequence

AGCCAAGGCA GTTTGAGTCC 20

Sequence No.: 46

Sequence length: 20

Sequence type: nucleic acid

Strand number: single-stranded

Topology: linear

Sequence variety: other nucleic acid, synthetic DNA

Sequence

AAGCCAAGGC AGTTTGAGTC 20

Sequence No.: 47

Sequence length: 20

Sequence type: nucleic acid

Strand number: single-stranded

Topology: linear

Sequence variety: other nucleic acid, synthetic DNA

Sequence

GAAGCCAAGG CAGTTTGAGT 20

Sequence No.: 48

Sequence length: 20

Sequence type: nucleic acid

Strand number: single-stranded

Topology: linear

Sequence variety: other nucleic acid, synthetic DNA

Sequence

TGAAGCCAAG GCAGTTTGAG 20

Sequence No.: 49

Sequence length: 20

Sequence type: nucleic acid

Strand number: single-stranded

Topology: linear

Sequence variety: other nucleic acid, synthetic DNA

Sequence

CTGAAGCCAA GGCACTTTGA 20

Sequence No.: 50

Sequence length: 20

Sequence type: nucleic acid

Strand number: single-stranded

Topology: linear

Sequence variety: other nucleic acid, synthetic DNA

Sequence

CCTGAAGCCA AGGCACTTTG 20

Sequence No.: 51

Sequence length: 20

Sequence type: nucleic acid

Strand number: single-stranded

Topology: linear

Sequence variety: other nucleic acid, synthetic DNA

Sequence

CCCTGAAGCC AAGGCACTTT 20

Sequence No.: 52

Sequence length: 20

Sequence type: nucleic acid

Strand number: single-stranded

Topology: linear

Sequence variety: other nucleic acid, synthetic DNA

Sequence

CCCCTGAAGC CAAGGCACTT 20

Sequence No.: 53

Sequence length: 20

Sequence type: nucleic acid

Strand number: single-stranded

Topology: linear

Sequence variety: other nucleic acid, synthetic DNA

Sequence

CTCCCCTGAA GCCAAGGCAG 20

Sequence No.: 54

Sequence length: 20

Sequence type: nucleic acid

Strand number: single-stranded

Topology: linear

Sequence variety: other nucleic acid, synthetic DNA

Sequence

GGACTCCCCT GAAGCCAAGG 20

Sequence No.: 55

Sequence length: 20

Sequence type: nucleic acid

Strand number: single-stranded

Topology: linear

Sequence variety: other nucleic acid, synthetic DNA

Sequence

TGACGGGACT CCCCTGAAGC 20

Sequence No.: 56

Sequence length: 20

Sequence type: nucleic acid

Strand number: single-stranded

Topology: linear

Sequence variety: other nucleic acid, synthetic DNA

Sequence

CTCAACGTCC TGACGGGACT 20

Sequence No.: 57

Sequence length: 20

Sequence type: nucleic acid

Strand number: single-stranded

Topology: linear

Sequence variety: other nucleic acid, synthetic DNA

Sequence

TCGAAAAGTC CTCAACGTCC 20

Sequence No.: 58

Sequence length: 20

Sequence type: nucleic acid

Strand number: single-stranded

Topology: linear

Sequence variety: other nucleic acid, synthetic DNA

Sequence

GTTGAATTGG TCGAAAAGTC 20

Sequence No.: 59

Sequence length: 20

Sequence type: nucleic acid

Strand number: single-stranded

Topology: linear

Sequence variety: other nucleic acid, synthetic DNA

Sequence

TAATAAAGGT GGGGCAAAGG 20

Sequence No.: 60

Sequence length: 20

Sequence type: nucleic acid

Strand number: single-stranded

Topology: linear

Sequence variety: other nucleic acid, synthetic DNA

Sequence

CGGCTTCCAG GCTTCACACT 20

Sequence No.: 61

Sequence length: 20

Sequence type: nucleic acid

Strand number: single-stranded

Topology: linear

Sequence variety: other nucleic acid, synthetic DNA

Sequence

CGGCACCCGG CGGCTTCCAG 20

Sequence No.: 62

Sequence length: 20

Sequence type: nucleic acid

Strand number: single-stranded

Topology: linear

Sequence variety: other nucleic acid, synthetic DNA

Sequence

TCCTACACAG CGGCACCCGG 20

Sequence No.: 63

Sequence length: 20

Sequence type: nucleic acid

Strand number: single-stranded

Topology: linear

Sequence variety: other nucleic acid, synthetic DNA

Sequence

TTAGCTTCTT TCCTACACAG 20

Sequence No.: 64

Sequence length: 20

Sequence type: nucleic acid

Strand number: single-stranded

Topology: linear

Sequence variety: other nucleic acid, synthetic DNA

Sequence

TGGAAGTGCT TTAGCTTCTT 20

Sequence No.: 65

Sequence length: 20

Sequence type: nucleic acid

Strand number: single-stranded

Topology: linear

Sequence variety: other nucleic acid, synthetic DNA

Sequence

GGACAGGCTC TGGAAGTGCT 20

Sequence No.: 66

Sequence length: 20

Sequence type: nucleic acid

Strand number: single-stranded

Topology: linear

Sequence variety: other nucleic acid, synthetic DNA

Sequence

TCTGAGCTCC GGACAGGCTC 20

Sequence No.: 67

Sequence length: 20

Sequence type: nucleic acid

Strand number: single-stranded

Topology: linear

Sequence variety: other nucleic acid, synthetic DNA

Sequence

CTTCCGAACC TCTGAGCTCC 20

Sequence No.: 68

Sequence length: 20

Sequence type: nucleic acid

Strand number: single-stranded

Topology: linear

Sequence variety: other nucleic acid, synthetic DNA

Sequence

GTCGATAAGT CTTCCGAACC 20

Sequence No.: 69

Sequence length: 20

Sequence type: nucleic acid

Strand number: single-stranded

Topology: linear

Sequence variety: other nucleic acid, synthetic DNA

Sequence

ATGCTCGATA AGTCTTCCGA 20

Sequence No.: 70

Sequence length: 20

Sequence type: nucleic acid

Strand number: single-stranded

Topology: linear

Sequence variety: other nucleic acid, synthetic DNA

Sequence

TCCATGCTCG ATAAGTCTTC 20

Sequence No.: 71

Sequence length: 20

Sequence type: nucleic acid

Strand number: single-stranded

Topology: linear

Sequence variety: other nucleic acid, synthetic DNA

Sequence

CGCTCCATGG TCGATAAGTC 20

Sequence No.: 72

Sequence length: 20

Sequence type: nucleic acid

Strand number: single-stranded

Topology: linear

Sequence variety: other nucleic acid, synthetic DNA

Sequence

GCGCTCCATG GTCGATAAGT 20

Sequence No.: 73

Sequence length: 20

Sequence type: nucleic acid

Strand number: single-stranded

Topology: linear

Sequence variety: other nucleic acid, synthetic DNA

Sequence

CGCGCTCCAT GGTCGATAAC 20

Sequence No.: 74

Sequence length: 20

Sequence type: nucleic acid

Strand number: single-stranded

Topology: linear

Sequence variety: other nucleic acid, synthetic DNA

Sequence

GCGCGCTCCA TGGTCGATAA 20

Sequence No.: 75

Sequence length: 20

Sequence type: nucleic acid

Strand number: single-stranded

Topology: linear

Sequence variety: other nucleic acid, synthetic DNA

Sequence

CGCGCGCTCC ATGGTCGATA 20

Sequence No.: 76

Sequence length: 20

Sequence type: nucleic acid

Strand number: single-stranded

Topology: linear

Sequence variety: other nucleic acid, synthetic DNA

Sequence

ACGCGCGCTC CATGGTCGAT 20

Sequence No.: 77

Sequence length: 20

Sequence type: nucleic acid

Strand number: single-stranded

Topology: linear

Sequence variety: other nucleic acid, synthetic DNA

Sequence

GACGCGCGCT CCATGGTCGA 20

Sequence No.: 78

Sequence length: 20

Sequence type: nucleic acid

Strand number: single-stranded

Topology: linear

Sequence variety: other nucleic acid, synthetic DNA

Sequence

GGACGCGCGC TCCATGGTCG 20

Sequence No.: 79

Sequence length: 20

Sequence type: nucleic acid

Strand number: single-stranded

Topology: linear

Sequence variety: other nucleic acid, synthetic DNA

Sequence

AGGACCCGCG CTCCATGGTC 20

Sequence No.: 80

Sequence length: 20

Sequence type: nucleic acid

Strand number: single-stranded

Topology: linear

Sequence variety: other nucleic acid, synthetic DNA

Sequence

CAGGACGCGC GCTCCATGGT 20

Sequence No.: 81

Sequence length: 20

Sequence type: nucleic acid

Strand number: single-stranded

Topology: linear

Sequence variety: other nucleic acid, synthetic DNA

Sequence

GCAGGACGCG CGCTCCATGG 20

Sequence No.: 82

Sequence length: 20

Sequence type: nucleic acid

Strand number: single-stranded

Topology: linear

Sequence variety: other nucleic acid, synthetic DNA

Sequence

AGCAGGACGC GCGCTCCATG 20

Sequence No.: 83

Sequence length: 20

Sequence type: nucleic acid

Strand number: single-stranded

Topology: linear

Sequence variety: other nucleic acid, synthetic DNA

Sequence

AAGCAGGACG CGCGCTCCAT 20

Sequence No.: 84

Sequence length: 20

Sequence type: nucleic acid

Strand number: single-stranded

Topology: linear

Sequence variety: other nucleic acid, synthetic DNA

Sequence

CAACAAGCAG GACGCGCGCT 20

Sequence No.: 85

Sequence length: 15

Sequence type: nucleic acid

Strand number: single-stranded

Topology: linear

Sequence variety: other nucleic acid, synthetic DNA

Sequence

CATGGTCGAT AACTC 15

Sequence No.: 86

Sequence length: 18

Sequence type: nucleic acid

Strand number: single-stranded

Topology: linear

Sequence variety: other nucleic acid, synthetic DNA

Sequence

CTCCATGGTC GATAACTC 18

Sequence No.: 87

Sequence length: 19

Sequence type: nucleic acid

Strand number: single-stranded

Topology: linear

Sequence variety: other nucleic acid, synthetic DNA

Sequence

GCTCCATGGT CGATAACTC 19

Sequence No.: 88

Sequence length: 21

Sequence type: nucleic acid

Strand number: single-stranded

Topology: linear

Sequence variety: other nucleic acid, synthetic DNA

Sequence

GCGCTCCATG GTCGATAAGT C 21

Sequence No.: 89

Sequence length: 22

Sequence type: nucleic acid

Strand number: single-stranded

Topology: linear

Sequence variety: other nucleic acid, synthetic DNA

Sequence

CGCGCTCCAT GGTCGATAAG TC 22

Sequence No.: 90

Sequence length: 25

Sequence type: nucleic acid

Strand number: single-stranded

Topology: linear

Sequence variety: other nucleic acid, synthetic DNA

Sequence

ACGCGCGCTC CATGGTCGAT AAGTC 25

Sequence No.: 91

Sequence length: 20

Sequence type: nucleic acid

Strand number: single-stranded

Topology: linear

Sequence variety: other nucleic acid, synthetic DNA

Sequence

CATGGTCGCT AGATTCTGAA 20

Sequence No.: 92

Sequence length: 25

Sequence type: nucleic acid

Strand number: single-stranded

Topology: linear

Sequence variety: other nucleic acid, synthetic DNA

Sequence

CACACGCTCC ATGGTCGGTA GATTC 25

Sequence No.: 93

Sequence length: 25

Sequence type: nucleic acid

Strand number: single-stranded

Topology: linear

Sequence variety: other nucleic acid, synthetic DNA

Sequence

GCACACGCTC CATGCTCGCT AGATT 25

Sequence No.: 94

Sequence length: 25

Sequence type: nucleic acid

Strand number: single-stranded

Topology: linear

Sequence variety: other nucleic acid, synthetic DNA

Sequence

AGCACACGCT CCATGGTCGG TAGAT 25

Sequence No.: 95

Sequence length: 25

Sequence type: nucleic acid

Strand number: single-stranded

Topology: linear

Sequence variety: other nucleic acid, synthetic DNA

Sequence

AAGCACACGC TCCATGGTCG GTAGA 25

Sequence No.: 96

Sequence length: 25

Sequence type: nucleic acid

Strand number: single-stranded

Topology: linear

Sequence variety: other nucleic acid, synthetic DNA

Sequence

CAAGCACACG CTCCATGGTC GGTAG 25

Sequence No.: 97

Sequence length: 25

Sequence type: nucleic acid

Strand number: single-stranded

Topology: linear

Sequence variety: other nucleic acid, synthetic DNA

Sequence

CCAAGCACAC GCTCCATGGT CGGTA 25

Sequence No.: 98

Sequence length: 25

Sequence type: nucleic acid

Strand number: single-stranded

Topology: linear

Sequence variety: other nucleic acid, synthetic DNA

Sequence

GCCAAGCACA CGCTCCATGG TCGGT 25

Sequence No.: 99

Sequence length: 25

Sequence type: nucleic acid

Strand number: single-stranded

Topology: linear

Sequence variety: other nucleic acid, synthetic DNA

Sequence

AAGCCAAGCA CACGCTCCAT GGTCG 25

Sequence No.: 100

Sequence length: 25

Sequence type: nucleic acid

Strand number: single-stranded

Topology: linear

Sequence variety: other nucleic acid, synthetic DNA

Sequence

ACAAGCCAAG CACACGCTCC ATGGT 25

Sequence No.: 101

Sequence length: 15

Sequence type: nucleic acid

Strand number: single-stranded

Topology: linear

Sequence variety: other nucleic acid

Sequence

GAACCTCTGA GCTCC 15

Sequence No.: 102

Sequence length: 15

Sequence type: nucleic acid

Strand number: single-stranded

Topology: linear

Sequence variety: other nucleic acid

Sequence

TCCATGGTCG ATAAG 15

Sequence No.: 103

Sequence length: 15

Sequence type: nucleic acid

Strand number: single-stranded

Topology: linear

Sequence variety: other nucleic acid

Sequence

GGACGCGCGC TCCAT 15

Sequence No.: 104

Sequence length: 15

Sequence type: nucleic acid

Strand number: single-stranded

Topology: linear

Sequence variety: other nucleic acid

Sequence

AGCAGCAGCA GCAAC 15

Sequence No.: 105

Sequence length: 15

Sequence type: nucleic acid

Strand number: single-stranded

Topology: linear

Sequence variety: other nucleic acid

Sequence

CACCAGCGGC AGCAG 15

Sequence No.: 106

Sequence length: 15

Sequence type: nucleic acid

Strand number: single-stranded

Topology: linear

Sequence variety: other nucleic acid

Sequence

CAGAGACGTG CACCA 15

Sequence No.: 107

Sequence length: 15

Sequence type: nucleic acid

Strand number: single-stranded

Topology: linear

Sequence variety: other nucleic acid

Sequence

GGCGTGGTCG CAGAG 15

Sequence No.: 108

Sequence length: 15

Sequence type: nucleic acid

Strand number: single-stranded

Topology: linear

Sequence variety: other nucleic acid

Sequence

ACAAGGTTCT GGCGT 15

Sequence No.: 109

Sequence length: 15

Sequence type: nucleic acid

Strand number: single-stranded

Topology: linear

Sequence variety: other nucleic acid

Sequence

CGTCCAGCTC ACAAG 15

Sequence No.: 110

Sequence length: 15

Sequence type: nucleic acid

Strand number: single-stranded

Topology: linear

Sequence variety: other nucleic acid

Sequence

AAATCTTCAT CGTCC 15

Sequence No.: 111

Sequence length: 15

Sequence type: nucleic acid

Strand number: single-stranded

Topology: linear

Sequence variety: other nucleic acid

Sequence

GACGCAGCGG AAATC 15

Sequence No.: 112

Sequence length: 15

Sequence type: nucleic acid

Strand number: single-stranded

Topology: linear

Sequence variety: other nucleic acid

Sequence

AGAAGTTGCA GACGC 15

Sequence No.: 113

Sequence length: 15

Sequence type: nucleic acid

Strand number: single-stranded

Topology: linear

Sequence variety: other nucleic acid

Sequence

TGAGGTTCGG AGAAG 15

Sequence No.: 114

Sequence length: 15

Sequence type: nucleic acid

Strand number: single-stranded

Topology: linear

Sequence variety: other nucleic acid

Sequence

CCAGTCGGGC TGAGG 15

Sequence No.: 115

Sequence length: 15

Sequence type: nucleic acid

Strand number: single-stranded

Topology: linear

Sequence variety: other nucleic acid

Sequence

AGGCTTCGGA CCAGT 15

Sequence No.: 116

Sequence length: 15

Sequence type: nucleic acid

Strand number: single-stranded

Topology: linear

Sequence variety: other nucleic acid

Sequence

ACACACTGGA AGGCT 15

Sequence No.: 117

Sequence length: 15

Sequence type: nucleic acid

Strand number: single-stranded

Topology: linear

Sequence variety: other nucleic acid

Sequence

TACTGCAGAC ACACA 15

Sequence No.: 118

Sequence length: 15

Sequence type: nucleic acid

Strand number: single-stranded

Topology: linear

Sequence variety: other nucleic acid

Sequence

TCTCCACCTC TACTG 15

Sequence No.: 119

Sequence length: 15

Sequence type: nucleic acid

Strand number: single-stranded

Topology: linear

Sequence variety: other nucleic acid

Sequence

CCGGCATGGA TCTCC 15

Sequence No.: 120

Sequence length: 15

Sequence type: nucleic acid

Strand number: single-stranded

Topology: linear

Sequence variety: other nucleic acid

Sequence

GTTGAGACCG CCGGC 15

Sequence No.: 121

Sequence length: 15

Sequence type: nucleic acid

Strand number: single-stranded

Topology: linear

Sequence variety: other nucleic acid

Sequence

ACGGCTCTAG GTTGA 15

Sequence No.: 122

Sequence length: 15

Sequence type: nucleic acid

Strand number: single-stranded

Topology: linear

Sequence variety: other nucleic acid

Sequence

CGCTTTAGAA ACGGC 15

Sequence No.: 123

Sequence length: 15

Sequence type: nucleic acid

Strand number: single-stranded

Topology: linear

Sequence variety: other nucleic acid

Sequence

CGCATCGACG CGCTT 15

Sequence No.: 124

Sequence length: 15

Sequence type: nucleic acid

Strand number: single-stranded

Topology: linear

Sequence variety: other nucleic acid

Sequence

GGTCGGCGTC CGCAT 15

Sequence No.: 125

Sequence length: 15

Sequence type: nucleic acid

Strand number: single-stranded

Topology: linear

Sequence variety: other nucleic acid

Sequence

TACTGCCGCG GGTCG 15

Sequence No.: 126

Sequence length: 15

Sequence type: nucleic acid

Strand number: single-stranded

Topology: linear

Sequence variety: other nucleic acid

Sequence

CGTGTCAGCA TACTG 15

Sequence No.: 127

Sequence length: 15

Sequence type: nucleic acid

Strand number: single-stranded

Topology: linear

Sequence variety: other nucleic acid

Sequence

GAGCCTTGAC CGTGT 15

Sequence No.: 128

Sequence length: 15

Sequence type: nucleic acid

Strand number: single-stranded

Topology: linear

Sequence variety: other nucleic acid

Sequence

CGCACGCGGA GAGCC 15

Sequence No.: 129

Sequence length: 15

Sequence type: nucleic acid

Strand number: single-stranded

Topology: linear

Sequence variety: other nucleic acid

Sequence

TGTGAGCCGC CGCAC 15

Sequence No.: 130

Sequence length: 15

Sequence type: nucleic acid

Strand number: single-stranded

Topology: linear

Sequence variety: other nucleic acid

Sequence

CGGCTCCCAC TGTGA 15

Sequence No.: 131

Sequence length: 15

Sequence type: nucleic acid

Strand number: single-stranded

Topology: linear

Sequence variety: other nucleic acid

Sequence

CGAACCTGTG CGGCT 15

Sequence No.: 132

Sequence length: 15

Sequence type: nucleic acid

Strand number: single-stranded

Topology: linear

Sequence variety: other nucleic acid

Sequence

TAGCTGAGCA CGAAC 15

Sequence No.: 133

Sequence length: 15

Sequence type: nucleic acid

Strand number: single-stranded

Topology: linear

Sequence variety: other nucleic acid

Sequence

CGCCTACCAG TAGCT 15

Sequence No.: 134

Sequence length: 15

Sequence type: nucleic acid

Strand number: single-stranded

Topology: linear

Sequence variety: other nucleic acid

Sequence

ACACGCAGGG CGCCT 15

Sequence No.: 135

Sequence length: 15

Sequence type: nucleic acid

Strand number: single-stranded

Topology: linear

Sequence variety: other nucleic acid

Sequence

GTACGCTAGC ACACG 15

Sequence No.: 136

Sequence length: 15

Sequence type: nucleic acid

Strand number: single-stranded

Topology: linear

Sequence variety: other nucleic acid

Sequence

TGAGGCGGGA GTACG 15

Sequence No.: 137

Sequence length: 15

Sequence type: nucleic acid

Strand number: single-stranded

Topology: linear

Sequence variety: other nucleic acid

Sequence

GTCAGTTCCT TGAGG 15

Sequence No.: 138

Sequence length: 15

Sequence type: nucleic acid

Strand number: single-stranded

Topology: linear

Sequence variety: other nucleic acid

Sequence

GTCCTCGAGC GTCAG 15

Sequence No.: 139

Sequence length: 15

Sequence type: nucleic acid

Strand number: single-stranded

Topology: linear

Sequence variety: other nucleic acid

Sequence

TTATCTTTAG GTCCT 15

Sequence No.: 140

Sequence length: 15

Sequence type: nucleic acid

Strand number: single-stranded

Topology: linear

Sequence variety: other nucleic acid

Sequence

ATGGTGCCGG TTATC 15

Sequence No.: 141

Sequence length: 15

Sequence type: nucleic acid

Strand number: single-stranded

Topology: linear

Sequence variety: other nucleic acid

Sequence

CAGCGGAGGC ATGGT 15

Sequence No.: 142

Sequence length: 15

Sequence type: nucleic acid

Strand number: single-stranded

Topology: linear

Sequence variety: other nucleic acid

Sequence

CTTCCAGAGG CAGCG 15

Sequence No.: 143

Sequence length: 15

Sequence type: nucleic acid

Strand number: single-stranded

Topology: linear

Sequence variety: other nucleic acid

Sequence

AGTCCTGTGG CTTCC 15

Sequence No.: 144

Sequence length: 15

Sequence type: nucleic acid

Strand number: single-stranded

Topology: linear

Sequence variety: other nucleic acid

Sequence

GGAAAGTGCA AGTCC 15

Sequence No.: 145

Sequence length: 15

Sequence type: nucleic acid

Strand number: single-stranded

Topology: linear

Sequence variety: other nucleic acid

Sequence

GGCGCAAGCT GGAAA 15

Sequence No.: 146

Sequene length: 15

Sequence type: nucleic acid

Strand number: single-stranded

Topology: linear

Sequence variety: other nucleic acid

Sequence

ACGTTGCGTA GGCGC 15

Sequence No.: 147

Sequene length: 15

Sequence type: nucleic acid

Strand number: single-stranded

Topology: linear

Sequence variety: other nucleic acid

Sequence

CGCCCACGAC ACGTT 15

Sequence No.: 148

Sequene length: 15

Sequence type: nucleic acid

Strand number: single-stranded

Topology: linear

Sequence variety: other nucleic acid

Sequence

AACGCCCTGT CGCCC 15

Sequence No.: 149

Sequene length: 15

Sequence type: nucleic acid

Strand number: single-stranded

Topology: linear

Sequence variety: other nucleic acid

Sequence

GCGACCCAAG AACGC 15

Sequence No.: 150

Sequence length: 15

Sequence type: nucleic acid

Strand number: single-stranded

Topology: linear

Sequence variety: other nucleic acid

Sequence

CTGCAGCTCG GCGAG 15

Sequence No.: 151

Sequene length: 15

Sequence type: nucleic acid

Strand number: single-stranded

Topology: linear

Sequence variety: other nucleic acid

Sequence

TGAGCCACTG CTGCA 15

Sequence No.: 152

Sequene length: 15

Sequence type: nucleic acid

Strand number: single-stranded

Topology: linear

Sequence variety: other nucleic acid

Sequence

AGGCCTGGCT TGAGC 15

Sequence No.: 153

Sequene length: 15

Sequence type: nucleic acid

Strand number: single-stranded

Topology: linear

Sequence variety: other nucleic acid

Sequence

CAGTACCTTG AGGCC 15

Sequence No.: 154

Sequene length: 15

Sequence type: nucleic acid

Strand number: single-stranded

Topology: linear

Sequence variety: other nucleic acid

Sequence

GGGCAATGCT CAGTA 15

Sequence No.: 155

Sequene length: 15

Sequence type: nucleic acid

Strand number: single-stranded

Topology: linear

Sequence variety: other nucleic acid

Sequence

GACTGTGCTT GGGCA 15

Sequence No.: 156

Sequene length: 15

Sequence type: nucleic acid

Strand number: single-stranded

Topology: linear

Sequence variety: other nucleic acid

Sequence

AAAGGCAGGC GAGTG 15

Sequence No.: 157

Sequene length: 15

Sequence type: nucleic acid

Strand number: single-stranded

Topology: linear

Sequence variety: other nucleic acid

Sequence

GTTCGTAGGA AAAGG 15

Sequence No.: 158

Sequene length: 15

Sequence type: nucleic acid

Strand number: single-stranded

Topology: linear

Sequence variety: other nucleic acid

Sequence

GCGCGAACCT GTTCG 15

Sequence No.: 159

Sequene length: 15

Sequence type: nucleic acid

Strand number: single-stranded

Topology: linear

Sequence variety: other nucleic acid

Sequence

GCCCGGGAAC GCGCG 15

Sequence No.: 160

Sequene length: 15

Sequence type: nucleic acid

Strand number: single-stranded

Topology: linear

Sequence variety: other nucleic acid

Sequence

GGCTGGTAAG GGCCG 15

Sequence No.: 161

Sequene length: 15

Sequence type: nucleic acid

Strand number: single-stranded

Topology: linear

Sequence variety: other nucleic acid

Sequence

GACAGGTCTA GGCTG 15

Sequence No.: 162

Sequene length: 15

Sequence type: nucleic acid

Strand number: single-stranded

Topology: linear

Sequence variety: other nucleic acid

Sequence

AGGATTGTCA GACAG 15

Sequence No.: 163

Sequene length: 15

Sequence type: nucleic acid

Strand number: single-stranded

Topology: linear

Sequence variety: other nucleic acid

Sequence

CGCCCAGTCC AGGAT 15

Sequence No.: 164

Sequene length: 15

Sequence type: nucleic acid

Strand number: single-stranded

Topology: linear

Sequence variety: other nucleic acid

Sequence

AGTCCGCGTT CGCCC 15

Sequence No.: 165

Sequene length: 15

Sequence type: nucleic acid

Strand number: single-stranded

Topology: linear

Sequence variety: other nucleic acid

Sequence

AGCCGCCATC AGTCC 15

Sequence No.: 166

Sequene length: 15

Sequence type: nucleic acid

Strand number: single-stranded

Topology: linear

Sequence variety: other nucleic acid

Sequence

GGGGACAGAG AGCCG 15

Sequence No.: 167

Sequene length: 15

Sequence type: nucleic acid

Strand number: single-stranded

Topology: linear

Sequence variety: other nucleic acid

Sequence

GGGAACTTGT GGGGA 15

Sequence No.: 168

Sequene length: 15

Sequence type: nucleic acid

Strand number: single-stranded

Topology: linear

Sequence variety: other nucleic acid

Sequence

CTGGATGGCC GGGAA 15

Sequence No.: 169

Sequene length: 15

Sequence type: nucleic acid

Strand number: single-stranded

Topology: linear

Sequence variety: other nucleic acid

Sequence

GCGCTAGATT CTGGA 15

Sequence No.: 170

Sequene length: 15

Sequence type: nucleic acid

Strand number: single-stranded

Topology: linear

Sequence variety: other nucleic acid

Sequence

GTGTTGCGC AGCGCT 15

Sequence No.: 171

Sequene length: 15

Sequence type: nucleic acid

Strand number: single-stranded

Topology: linear

Sequence variety: other nucleic acid

Sequence

CTCCATTCCT CTGTT 15

Sequence No.: 172

Sequene length: 15

Sequence type: nucleic acid

Strand number: single-stranded

Topology: linear

Sequence variety: other nucleic acid

Sequence

CTGTGGGCGT CTCCA 15

Sequence No.: 173

Sequene length: 15

Sequence type: nucleic acid

Strand number: single-stranded

Topology: linear

Sequence variety: other nucleic acid

Sequence

GCGCACACGC CTGTG 15

Sequence No.: 174

Sequene length: 15

Sequence type: nucleic acid

Strand number: single-stranded

Topology: linear

Sequence variety: other nucleic acid

Sequence

CGCCAGTGCG CCGCA 15

Sequence No.: 175

Sequene length: 15

Sequence type: nucleic acid

Strand number: single-stranded

Topology: linear

Sequence variety: other nucleic acid

Sequence

CACCTGCCGC CGCCA 15

Sequence No.: 176

Sequene length: 15

Sequence type: nucleic acid

Strand number: single-stranded

Topology: linear

Sequence variety: other nucleic acid

Sequence

TGGGGCTGCA CACCT 15

Sequence No.: 177

Sequene length: 15

Sequence type: nucleic acid

Strand number: single-stranded

Topology: linear

Sequence variety: other nucleic acid

Sequence

GTCTAGGCTG TGGGG 15

Sequence No.: 178

Sequene length: 15

Sequence type: nucleic acid

Strand number: single-stranded

Topology: linear

Sequence variety: other nucleic acid

Sequence

TGTGGCTGAG GTCTA 15

Sequence No.: 179

Sequene length: 15

Sequence type: nucleic acid

Strand number: single-stranded

Topology: linear

Sequence variety: other nucleic acid

Sequence

CGCAGCGAGT TGTGG 15

Sequence No.: 180

Sequene length: 15

Sequence type: nucleic acid

Strand number: single-stranded

Topology: linear

Sequence variety: other nucleic acid

Sequence

TACGGTGGCG CGCAG 15

Sequence No.: 181

Sequene length: 15

Sequence type: nucleic acid

Strand number: single-stranded

Topology: linear

Sequence variety: other nucleic acid

Sequence

CGCTAGGCTT TACGG 15

Sequence No.: 182

Sequene length: 15

Sequence type: nucleic acid

Strand number: single-stranded

Topology: linear

Sequence variety: other nucleic acid

Sequence

CATCTCGGAG CGCTA 15

Sequence No.: 183

Sequene length: 15

Sequence type: nucleic acid

Strand number: single-stranded

Topology: linear

Sequence variety: other nucleic acid

Sequence

GGACCACATG CATCT 15

Sequence No.: 184

Sequene length: 15

Sequence type: nucleic acid

Strand number: single-stranded

Topology: linear

Sequence variety: other nucleic acid

Sequence

TCAGGGCGCT GGACC 15

Sequence No.: 185

Sequene length: 15

Sequence type: nucleic acid

Strand number: single-stranded

Topology: linear

Sequence variety: other nucleic acid

Sequence

TTGAGGGACT TCAGG 15

Sequence No.: 186

Sequene length: 15

Sequence type: nucleic acid

Strand number: single-stranded

Topology: linear

Sequence variety: other nucleic acid

Sequence

GAACGACAGA TTGAG 15

Sequence No.: 187

Sequene length: 15

Sequence type: nucleic acid

Strand number: single-stranded

Topology: linear

Sequence variety: other nucleic acid

Sequence

CCAGCCCAGC GAACG 15

Sequence No.: 188

Sequene length: 15

Sequence type: nucleic acid

Strand number: single-stranded

Topology: linear

Sequence variety: other nucleic acid

Sequence

GGCACCTGTT CCAGC 15

Sequence No.: 189

Sequene length: 15

Sequence type: nucleic acid

Strand number: single-stranded

Topology: linear

Sequence variety: other nucleic acid

Sequence

CAGTCCTTTA GGCAC 15

Sequence No.: 190

Sequene length: 15

Sequence type: nucleic acid

Strand number: single-stranded

Topology: linear

Sequence variety: other nucleic acid

Sequence

GCTTGGCTGG CAGTC 15

Sequence No.: 191

Sequene length: 15

Sequence type: nucleic acid

Strand number: single-stranded

Topology: linear

Sequence variety: other nucleic acid

Sequence

AGCACTCTGA GCTTG 15

Sequence No.: 192

Sequene length: 15

Sequence type: nucleic acid

Strand number: single-stranded

Topology: linear

Sequence variety: other nucleic acid

Sequence

GCTGAGATCG AGCAC 15

Sequence No.: 193

Sequene length: 15

Sequence type: nucleic acid

Strand number: single-stranded

Topology: linear

Sequence variety: other nucleic acid

Sequence

GTCTGTTGCA GCTGA 15

Sequence No.: 194

Sequene length: 15

Sequence type: nucleic acid

Strand number: single-stranded

Topology: linear

Sequence variety: other nucleic acid

Sequence

GCCCTGTTCA GTCTG 15

Sequence No.: 195

Sequene length: 15

Sequence type: nucleic acid

Strand number: single-stranded

Topology: linear

Sequence variety: other nucleic acid

Sequence

GCAGCTCCTC AGGCT 15

Sequence No.: 196

Sequene length: 15

Sequence type: nucleic acid

Strand number: single-stranded

Topology: linear

Sequence variety: other nucleic acid

Sequence

TCCACCTCGG GCAGC 15

Sequence No.: 197

Sequene length: 15

Sequence type: nucleic acid

Strand number: single-stranded

Topology: linear

Sequence variety: other nucleic acid

Sequence

TGTCAGGTTA TCCAC 15

Sequence No.: 198

Sequene length: 15

Sequence type: nucleic acid

Strand number: single-stranded

Topology: linear

Sequence variety: other nucleic acid

Sequence

TCCCGTCCAG TGTCA 15

Sequence No.: 199

Sequene length: 15

Sequence type: nucleic acid

Strand number: single-stranded

Topology: linear

Sequence variety: other nucleic acid

Sequence

AGGAAGGGAT TCCCG 15

Sequence No.: 200

Sequene length: 15

Sequence type: nucleic acid

Strand number: single-stranded

Topology: linear

Sequence variety: other nucleic acid

Sequence

TCCAGGGACC AGGAA 15

Sequence No.: 201

Sequene length: 15

Sequence type: nucleic acid

Strand number: single-stranded

Topology: linear

Sequence variety: other nucleic acid

Sequence

GGAGGGCAGT TCCAG 15

Sequence No.: 202

Sequene length: 15

Sequence type: nucleic acid

Strand number: single-stranded

Topology: linear

Sequence variety: other nucleic acid

Sequence

CCCTCGTGGG GGAGG 15

Sequence No.: 203

Sequene length: 15

Sequence type: nucleic acid

Strand number: single-stranded

Topology: linear

Sequence variety: other nucleic acid

Sequence

GTTCATTGAG CCCTC 15

Sequence No.: 204

Sequene length: 15

Sequence type: nucleic acid

Strand number: single-stranded

Topology: linear

Sequence variety: other nucleic acid

Sequence

CCACGCCGGA GTTCA 15

Sequence No.: 205

Sequene length: 15

Sequence type: nucleic acid

Strand number: single-stranded

Topology: linear

Sequence variety: other nucleic acid

Sequence

CAGGCTGGGA CCACG 15

Sequence No.: 206

Sequene length: 15

Sequence type: nucleic acid

Strand number: single-stranded

Topology: linear

Sequence variety: other nucleic acid

Sequence

CGAACGTGCA CAGGC 15

Sequence No.: 207

Sequene length: 15

Sequence type: nucleic acid

Strand number: single-stranded

Topology: linear

Sequence variety: other nucleic acid

Sequence

CCGACACGGT CGAAC 15

Sequence No.: 208

Sequene length: 15

Sequence type: nucleic acid

Strand number: single-stranded

Topology: linear

Sequence variety: other nucleic acid

Sequence

GACACCCCCA CCGAC 15

Sequence No.: 209

Sequene length: 15

Sequence type: nucleic acid

Strand number: single-stranded

Topology: linear

Sequence variety: other nucleic acid

Sequence

CAGGGTTCCC GACAC 15

Sequence No.: 210

Sequene length: 15

Sequence type: nucleic acid

Strand number: single-stranded

Topology: linear

Sequence variety: other nucleic acid

Sequence

GGAGCAGCAC CAGGG 15

Sequence No.: 211

Sequene length: 15

Sequence type: nucleic acid

Strand number: single-stranded

Topology: linear

Sequence variety: other nucleic acid

Sequence

CGGGCCCCTT GGAGC 15

Sequence No.: 212

Sequene length: 15

Sequence type: nucleic acid

Strand number: single-stranded

Topology: linear

Sequence variety: other nucleic acid

Sequence

GGCAAAGCCC CGGGC 15

Sequence No.: 213

Sequene length: 15

Sequence type: nucleic acid

Strand number: single-stranded

Topology: linear

Sequence variety: other nucleic acid

Sequence

TTGGATCTTA GGCAA 15

Sequence No.: 214

Sequene length: 15

Sequence type: nucleic acid

Strand number: single-stranded

Topology: linear

Sequence variety: other nucleic acid

Sequence

TTATTCTGTC TTGGA 15

Sequence No.: 215

Sequene length: 15

Sequence type: nucleic acid

Strand number: single-stranded

Topology: linear

Sequence variety: other nucleic acid

Sequence

ACTCCATTCA TTATT 15

Sequence No.: 216

Sequene length: 15

Sequence type: nucleic acid

Strand number: single-stranded

Topology: linear

Sequence variety: other nucleic acid

Sequence

AGGCAGTTTG ACTCC 15

Sequence No.: 217

Sequene length: 15

Sequence type: nucleic acid

Strand number: single-stranded

Topology: linear

Sequence variety: other nucleic acid

Sequence

CCTGAAGCCA AGGCA 15

Sequence No.: 218

Sequene length: 15

Sequence type: nucleic acid

Strand number: single-stranded

Topology: linear

Sequence variety: other nucleic acid

Sequence

ACGGGACTCC CCTGA 15

Sequence No.: 219

Sequene length: 15

Sequence type: nucleic acid

Strand number: single-stranded

Topology: linear

Sequence variety: other nucleic acid

Sequence

CAACGTCCTG ACGGG 15

Sequence No.: 220

Sequene length: 15

Sequence type: nucleic acid

Strand number: single-stranded

Topology: linear

Sequence variety: other nucleic acid

Sequence

GAAAAGTCCT CAACG 15

Sequence No.: 221

Sequene length: 15

Sequence type: nucleic acid

Strand number: single-stranded

Topology: linear

Sequence variety: other nucleic acid

Sequence

TGAATTGGTC GAAAA 15

Sequence No.: 222

Sequene length: 15

Sequence type: nucleic acid

Strand number: single-stranded

Topology: linear

Sequence variety: other nucleic acid

Sequence

GGCAAAGCGT TGAAT 15

Sequence No.: 223

Sequene length: 15

Sequence type: nucleic acid

Strand number: single-stranded

Topology: linear

Sequence variety: other nucleic acid

Sequence

ATAAAGGTGG GGCAA 15

Sequence No.: 224

Sequene length: 30

Sequence type: nucleic acid

Strand number: single-stranded

Topology: linear

Sequence variety: other nucleic acid

Sequence

GCAGGACGCG CGCTCCATGG TCGATAAGTC 30

Sequence No.: 225

Sequene length: 20

Sequence type: nucleic acid

Strand number: single-stranded

Topology: linear

Sequence variety: other nucleic acid

Sequence

TTCATCGTCC AGCTCACAAG 20

Sequence No.: 226

Sequene length: 20

Sequence type: nucleic acid

Strand number: single-stranded

Topology: linear

Sequence variety: other nucleic acid

Sequence

GCGTCCGCAT CGACGCGCTT 20

Sequence No.: 227

Sequene length: 20

Sequence type: nucleic acid

Strand number: single-stranded

Topology: linear

Sequence variety: other nucleic acid

Sequence

CTGTGCGGCT CCCACTGTGA 20

Sequence No.: 228

Sequene length: 20

Sequence type: nucleic acid

Strand number: single-stranded

Topology: linear

Sequence variety: other nucleic acid

Sequence

CGGGAGTACG CTAGCACACG 20

Sequence No.: 229

Sequene length: 20

Sequence type: nucleic acid

Strand number: single-stranded

Topology: linear

Sequence variety: other nucleic acid

Sequence

CAGTTCCTTG AGGCGGGAGT 20

Sequence No.: 230

Sequene length: 20

Sequence type: nucleic acid

Strand number: single-stranded

Topology: linear

Sequence variety: other nucleic acid

Sequence

GGTCCTCGAG CGTCAGTTCC 20

Sequence No.: 231

Sequene length: 20

Sequence type: nucleic acid

Strand number: single-stranded

Topology: linear

Sequence variety: other nucleic acid

Sequence

AAGCTGGAAA GTGCAAGTCC 20

Sequence No.: 232

Sequene length: 20

Sequence type: nucleic acid

Strand number: single-stranded

Topology: linear

Sequence variety: other nucleic acid

Sequence

AAAGGCAGGC GAGTGTGCTT 20

Sequence No.: 233

Sequene length: 20

Sequence type: nucleic acid

Strand number: single-stranded

Topology: linear

Sequence variety: other nucleic acid

Sequence

AGTCCAGGAT TGTCAGACAG 20

Sequence No.: 234

Sequene length: 20

Sequence type: nucleic acid

Strand number: single-stranded

Topology: linear

Sequence variety: other nucleic acid

Sequence

TCGCCCAGTC CAGGATTGTC 20

Sequence No.: 235

Sequene length: 20

Sequence type: nucleic acid

Strand number: single-stranded

Topology: linear

Sequence variety: other nucleic acid

Sequence

CTGTGGCCGT CTCCATTCCT 20

Sequence No.: 236

Sequene length: 20

Sequence type: nucleic acid

Strand number: single-stranded

Topology: linear

Sequence variety: other nucleic acid

Sequence

CTGAGCTCTA GCCTGTGGCG 20

Sequence No.: 237

Sequene length: 20

Sequence type: nucleic acid

Strand number: single-stranded

Topology: linear

Sequence variety: other nucleic acid

Sequence

CGCTAGGGTT TACGGTGGCG 20

Sequence No.: 238

Sequene length: 20

Sequence type: nucleic acid

Strand number: single-stranded

Topology: linear

Sequence variety: other nucleic acid

Sequence

TTGCAGCTGA GATCGAGCAC 20

Sequence No.: 239

Sequene length: 20

Sequence type: nucleic acid

Strand number: single-stranded

Topology: linear

Sequence variety: other nucleic acid

Sequence

TCCAGTCTCA GGTTATCCAC 20

Sequence No.: 240

Sequene length: 20

Sequence type: nucleic acid

Strand number: single-stranded

Topology: linear

Sequence variety: other nucleic acid

Sequence

GGAGCAGCAC CAGGGTTCCC 20

Sequence No.: 241

Sequene length: 20

Sequence type: nucleic acid

Strand number: single-stranded

Topology: linear

Sequence variety: other nucleic acid

Sequence

CCCTTGGAGC AGCACCAGGG 20

Sequence No.: 242

Sequene length: 21

Sequence type: nucleic acid

Strand number: single-stranded

Topology: linear

Sequence variety: other nucleic acid

Characteristic of sequence

Other information: i indicates inosine.

Sequence

CiCGCTCCAT GGTCGiTAii T 21

Sequence No.: 243

Sequene length: 21

Sequence type: nucleic acid

Strand number: single-stranded

Topology: linear

Sequence variety: other nucleic acid

Characteristic of sequence

Other information: i indicates inosine.

Sequence

iCiCGCTCCA TGGTCGiTAi i 21

Sequence No.: 244

Sequene length: 21

Sequence type: nucleic acid

Strand number: single-stranded

Topology: linear

Sequence variety: other nucleic acid

Characteristic of sequence

Other information: i indicates inosine.

Sequence

CiCiCGCTCC ATGGTCGiTA i 21

Sequence No.: 245

Sequene length: 21

Sequence type: nucleic acid

Strand number: single-stranded

Topology: linear

Sequence variety: other nucleic acid

Characteristic of sequence

Other information: i indicates inosine.

Sequence

iCiCiCGCTC CATGGTCGiT A 21

Sequence No.: 246

Sequene length: 21

Sequence type: nucleic acid

Strand number: single-stranded

Topology: linear

Sequence variety: other nucleic acid

Characteristic of sequence

Other information: i indicates inosine.

Sequence

iiCiCiCGCT CCATGCTCGi T 21

Sequence No.: 247

Sequene length: 21

Sequence type: nucleic acid

Strand number: single-stranded

Topology: linear

Sequence variety: other nucleic acid

Characteristic of sequence

Other information: i indicates inosine.

Sequence

iiiCiCiCGC TCCATGGTCC i 21

Sequence No.: 248

Sequene length: 21

Sequence type: nucleic acid

Strand number: single-stranded

Topology: linear

Sequence variety: other nucleic acid

Characteristic of sequence

Other information: i indicates inosine.

Sequence

iiiiCiCiCG CTCCATGGTC C 21

Sequence No.: 249

Sequene length: 21

Sequence type: nucleic acid

Strand number: single-stranded

Topology: linear

Sequence variety: other nucleic acid

Characteristic of sequence

Other information: i indicates inosine.

Sequence

CiiiiCiCiC GCTCCATGGT C 21

Sequence No.: 250

Sequene length: 21

Sequence type: nucleic acid

Strand number: single-stranded

Topology: linear

Sequence variety: other nucleic acid

Characteristic of sequence

Other information: i indicates inosine.

Sequence

GCiiiiCiCi CGCTCCATGG T 21

Sequence No.: 251

Sequene length: 21

Sequence type: nucleic acid

Strand number: single-stranded

Topology: linear

Sequence variety: other nucleic acid

Characteristic of sequence

Other information: i indicates inosine.

Sequence

AGCiiiiCiC iCGCTCCATG G 21

Sequence No.: 252

Sequene length: 21

Sequence type: nucleic acid

Strand number: single-stranded

Topology: linear

Sequence variety: other nucleic acid

Characteristic of sequence

Other information: i indicates inosine.

Sequence

AAGCiiiiCi CiCGCTCCAT G 21

Sequence No.: 253

Sequene length: 21

Sequence type: nucleic acid

Strand number: single-stranded

Topology: linear

Sequence variety: other nucleic acid

Characteristic of sequence

Other information: i indicates inosine.

Sequence

CAAGCiiiiC iCiCGCTCCA T 21

Sequence No.: 254

Sequene length: 21

Sequence type: nucleic acid

Strand number: single-stranded

Topology: linear

Sequence variety: other nucleic acid

Characteristic of sequence

Other information: i indicates inosine.

Sequence

ACAAGCiiii CiCiCGCTCC A 21

Sequence No.: 255

Sequene length: 21

Sequence type: nucleic acid

Strand number: single-stranded

Topology: linear

Sequence variety: other nucleic acid

Characteristic of sequence

Other information: i indicates inosine.

Sequence

AACAAGCiii iCiCiCGCTC C 21

Sequence No.: 256

Sequene length: 21

Sequence type: nucleic acid

Strand number: single-stranded

Topology: linear

Sequence variety: other nucleic acid

Characteristic of sequence

Other information: i indicates inosine.

Sequence

CAACAAGCii iiCiCiCGCT C 21

Sequence No.: 257

Sequene length: 21

Sequence type: nucleic acid

Strand number: single-stranded

Topology: linear

Sequence variety: other nucleic acid

Characteristic of sequence

Other information: i indicates inosine.

Sequence

GCAACAAGCi iiiCiCiCGC T 21

Sequence No.: 258

Sequene length: 21

Sequence type: nucleic acid

Strand number: single-stranded

Topology: linear

Sequence variety: other nucleic acid

Sequence

CACACGCTCC ATGGTCGGTA G 21

## Claims

1. An oligonucleotide which is capable of hybridizing with at least part of a gene encoding human CD14.

2. An oligonucleotide according to Claim 1, containing a sequence complementary to at least a part of a gene encoding human CD14.

3. An oligonucleotide according to Claim 1 or 2, wherein the oligonucleotide comprising at least a sequence which is complementary to a sequence selected from the group consisting of a 5, non-coding region, translation initiation region, coding region and 3' non-coding region of mRNA encoding human CD14 mRNA, at least part thereof.

4. An oligonucleotide according to any one of Claims 1 to 3, wherein the oligonucleotide is comprising a nucleotide sequence, which is hybridizable with or being complementary to any one of nucleotide sequences selected from the group consisting of following (1) - (19) or at least a part thereof:

(1) a nucleotide sequence of 40 mer of nucleotides positioning from 23th cytosine to 62th adenine,
(2) a nucleotide sequence of 39 mer of nucleotides positioning from 93th guanine to 131th cytosine,

(3) a nucleotide sequence of 29 mer of nucleotides positioning from 117th guanine to 145th uridine,
(4) a nucleotide sequence of 40 mer of nucleotides positioning from 1241th adenine to 1280th guanine,
(5) a nucleotide sequence of 22 mer of nucleotides positioning from 1264th guanine to 1285th cytosine,
(6) a nucleotide sequence of 54 mer of nucleotides positioning from 1267th cytosine to 1320th adenine,
(7) a nucleotide sequence of 50 mer of nucleotides positioning from 1301th guanine to 1350th adenine,
(8) a nucleotide sequence of 20 mer of nucleotides positioning from 184th cytosine to 203th adenine,
(9) a nucleotide sequence of 20 mer of nucleotides positioning from 324th adenine to 343th cytosine,
(10) a nucleotide sequence of 20 mer of nucleotides positioning from 394th uridine to 413th guanine,
(11) a nucleotide sequence of 46 mer of nucleotides positioning from 444th cytosine to 489th cytosine,
(12) a nucleotide sequence of 20 mer of nucleotides positioning from 534th guanine to 553th uridine,
(13) a nucleotide sequence of 25 mer of nucleotides positioning from 644th uridine to 668th uridine,
(14) a nucleotide sequence of 75 mer of nucleotides positioning from 684th cytosine to 758th uridine,
(15) a nucleotide sequence of 35 mer of nucleotides positioning from 794th adenine to 828th guanine,
(16) a nucleotide sequence of 55 mer of nucleotides positioning from 864th cytosine to 918th guanine,
(17) a nucleotide sequence of 55 mer of nucleotides positioning from 994th guanine to 1048th cytosine,
(18) a nucleotide sequence of 45 mer of nucleotides positioning from 1064th guanine to 1108th uridine, and
(19) a nucleotide sequence of 30 mer of nucleotides positioning from 1194th guanine to 1223th guanine, in a nucleotide sequence of SEQ. ID. No. 1.

**5.** An oligonucleotide according to any one of Claims 1 to 4, wherein the oligonucleotide is comprising a nucleotide sequence complementary to any one of nucleotide sequences selected from the group consisting of the following (1) - (19) or a nucleotide sequence complementary to at least a part thereof:

(1) a nucleotide sequence of 40 mer of nucleotides positioning from 23th cytosine to 62th adenine,
(2) a nucleotide sequence of 39 mer of nucleotides positioning from 93th guanine to 131th cytosine,
(3) a nucleotide sequence of 29 mer of nucleotides positioning from 117th guanine to 145th uridine,
(4) a nucleotide sequence of 40 mer of nucleotides positioning from 1241th adenine to 1280th guanine,
(5) a nucleotide sequence of 22 mer of nucleotides positioning from 1264th guanine to 1285th cytosine,
(6) a nucleotide sequence of 54 mer of nucleotides positioning from 1267th cytosine to 1320th adenine,
(7) a nucleotide sequence of 50 mer of nucleotides positioning from 1301th guanine to 1350th adenine,
(8) a nucleotide sequence of 20 mer of nucleotides positioning from 184th cytosine to 203th adenine,
(9) a nucleotide sequence of 20 mer of nucleotides positioning from 324th adenine to 343th cytosine,
(10) a nucleotide sequence of 20 mer of nucleotides positioning from 394th uridine to 413th guanine,
(11) a nucleotide sequence of 46 mer of nucleotides positioning from 444th cytosine to 489th cytosine,
(12) a nucleotide sequence of 20 mer of nucleotides positioning from 534th guanine to 553th uridine,
(13) a nucleotide sequence of 25 mer of nucleotides positioning from 644th uridine to 668th uridine,
(14) a nucleotide sequence of 75 mer of nucleotides positioning from 684th cytosine to 758th uridine,
(15) a nucleotide sequence of 35 mer of nucleotides positioning from 794th adenine to 828th guanine,
(16) a nucleotide sequence of 55 mer of nucleotides positioning from 864th cytosine to 918th guanine,
(17) a nucleotide sequence of 55 mer of nucleotides positioning from 994th guanine to 1048th cytosine,
(18) a nucleotide sequence of 45 mer of nucleotides positioning from 1064th guanine to 1108th uridine, and
(19) a nucleotide sequence of 30 mer of nucleotides positioning from 1194th guanine to 1223th guanine, in a nucleotide sequence of SEQ. ID. No. 1.

**6.** An oligonucleotide according to claim 4 wherein the oligonucleotide is hybridizable with any one of nucleotide sequences selected from (1), (2), (4), (5), (7), (8), (11), (16) and (19) among the nucleotide sequences according to Claim 4; or hybridizable with at least a part of any one of nucleotide sequences selected from the (1), (2), (4), (5), (7), (8), (11), (16) and (19).

**7.** An oligonucleotide according to Claim 5, wherein the oligonucleotide has a nucleotide sequence complementary to any one of nucleotide sequences selected from (1), (2), (4), (5), (7), (8), (11), (16) and (19) among the nucleotide sequences according to Claim 5; or a nucleotide sequence complementary to at least part of any one of nucleotide sequences selected from (1), (2), (4), (5), (7), (8), (11), (16) and (19).

**8.** An oligonucleotide according to any one of Claims 1 to 7, wherein the oligonucleotide is capable of suppressing the expression of human CD14.

**9.** An oligonucleotide according to Claim 8, wherein the oligonucleotide is capable of suppressing the expression of human CD14 by at least 30 % in a translation inhibition experiment.

**10.** An oligonucleotide according to Claim 8, wherein the oligonucleotide is exhibiting at least score 1 of binding ability with a mRNA encoding human CD14 mRNA in an RNase H cleavage experiment.

**11.** An oligonucleotide according to any one of Claims 1 to 10, wherein a nucleotide number is any of 10 to 50.

**12.** An oligonucleotide according to Claim 11, wherein a nucleotide number is any of 15 to 30.

**13.** An oligonucleotide according to any one of Claims 1 to 12, wherein at least one of internucleotides linkages between nucleotides contains a sulphur atom.

**14.** An oligonucleotide, containing at least one of nucleotide sequences selected from the group consisting of sequence No. 10, 11, 12, 13, 16, 19, 20, 21, 22, 23, 24, 25, 26, 27, 28, 29, 32, 33, 34, 35, 36, 37, 39, 40, 41, 42, 45, 46, 47, 48, 49, 50, 51, 52, 53, 54, 55, 56, 57, 58, 59, 61, 62, 63, 64, 70, 71, 72, 73, 74, 75, 76, 77, 78, 79, 81, 83, 85, 86, 87, 88, 89, 90, 102, 103, 109, 123, 124, 125, 130, 135, 136, 137, 138, 144, 155, 156, 159, 160, 161, 162, 163, 164, 165, 170, 171, 172, 177, 178, 179, 180, 181, 190, 191, 192, 193, 194, 196, 197, 198, 199, 209, 210, 215, 216, 220, 221, 224, 225, 226, 227, 228, 229, 230, 231, 232, 233, 234, 235, 236, 237, 238, 239, 240, 241, 242, 243, 244, 245, 246, 247 and 248; and composed of 30 or less nucleotides.

**15.** An oligonucleotide according to Claims 1 to 14, capable of hybridizing with also a gene encoding CD14 of an animal other than human.

**16.** An oligonucleotide according to Claim 15, wherein the animal other than human is mouse and/or simian.

**17.** An oligonucleotide according to Claim 15 or 16, containing a nucleotide sequence wherein arbitrary at least one nucleotide is substituted with universal base or bases, in a nucleotide sequence complementary to any one of nucleotide sequences selected from the group consisting of following (1) - (8) or nucleotide sequence complementary to at least a part of the sequence:

(1) a nucleotide sequence of 29 mer of nucleotides positioning from 103th adenine to 131th cytosine,
(2) a nucleotide sequence of 20 mer of nucleotides positioning from 184th cytosine to 203th adenine,
(3) a nucleotide sequence of 20 mer of nucleotides positioning from 324th adenine to 343th cytosine,
(4) a nucleotide sequence of 46 mer of nucleotides positioning from 444th cytosine to 489th cytosine,
(5) a nucleotide sequence of 75 mer of nucleotides positioning from 684th cytosine to 758th uridine,
(6) a nucleotide sequence of 35 mer of nucleotides positioning from 794th adenine to 828th quanine,
(7) a nucleotide sequence of 45 mer of nucleotides positioning from 864th cytosine to 908th adenine,
(8) a nucleotide sequence of 53 mer of nucleotides positioning from 994th guanine to 1046th guanine, and
(9) a nucleotide sequence of 45 mer of nucleotides positioning from 1064th guanine to 1108th uridine, of a nucleotide sequence of SEQ. ID. No. 1.

**18.** A pharmaceutical composition, comprising an oligonucleotide according to any one of Claims 1 to 17, and optionally further comprising a pharmacologically acceptable carrier.

**19.** A pharmaceutical composition according to Claim 18 for the treatment of diseases caused by an inflammatory factor induced through human CD14.

**20.** A paharmaceutical composition according to Claim 19, wherein said diseases are sepsis or endotoxemia, or septic shock or endotoxin shock.

**21.** A pharmaceutical composition employed for the prevention/treatement of sepsis or endotoxemia, or septic shock or endotoxin shock, which contains an oligonucleotide binding to a gene encoding human CD14 and capable of suppressing the expression of the human CD 14 as its effective ingradient.

**22.** A method of prevention/treatment of diseases caused by an inflammatory factor induced through human CD14, wherein an oligonucleotide according to any one of Claims 1 to 17 and optionally further a pharmacologically acceptable carrier is/are administered.

# FIG. 1

Translation inhibitory activity of human CD14 anti-sense in non-coding region and coding region

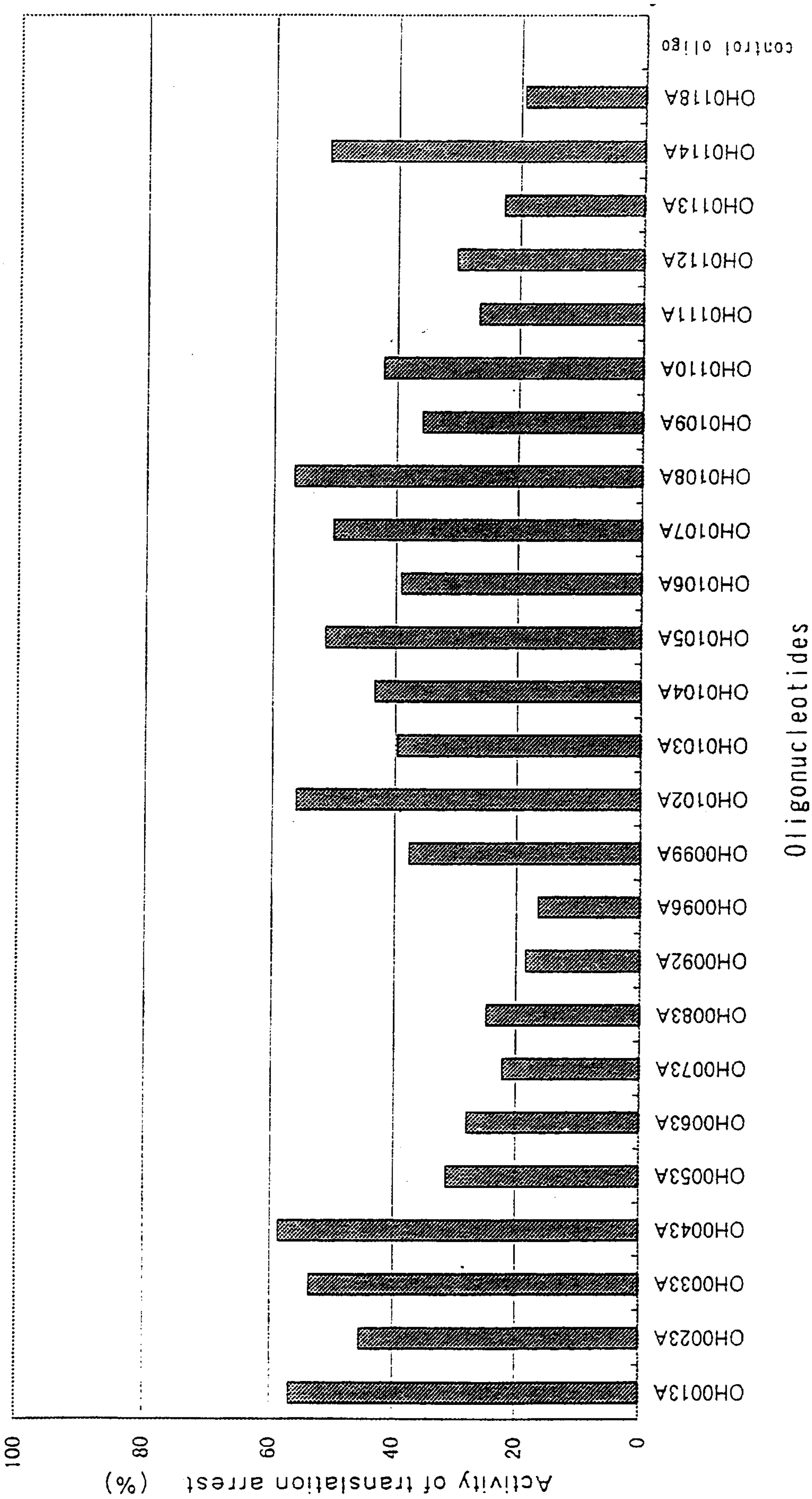

FIG. 2

Relation between oligonucleotide length and their inhibitory activities

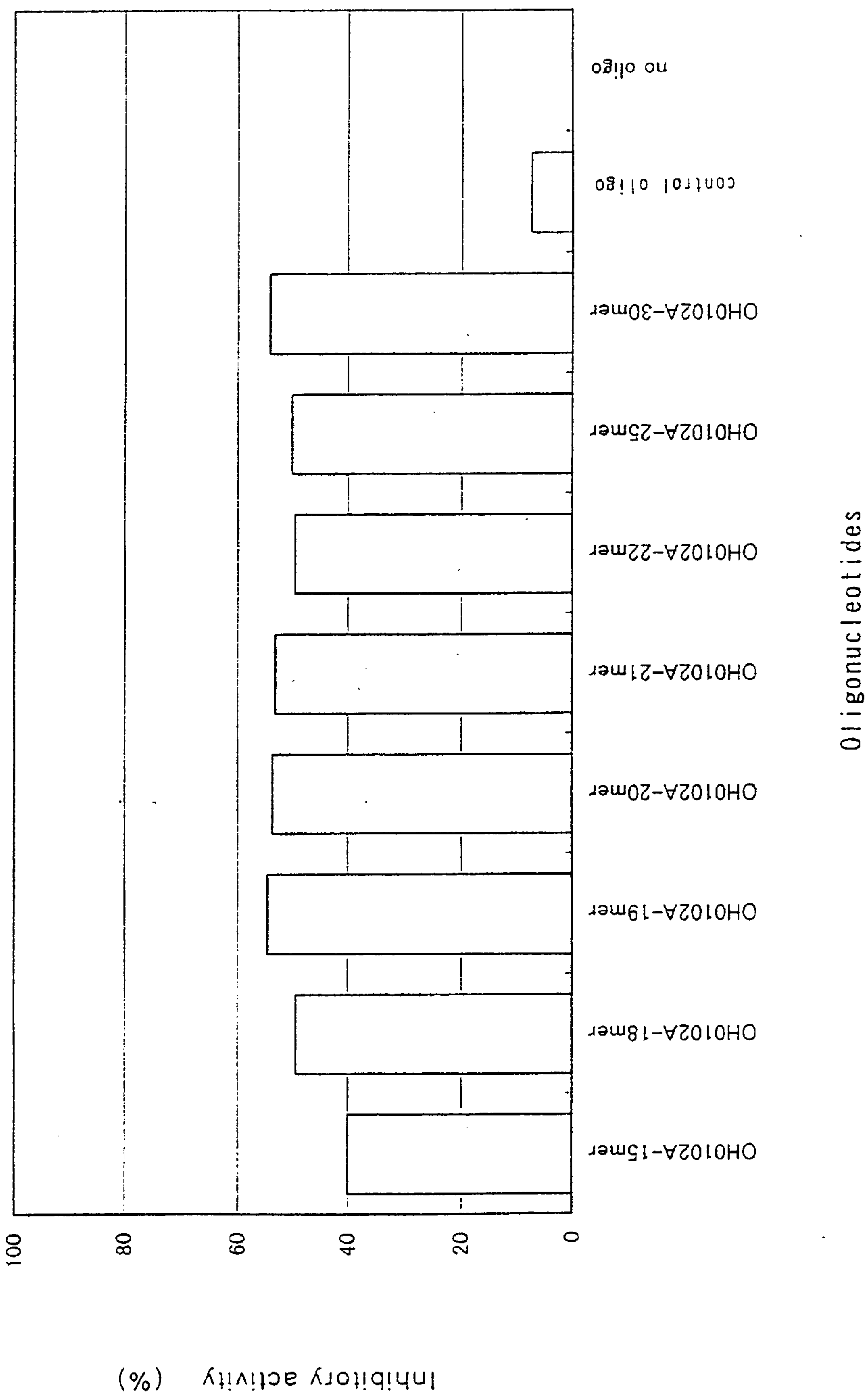

FIG. 3

Inhibitory activities in TNF production by human CD14 antisense oligonucleotides to 5' noncoding region and translation initiation region

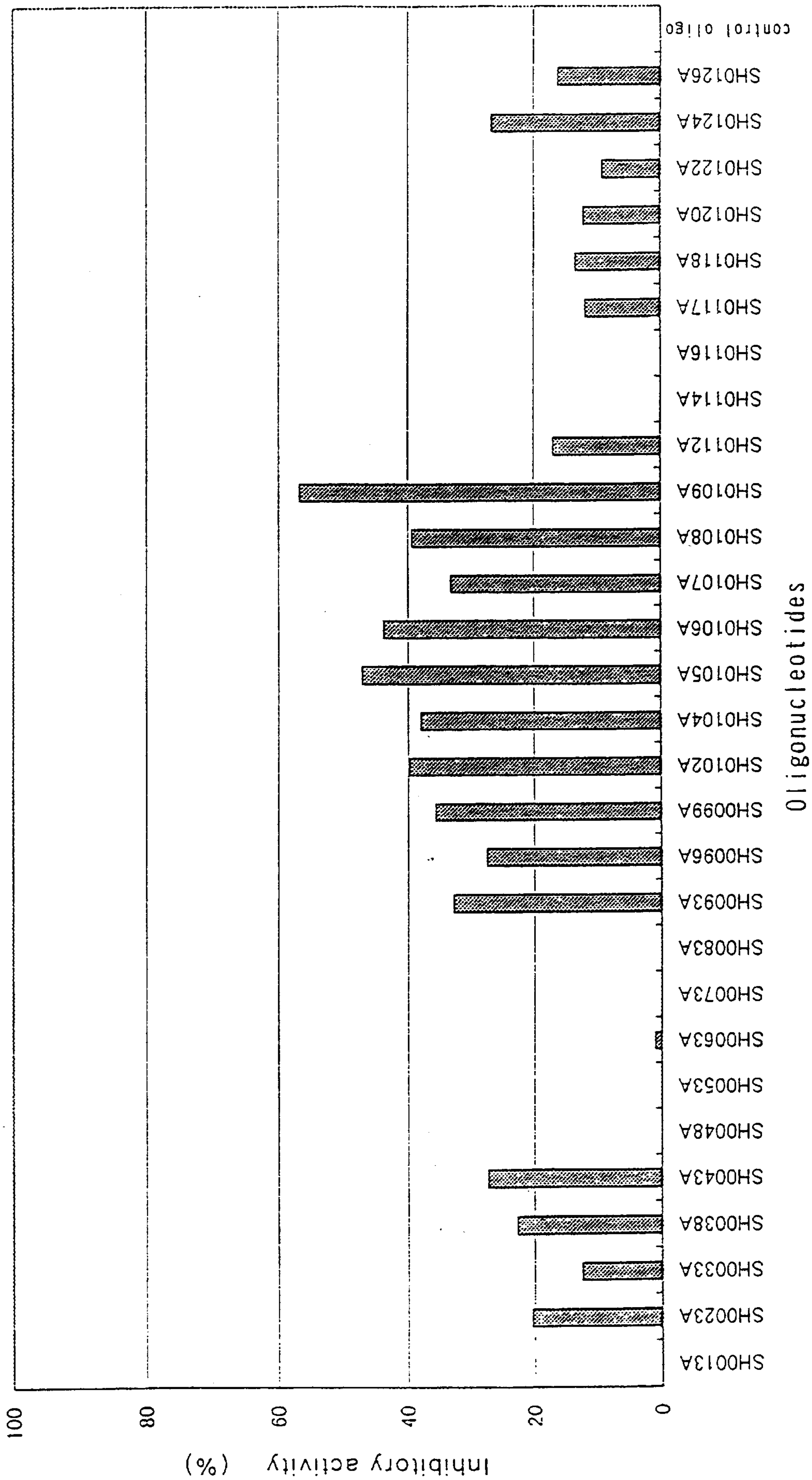

FIG. 4

Effect of human CD14 antisense oligonucleotide complimentary to 3' non-coding region on TNF production

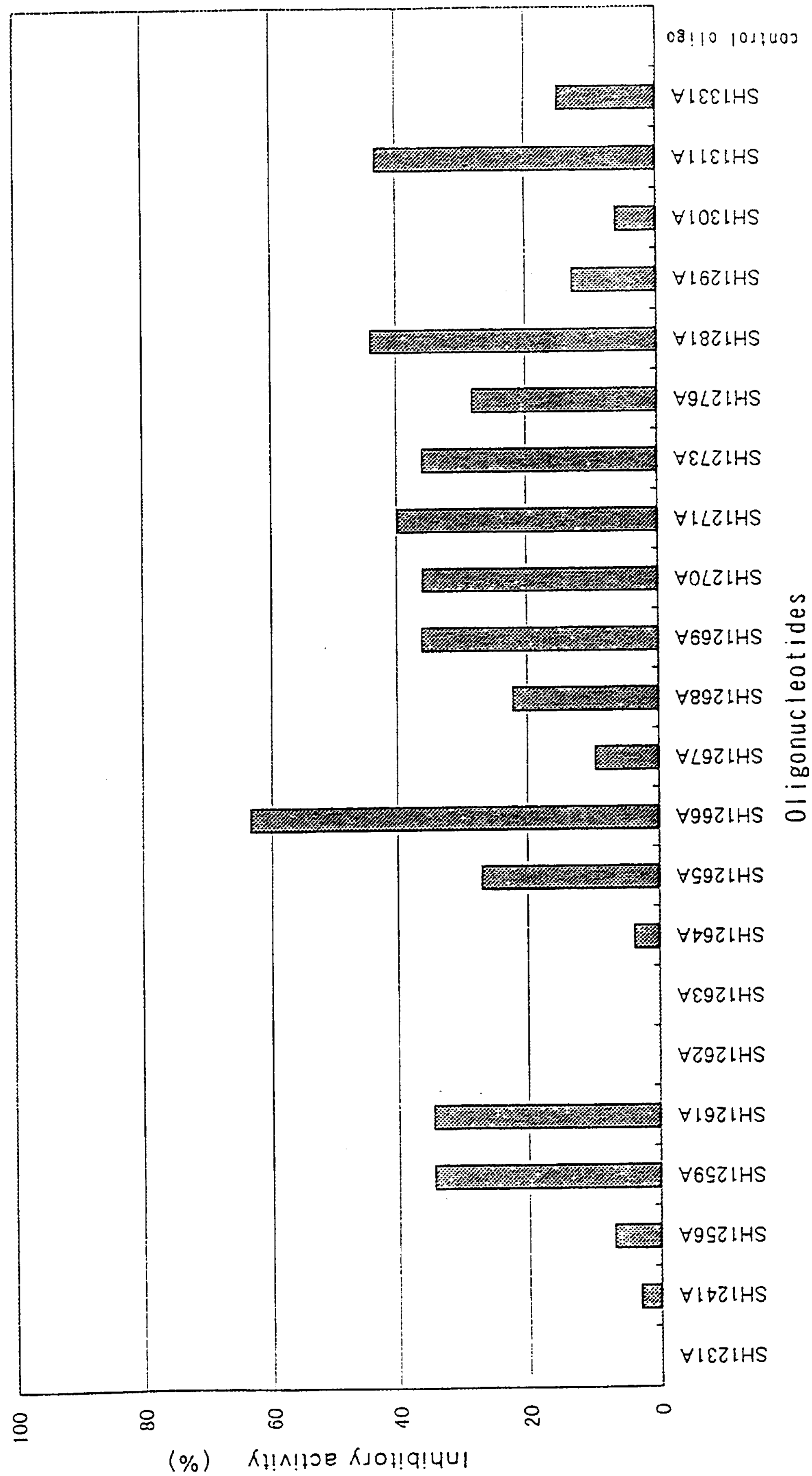

FIG. 5

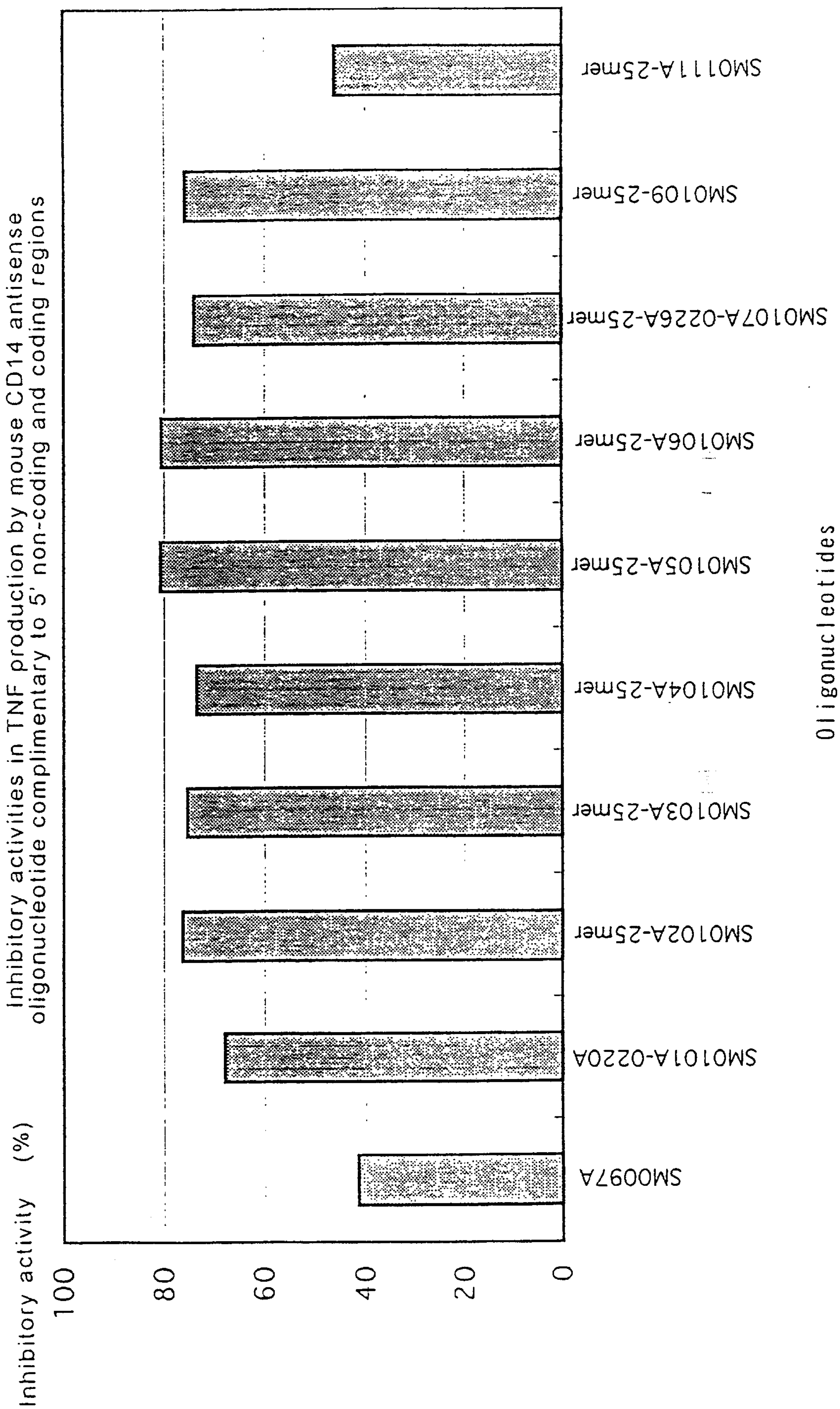
Inhibitory activities in TNF production by mouse CD14 antisense oligonucleotide complimentary to 5' non-coding and coding regions
Inhibitory activity (%)
100
80
60
40
20
0
SM0097A
SM0101A-0220A
SM0102A-25mer
SM0103A-25mer
SM0104A-25mer
SM0105A-25mer
SM0106A-25mer
SM0107A-0226A-25mer
SM0109-25mer
SM0111A-25mer
Oligonucleotides

FIG. 6

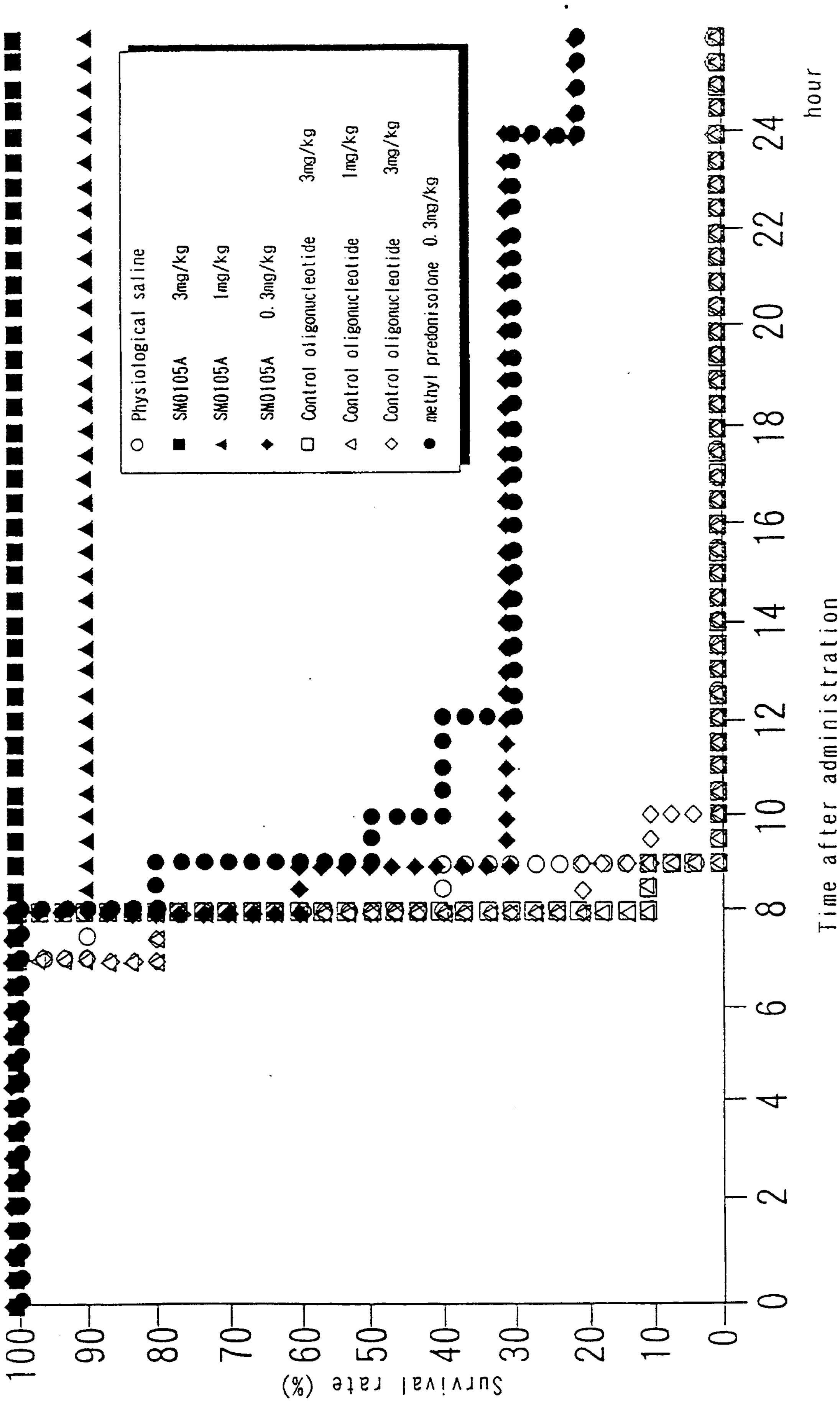
Physiological saline
SM0105A 3mg/kg
SM0105A 1mg/kg
SM0105A 0.3mg/kg
Control oligonucleotide 3mg/kg
Control oligonucleotide 1mg/kg
Control oligonucleotide 3mg/kg
methyl predonisolone 0.3mg/kg
Survival rate (%)
100
90
80
70
60
50
40
30
20
10
0
0
2
4
6
8
10
12
14
16
18
20
22
24
hour
Time after administration

FIG. 7

Effect of SM0105A on GPT activity in endotoxin shock model

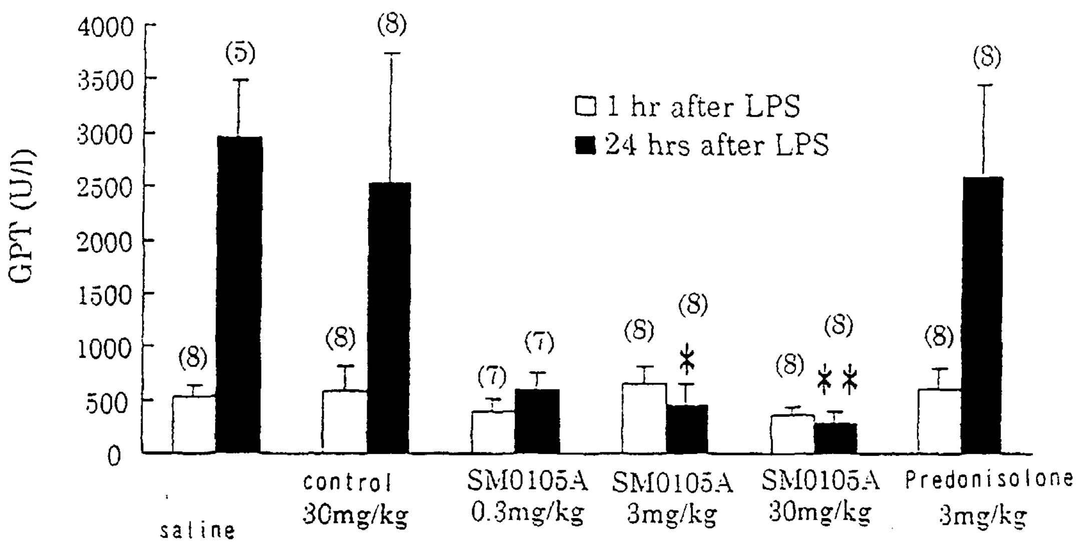

FIG.8

Inhibitory activities in human CD14 / luciferase fusion protein expression by human CD14 antisense oligonucleotides complimentary to 5' non-coding region

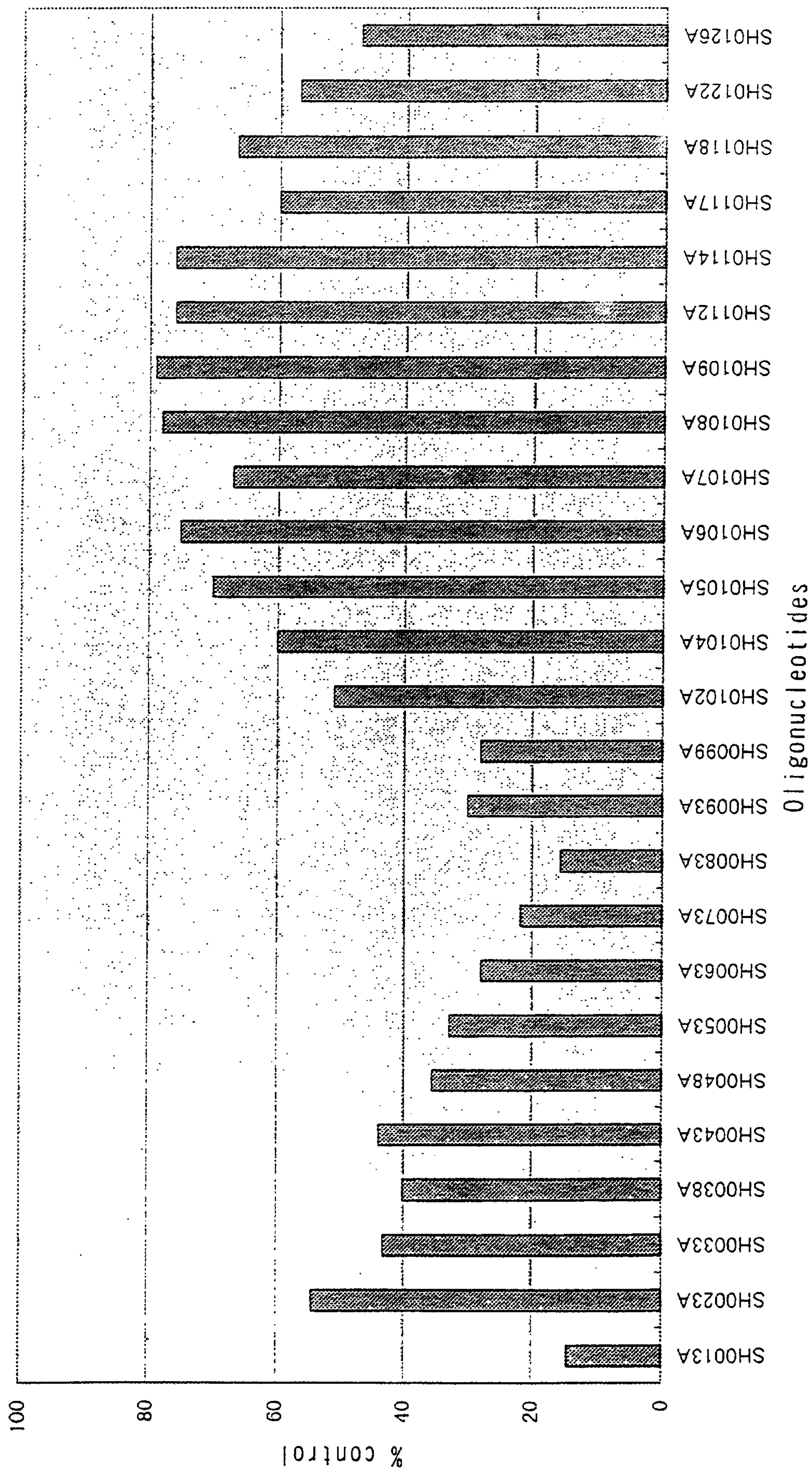

FIG.9

Inhibitory activities in TNF production by human CD14 antisense oligonucleotides complimentary to coding region

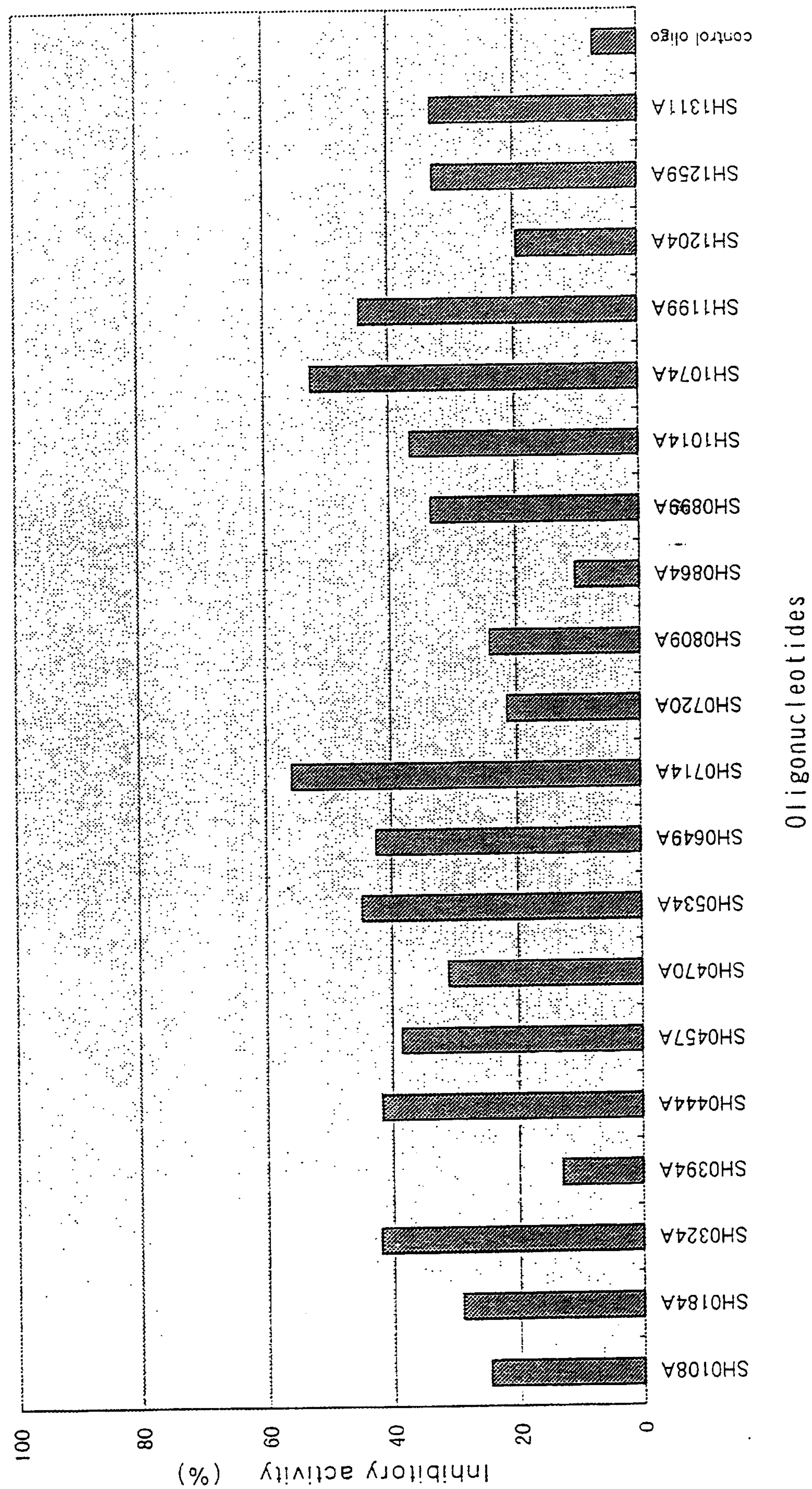

# FIG.10

| | | | | |
|---|---|---|---|---|
| 5' | 103 | | 137 | 3' |
| human | A CUU AUC GAC CAU GGA GCG CGC GUC CUG CUU GUU G | | | |
| mouse | A UCU ACC GAC CAU GGA GCG UGU GCU UGG CUU GUU G | | | |

Sequence of consensus oligonucleotide

| | | | | |
|---|---|---|---|---|
| 3' | 103 | | 137 | 5' |
| | T XXA TXG CTG GTA CCT CGC XCX CXX XXC GAA CAA C | | | |

FIG. 11

Inhibitory effect of consensus oligonucleotides in expression of human CD14 luciferase fusion protein

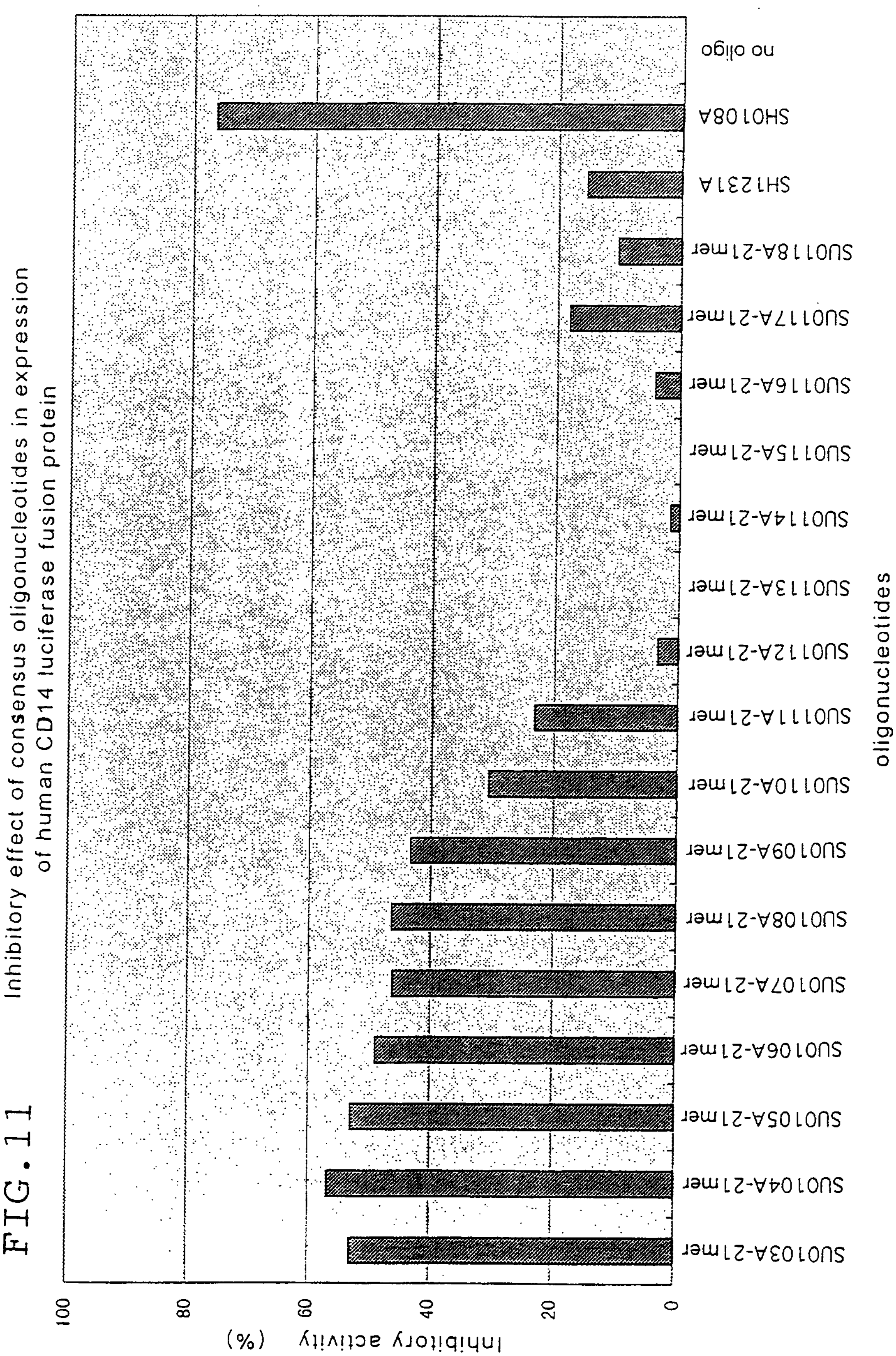

FIG.12

Inhibitory activities of consensus oligonucleotides in mouse TNFα production

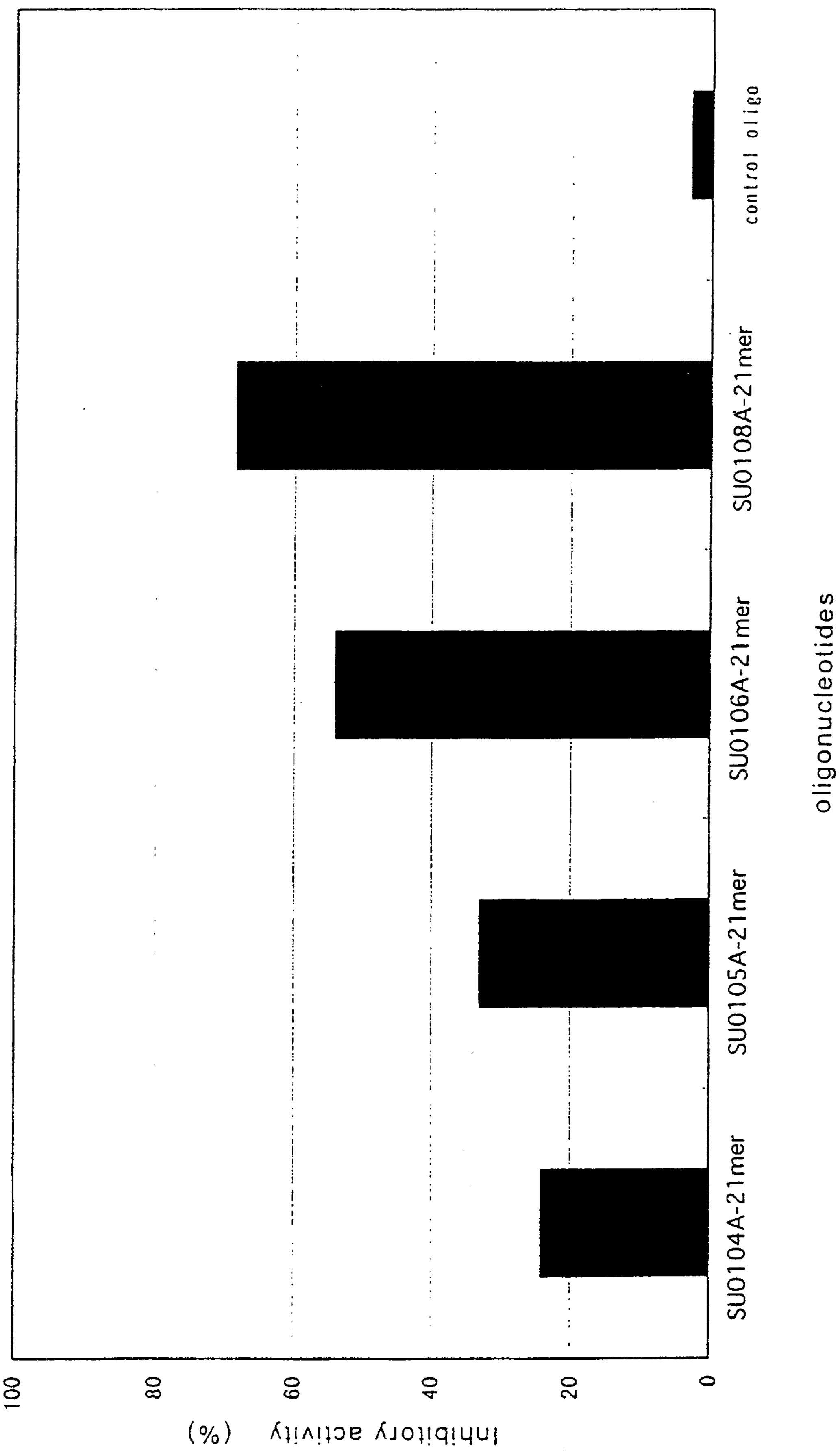

## INTERNATIONAL SEARCH REPORT

International application No.
PCT/JP98/00953

A. CLASSIFICATION OF SUBJECT MATTER
Int.Cl[6] C12N15/12, A61K31/70

According to International Patent Classification (IPC) or to both national classification and IPC

B. FIELDS SEARCHED

Minimum documentation searched (classification system followed by classification symbols)
Int.Cl[6] C12N15/12, A61K31/70

Documentation searched other than minimum documentation to the extent that such documents are included in the fields searched

Electronic data base consulted during the international search (name of data base and, where practicable, search terms used)
BIOSIS (DIALOG), WPI (DIALOG)

C. DOCUMENTS CONSIDERED TO BE RELEVANT

| Category* | Citation of document, with indication, where appropriate, of the relevant passages | Relevant to claim No. |
|---|---|---|
| X<br>Y | FERRERO, E. et al., "Nucleotide sequence of the gene encoding the monocyte differentiation antigen, CD14", Nucleic Acids Research (1988) Vol. 16, No. 9 p.4173 | 1-18<br>19-21 |
| X<br>Y | US, 5543303, A (Sanna M. Goyert), August 6, 1996 (06. 08. 96) (Family: none) | 1-18<br>19-21 |
| Y | DELUDE, R.L. et al., "CD14 enhances cellular responses to endotoxin without imparting ligand-specific recognition", Proc. Natl. Acad. Sci. USA (1995) Vol. 92, No. 20 p.9288-9292 | 19-21 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| May 21, 1998 (21. 05. 98) | June 2, 1998 (02. 06. 98) |
| Name and mailing address of the ISA/<br>Japanese Patent Office | Authorized officer |
| Facsimile No. | Telephone No. |

Form PCT/ISA/210 (second sheet) (July 1992)